(19) Europäisches Patentamt
European Patent Office
Office européen des brevets

(11) EP 2 363 407 A1

(12) **EUROPEAN PATENT APPLICATION**

(43) Date of publication:
**07.09.2011 Bulletin 2011/36**

(51) Int Cl.:
***C07K 14/20*** *(2006.01)*

(21) Application number: **08152093.4**

(22) Date of filing: **28.02.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **Murdoch University**
**Murdoch, W.A. 6150 (AU)**

(72) Inventor: **The designation of the inventor has not yet been filed**

(74) Representative: **Dean, John Paul**
**Withers & Rogers LLP**
**Goldings House**
**2 Hays Lane**
**London**
**SE1 2HW (GB)**

(54) **Novel sequences of Brachyspira, immunogenic compositions, methods for preparation and use thereof**

(57) Novel polynucleotide and amino acids of *Brachyspira hyodysenteriae* are described. Thes sequences are useful for diagnosis of *B. hyodysenteriae* disease in animals and as a therapeutic or prophylactic treatment of *B. hyodysenteriae* disease in animals. These sequences may also be useful for diagnostic and therapeutic and/or prophylactic treatment of diseases in animals caused by other *Brachyspira* species.

EP 2 363 407 A1

## Description

### Field of Invention

This invention relates to novel genes in *Brachyspira hyodysenteriae* and the proteins encoded therein. This invention further relates to use of these novel genes and proteins for diagnosis of *B. hyodysenteriae* disease, vaccines against *B. hyodysenteriae* and for screening for compounds that kill *B. hyodysenteriae* or block the pathogenic effects of *B. hyodysenteriae.* These sequences may also be useful for diagnostic and therapeutic and/or prophylactic treatment of diseases in animals caused by other *Brachyspira* species, including *B. intermedia, B. alvinipulli, B. aalborgi, B. innocens, B. murdochii, and B. pilosicoli.*

### Background of Invention

Swine dysentery is a significant endemic disease of pigs in Australia and worldwide. Swine dysentery is a contagious mucohaemorrhagic diarrhoeal disease, characterised by extensive inflammation and necrosis of the epithelial surface of the large intestine. Economic losses due to swine dysentery result mainly from growth retardation, costs of medication and mortality. The causative agent of swine dysentery was first identified as an anaerobic spirochaete (*Treponema hyodysenteriae*) in 1971, and was recently reassigned to the genus *Brachyspira* as *B. hyodysenteriae.* Where swine dysentery is established in a piggery, the disease spectrum can vary from being mild, transient or unapparent, to being severe and even fatal. Medication strategies on individual piggeries may mask clinical signs and on some piggeries the disease may go unnoticed, or may only be suspected. Whether or not obvious disease occurs, *B. hyodysenteriae* may persist in infected pigs, or in other reservoir hosts such as rodents, or in the environment. All these sources pose potential for transmission of the disease to uninfected herds. Commercial poultry may also be colonized by *B. hyodysenteriae,* although it is not clear how commonly this occurs under field conditions.

Colonisation by *B. hyodysenteriae* elicits a strong immunological response against the spirochaete, hence indirect evidence of exposure to the spirochaete can be obtained by measuring circulating antibody titres in the blood of infected animals. These antibody titres have been reported to be maintained at low levels, even in animals that have recovered from swine dysentery. Serological tests for detection of antibodies therefore have considerable potential for detecting subclinical infections and recovered carrier pigs that have undetectable numbers of spirochaetes in their large intestines. These tests would be particularly valuable in an easy to use kit form, such as an enzyme- linked immunosorbent assay. A variety of techniques have been developed to demonstrate the presence of circulating antibodies against *B. hyodysenteriae,* including indirect fluorescent antibody tests, haemagglutination tests, microtitration agglutination tests, complement fixation tests, and ELISA using either lipopolysaccharide or whole sonicated spirochaetes as antigen. All these tests have suffered from problems of specificity, as related non-pathogenic intestinal spirochaetes can induce cross-reactive antibodies. These tests are useful for detecting herds where there is obvious disease and high circulating antibody titres, but they are problematic for identifying sub-clinically infected herds and individual infected pigs. Consequently, to date, no completely sensitive and specific assays are available for the detection of antibodies against *B. hyodysenteriae.* The lack of suitable diagnostic tests has hampered control of swine dysentery.

A number of methods are employed to control swine dysentery, varying from the prophylactic use of antimicrobial agents, to complete destocking of infected herds and prevention of re-entry of infected carrier pigs. All these options are expensive and, if they are to be fully effective, they require the use of sophisticated diagnostic tests to monitor progress. Currently, detection of swine dysentery in herds with sub-clinical infections, and individual healthy carrier animals, remains a major problem and is hampering implementation of effective control measures. A definitive diagnosis of swine dysentery traditionally has required the isolation and identification of *B, hyodysenteriae* from the faeces or mucosa of diseased pigs. Major problems involved include the slow growth and fastidious nutritional requirements of these anaerobic bacteria and confusion due to the presence of morphologically similar spirochaetes in the normal flora of the pig intestine. A significant improvement in the diagnosis of individual affected pigs was achieved with the development of polymerase chain reaction (PCR) assays for the detection of spirochaetes from faeces. Unfortunately in practical applications the limit of detection of PCRs rendered it unable to detect carrier animals with subclinical infections. As a consequence of these diagnostic problems, there is a clear need to develop a simple and effective diagnostic tool capable of detecting *B. hyodysenteriae* infection at the herd and individual pig level.

A strong immunological response is induced against the spirochaete following colonization with *B. hyodysenteriae,* and pigs recovered from swine dysentery are protected from re-infection. Despite this, attempts to develop vaccines to control swine dysentery have met with very limited success, either because they have provided inadequate protection on a herd basis, or they have been too costly and difficult to produce to make them commercially viable. Bacterin vaccines provide some level of protection, but they tend to be lipopolysaccharide serogroup-specific, which then requires the use of multivalent bacterins. Furthermore they are difficult and costly to produce on a large scale because of the fastidious anaerobic growth requirements of the spirochaete.

Several attempts have been made to develop attenuated live vaccines for swine dysentery. This approach has the disadvantage that attenuated strains show reduced colonisation, and hence cause reduced immune stimulation. There also is reluctance on the part of producers and veterinarians to use live vaccines for swine dysentery because of the possibility of reversion to virulence, especially as very little is known about genetic regulation and organization in *B. hyodysenteriae..*

The use of recombinant subunit vaccines is an attractive alternative, since the products would be well-defined (essential for registration purposes), and relatively easy to produce on a large scale. To date the first reported use of a recombinant protein from *B. hyodysenteriae* as a vaccine candidate (a 38-kilodalton flagellar protein) failed to prevent colonisation in pigs. This failure is likely to relate specifically to the particular recombinant protein used, as well as to other more down-stream issues of delivery systems and routes, dose rates, choice of adjuvants etc. (Gabe, JD, Chang, RJ, Slomiany, R, Andrews, WH and McCaman, MT (1995) Isolation of extracytoplasmic proteins from Serpulina hyodysenteriae B204 and molecular cloning of the flaB1 gene encoding a 38-kilodalton flagellar protein. Infection and Immunity 63:142-148). The first reported partially protective recombinant *B. hyodysenteriae* protein used for vaccination was a 29.7 kDa outer membrane lipoprotein (Bhlp29.7, also referred to as BmpB and BlpA) which had homology with the methionine-binding lipoproteins of various pathogenic bacteria. The use of the his-tagged recombinant Bhlp29.7 protein for vaccination of pigs, followed by experimental challenge with *B. hyodysenteriae,* resulted in 17-40% of vaccinated pigs developing disease compared to 50-70% of the unvaccinated control pigs developing disease. Since the incidence of disease for the Bhlp29.7 vaccinated pigs was significantly ($P$=0.047) less than for the control pigs, Bhlp29.7 appeared to have potential as a swine dysentery vaccine component (La, T, Phillips, ND, Reichel, MP and Hampson, DJ (2004). Protection of pigs from swine dysentery by vaccination with recombinant BmpB, a 29.7 kDa outer-membrane lipoprotein of Brachyspira hyodysenteriae. Veterinary Microbiology 102:97-109). A number of other attempts have been made to identify outer envelop proteins from *B. hyodysenteriae* that could be used as recombinant vaccine components, but again no successful vaccine has yet been made. A much more global approach is needed to the identification of potentially useful immunogenic recombinant proteins from *B. hyodysenteriae* is needed.

To date, only one study using DNA for vaccination has been reported. In this study, the *B. hyodysenteriae ftnA* gene, encoding a putative ferritin, was cloned into an *E. coli* plasmid and the plasmid DNA used to coat gold beads for ballistic vaccination. A murine model for swine dysentery was used to determine the protective nature of vaccination with DNA and/or recombinant protein. Vaccination with recombinant protein induced a good systemic response against ferritin however vaccination with DNA induced only a detectable systemic response. Vaccination with DNA followed a boost with recombinant protein induced a systemic immune response to ferritin only after boosting with protein. However, none of the vaccination regimes tested was able to provide the mice with protection against *B. hyodysenteriae* colonisation and the associated lesions. Interestingly, vaccination of the mice with DNA alone resulted in significant exacerbation of disease (Davis, A.J., Smith, S.C. and Moore, R.J. (2005). The Brachyspira hyodysenteriae ftnA gene: DNA vaccination and real-time PCR quantification of bacteria in a mouse model of disease. Current Microbiology 50: 285-291).

**Brief Summary of Invention**

It is an object of this invention to have novel genes from *B. hyodysenteriae* and the proteins encoded by those genes. It is a further object of this invention that the novel genes and the proteins encoded by those genes can be used for therapeutic and diagnostic purposes. One can use the genes and/or the proteins in a vaccine against *B. hyodysenteriae* and to diagnose *B. hyodysenteriae* infections.

It is an object of this invention to have novel *B. hyodysenteriae* genes having the nucleotide sequence contained in SEQ ID NOs: 1, 3, 5, 7, 9, 11, 13, 15, 17, 19, 21, 23, 25, 27, 29, and 31. It is also an object of this invention to have nucleotide sequences that are homologous to SEQ ID NOs: 1, 3, 5, 7, 9, 11, 13, 15, 17, 19, 21, 23, 25, 27, 29, and 31 where the homology can be 95%, 90%, 85%, 80%, 75% and 70%, This invention also includes a DNA vaccine or DNA immunogenic composition containing the nucleotide sequence of SEQ ID NOs: 1, 3, 5, 7, 9, 11, 13, 15, 17, 19, 21, 23, 25, 27, 29, and 31 and sequences that are 95%, 90%, 85%, 80%, 75% and 70% homologous to these sequences. This invention further includes a diagnostic assay containing DNA having the nucleotide sequence of SEQ ID NOs: 1, 3, 5, 7, 9, 11, 13, 15, 17, 19, 21, 23, 25, 27, 29, and 31 and sequences that are 95%, 90%, 85%, 80%, 75% and 70% homologous to these sequences.

In one aspect of the invention, the vaccine composition is a vaccine composition comprising at least two polynucleotides encoding protease molecules selected from the group consisting of SEQ ID No.1, 3, 5 and 7 for the treatment or prevention of swine dysentery associated with *Brachyspira hyodysenteriae.*

In another aspect of the invention, the vaccine composition is a vaccine composition comprising at least three polynucleotides encoding protease molecules selected from the group consisting of SEQ ID No.1, 3, 5 and 7 for the treatment or prevention of swine dysentery associated with *Brachyspira hyodysenteriae.*

In yet another aspect of the invention, the vaccine composition is a vaccine composition consisting of polynucleotides encoding protease molecules selected from the group consisting of: SEQ ID No.1, 3, 5 and 7 for the treatment or prevention of swine dysentery associated with *Brachyspira hyodysenteriae..*

In a further aspect of the invention, the vaccine composition is a vaccine composition comprising at least two polynucleotides encoding lipase and/or peptidase molecules selected from the group consisting of SEQ ID No.9, 11, 13 and 15 for the treatment or prevention of swine dysentery associated with *Brachyspira hyodysenteriae.*

In another aspect of the invention, the vaccine composition is a vaccine composition comprising at least three polynucleotides encoding lipase and/or peptidase molecules selected from the group consisting of SEQ ID No.9, 11, 13 and 15 for the treatment or prevention of swine dysentery associated with *Brachyspira hyodysenteriae.*

In yet another aspect of the invention, the vaccine composition is a vaccine composition consisting of polynucleotides encoding lipase and/or peptidase molecules selected from the group consisting of: SEQ ID No.9, 11, 13 and 15 for the treatment or prevention of swine dysentery associated with *Brachyspira hyodysenteriae.*

In a further aspect of the invention, the vaccine composition is a vaccine composition comprising at least two polynucleotides encoding toxin molecules selected from the group consisting of SEQ ID No.17, 19, 21 and 23 for the treatment or prevention of swine dysentery associated with *Brachyspira hyodysenteriae.*

In another aspect of the invention, the vaccine composition is a vaccine composition comprising at least three polynucleotides encoding toxin molecules selected from the group consisting of SEQ ID No.17, 19, 21 and 23 for the treatment or prevention of swine dysentery associated with *Brachyspira hyodysenteriae.*

In yet another aspect of the invention, the vaccine composition is a vaccine composition consisting of polynucleotides encoding toxin molecules selected from the group consisting of: SEQ ID No.17, 19, 21 and 23 for the treatment or prevention of swine dysentery associated with *Brachyspira hyodysenteriae.*

In another aspect of the invention, the vaccine composition is vaccine composition comprising at least two polynucleotides encoding hemolysin molecules selected from the group consisting of SEQ ID No.25, 27, 29 and 31 for the treatment or prevention of swine dysentery associated with *Brachyspira hyodysenteriae.*

In yet another aspect of the invention, the vaccine composition is a vaccine composition comprising at least three polynucleotides encoding hemolysin molecule selected from the group consisting of SEQ ID No.25, 27, 29 and 31 for the treatment or prevention of swine dysentery associated with *Brachyspira hyodysenteriae.*

In a further aspect of the invention, the vaccine composition is a vaccine composition consisting of polynucleotides encoding hemolysin molecules selected from the group consisting of: SEQ ID No.25, 27, 29 and 31 for the treatment or prevention of swine dysentery associated with *Brachyspira hyodysenteriae.*

In another aspect of the invention, the vaccine composition is a vaccine composition comprising:

(i) at least one polynucleotide encoding a protease molecule selected from the group of: SEQ ID No. 1, 3, 5 and 7
(ii) at least one polynucleotide encoding a lipase and/or peptidase molecule selected from the group of: SEQ ID No. 9, 11, 13 and 15
(iii) at least one polynucleotide encoding a toxin molecule selected from the group of: SEQ ID No. 17, 19, 21 and 23
(iv) at least one polynucleotide encoding a hemolysin molecule selected from the group of: SEQ ID No. 25, 27, 29 and 31 for the treatment or prevention of swine dysentery associated with *Brachyspira hyodysenteriae.*

It is also an object of this invention to have plasmids containing DNA having the sequence of SEQ ID NOs: 1, 3, 5, 7, 9, 11, 13, 15, 17, 19, 21, 23, 25, 27, 29, and 31; prokaryotic and/or eukaryotic expression vectors containing DNA having the sequence of SEQ ID NOs:1, 3, 5, 7, 9, 11, 13, 15, 17, 19, 21, 23, 25, 27, 29, and 31; and a cell containing the plasmids which contain DNA having the sequence of SEQ ID NOs: 1, 3, 5, 7, 9, 11, 13, 15, 17, 19, 21, 23, 25, 27, 29, and 31.

It is an object of this invention to have navel *B. hyodysenteriae* proteins having the amino acid sequence contained in SEQ ID NOs: 2, 4, 6, 8, 10, 12, 14, 16, 18, 20, 22, 24, 26, 28, 30 and 32. It is another object of this invention to have proteins that are 95%, 90%, 85%, 80%, 75% and 70% homologous to the sequences contained in SEQ ID NOs: 2, 4, 6, 8, 10, 12, 14, 16, 18, 20, 22, 24, 26, 28, 30 and 32. It is also an object of this invention for a vaccine or immunogenic composition to contain the proteins having the amino acid sequence contained in SEQ ID NOs: 2,4, 6, 8, 10, 12, 14, 16, 18, 20, 22, 24, 26, 28, 30 and 32 or amino acid sequences that are 95%, 90%, 85%, 80%, 75% and 70% homologous to the sequences contained in SEQ ID NOs: 2, 4, 6, 8, 10, 12, 14, 16, 18, 20, 22, 24, 26, 28, 30 and 32.

In one aspect of the invention, the vaccine composition is a vaccine composition comprising at least two protease polypeptides selected from the group consisting of SEQ ID No.2, 4, 6 and 8 for the treatment or prevention of swine dysentery associated with Brachyspira hyodysenteriae.

In another aspect of the invention, the vaccine composition is a vaccine composition comprising at least three protease polypeptides selected from the group consisting of SEQ ID No.2, 4, 6 and 8 for the treatment or prevention of swine dysentery associated with Brachyspira hyodysenteriae.

In yet another aspect of the invention, the vaccine composition is a vaccine composition consisting of protease polypeptides SEQ ID No,2, 4, 6 and 8 for the treatment or prevention of swine dysentery associated with Brachyspira hyodysenteriae.

In one aspect of the invention, the vaccine composition is a vaccine composition comprising at least two lipase and/or peptidase polypeptides selected from the group consisting of SEQ ID No.10, 12, 14 and 16 for the treatment or prevention of swine dysentery associated with Brachyspira hyodysenteriae.

In another aspect of the invention, the vaccine composition is a vaccine composition comprising at least three lipase and/or peptidase polypeptides selected from the group consisting of SEQ ID No. 10, 12, 14 and 16 for the treatment or prevention of swine dysentery associated with Brachyspira hyodysenteriae.

In yet another aspect of the invention, the vaccine composition is a vaccine composition consisting of lipase and/or peptidase polypeptides from the group consisting of: SEQ ID No.10, 12, 14 and 16 for the treatment or prevention of swine dysentery associated with Brachyspira hyodysenteriae.

In one aspect of the invention, the vaccine composition is a vaccine composition comprising at least two toxin polypeptides selected from the group consisting of SEQ ID No.18, 20, 22 and 24 for the treatment or prevention of swine dysentery associated with Brachyspira hyodysenteriae.

In another aspect of the invention, the vaccine composition is a vaccine composition comprising at least three toxin polypeptides selected from the group consisting of SEQ ID No.18, 20, 22 and 24 for the treatment or prevention of swine dysentery associated with Brachyspira hyodysenteriae.

In yet another aspect of the invention, the vaccine composition is a vaccine composition consisting of toxin polypeptides selected from the group consisting of: SEQ ID No.18, 20, 22 and 24 for the treatment or prevention of swine dysentery associated with Brachyspira hyodysenteriae.

In one aspect of the invention, the vaccine composition is a vaccine composition comprising at least two hemolysin polypeptides selected from the group consisting of SEQ ID No.25, 27, 29 and 31 for the treatment or prevention of swine dysentery associated with Brachyspira hyodysenteriae.

In another aspect of the invention, the vaccine composition is a vaccine composition comprising at least three hemolysin polypeptides selected from the group consisting of SEQ ID No.26, 28, 30 and 32 for the treatment or prevention of swine dysentery associated with Brachyspira hyodysenteriae.

In yet another aspect of the invention, the vaccine composition is a vaccine composition consisting of hemolysin polypeptides SEQ ID No.26, 28, 30 and 32 for the treatment or prevention of swine dysentery associated with Brachyspira hyodysenteriae.

In a further aspect of the invention, the vaccine composition is a vaccine composition comprising:

(i) at least one protease polypeptide selected from the group of: SEQ ID No.2, 4, 6, or 8
(ii) at least one lipase and/or peptidase polypeptide selected from the group of: SEQ ID No. 10, 12, 14, or 16
(iii) at least one toxin selected from the group of: SEQ ID No. 18, 20, 22 or 24
(iv) at least one hemolysin selected from the group of: SEQ ID No. 26, 28, 30 or 32 for the treatment or prevention of swine dysentery associated with Brachyspira hyodysenteriae.

It is a further aspect of this invention to have a diagnostic kit containing one or more proteins having a sequence contained in SEQ ID NOs: 2, 4, 6, 8, 10, 12, 14, 16, 18, 20, 22, 24, 26, 28, 30 and 32 or that are 95%, 90%, 85%, 80%, 75% and 70% homologous to the sequences contained in SEQ ID NOs: 2, 4, 6, 8, 10, 12, 14, 16, 18, 20, 22, 24, 26, 28, 30 and 32.

It is another aspect of this invention to have nucleotide sequences which encode the proteins having the amino acid sequence contained in SEQ ID NOs: 2, 4, 6, 8, 10, 12, 14, 16, 18, 20, 22, 24, 26, 28, 30 and 32. The invention also covers plasmids, eukaryotic and prokaryotic expression vectors, and DNA vaccines which contain DNA having a sequence which encodes a protein having the amino acid sequence contained in SEQ ID NOs: 2, 4, 6, 8, 10, 12, 14, 16, 18, 20, 22, 24, 26, 28, 30 and 32. Cells which contain these plasmids and expression vectors are included in this invention.

This invention includes monoclonal antibodies that bind to proteins having an amino acid sequence contained in SEQ ID NOs: 2, 4, 6, 8, 10, 12, 14, 16, 18, 20, 22, 24, 26, 28, 30 and 32 or bind to proteins that are 95%, 90%, 85%, 80%, 75% and 70% homologous to the sequences contained in SEQ ID NOs: 2,4, 6, 8, 10, 12, 14, 16, 18, 20, 22, 24, 26, 28, 30 and 32. Diagnostic kits containing the monoclonal antibodies that bind to proteins having an amino acid sequence contained in SEQ ID NOs: 2, 4, 6, 8, 10, 12, 14, 16, 18, 20, 22, 24, 26, 28, 30 and 32 or bind to proteins that are 95%, 90%, 85%, 80%, 75% and 70% homologous to the sequences contained in SEQ ID NOs: 2, 4, 6, 8, 10, 12, 14, 16, 18, 20, 22, 24, 26, 28, 30 and 32 are included in this invention. These diagnostic kits can detect the presence of *B. hyodysenteriae* in an animal. The animal is preferably any mammal and bird; more preferably, chicken, goose, duck, turkey, parakeet, dog, cat, hamster, gerbil, rabbit, ferret, horse, cow, sheep, pig, monkey, and human.

The invention also contemplates the method of preventing or treating an infection of *B. hyodysenteriae* in an animal by administering to an animal a DNA vaccine containing one or more nucleotide sequences listed in SEQ ID NOs: 2, 3, 5, 7, 9, 11, 13, 15, 17, 19, 21, 23, 25, 27, 29, and 31 or sequences that are 95%, 90%, 85%, 80%, 75% and 70% homologous to these sequences. This invention also covers a method of preventing or treating an infection of *B. hyodysenteriae* in an animal by administering to an animal a vaccine containing one or more proteins having the amino acid sequence containing in SEQ ID NOs: 2, 4, 6, 8, 10, 12, 14, 16, 18, 20, 22, 24, 26, 28, 30 and 32 or sequences that are 95%, 90%, 85%, 80%, 75% and 70% homologous to these sequences. The animal is preferably any mammal and bird; more preferably, chicken, goose, duck, turkey, parakeet, dog, cat, hamster, gerbil, rabbit, ferret, horse, cow, sheep, pig, monkey, and human.

The invention also contemplates the method of generating an immune response in an animal by administering to an animal an immunogenic composition containing one or more nucleotide sequences listed in SEQ ID NOs: 1, 3, 5, 7, 9, 11, 13, 15, 17, 19, 21, 23, 25, 27, 29, and 31 or sequences that are 95%, 90%, 85%, 80%, 75% and 70% homologous to these sequences. This invention also covers a method of generating an immune response in an animal by administering to an animal an immunogenic composition containing one or more proteins having the amino acid sequence containing in SEQ ID NOs: 2, 4, 6, 8, 10, 12, 14, 16, 18, 20, 22, 24, 26, 28, 30 and 32 or sequences that are 95%, 90%, 85%, 80%, 75% and 70% homologous to these sequences. The animal is preferably any mammal and bird; more preferably, chicken, goose, duck, turkey, parakeet, dog, cat, hamster, gerbil, rabbit, ferret, horse, cow, sheep, pig, monkey, and human.

**Detailed Summary of Invention**

The articles "a" and "an" are used herein to refer to one or to more than one (i.e., to at least one) of the grammatical object of the article. By way of example, "an element" means one element or more than one element.

The term "amino acid" is intended to embrace all molecules, whether natural or synthetic, which include both an amino functionality and an acid functionality and capable of being included in a polymer of naturally-occurring amino acids. Exemplary amino acids include naturally-occurring amino acids; analogs, derivatives and congeners thereof; amino acid analogs having variant side chains; and all stereoisomers of any of any of the foregoing.

An animal can be any mammal or bird. Examples of mammals include dog, cat, hamster, gerbil, rabbit, ferret, horse, cow, sheep, pig, monkey, and human. Examples of birds include chicken, goose, duck, turkey, and parakeet.

The term "conserved residue" refers to an amino acid that is a member of a group of amino acids having certain common properties. The term "conservative amino acid substitution" refers to the substitution (conceptually or otherwise) of an amino acid from one such group with a different amino acid from the same group. A functional way to define common properties between individual amino acids is to analyze the normalized frequencies of amino acid changes between corresponding proteins of homologous organisms (Schulz, G. E. and R. H. Schinner., Principles of Protein Structure, Springer-Verlag). According to such analyses, groups of amino acids may be defined where amino acids within a group exchange preferentially with each other, and therefore resemble each other most in their impact on the overall protein structure (Schulz, G. E. and R. H. Schirmer, Principles of Protein Structure, Springer-Verlag). Examples of amino acid groups defined in this manner include: (i) a positively-charged group containing Lys, Arg and His, (ii) a negatively-charged group containing Glu and Asp, (iii) an aromatic group containing Phe, Tyr and Trp, (iv) a nitrogen ring group containing His and Trp, (v) a large aliphatic nonpolar group containing Val, Leu and De, (vi) a slightly-polar group containing Met and Cys, (vii) a small-residue group containing Ser, Thr; Asp, Asn, Gly, Ala, Glu, Gln and Pro, (viii) an aliphatic group containing Val, Leu, De, Met and Cys, and (ix) a small, hydroxyl group containing Ser and Thr.

A "fusion protein" or "fusion polypeptide" refers to a chimeric protein as that term is known in the art and may be constructed using methods known in the art. In many examples of fusion proteins, there are two different polypeptide sequences, and in certain cases, there may be more. The polynucleotide sequences encoding the fusion protein may be operably linked in frame so that the fusion protein may be translated correctly. A fusion protein may include polypeptide sequences from the same species or from different species. In various embodiments, the fusion polypeptide may contain one or more amino acid sequences linked to a first polypeptide. In the case where more than one amino acid sequence is fused to a first polypeptide, the fusion sequences may be multiple copies of the same sequence, or alternatively, may be different amino acid sequences. The fusion polypeptides may be fused to the N-terminus, the C-terminus, or the N- and C-terminus of the first polypeptide. Exemplary fusion proteins include polypeptides containing a glutathione S-transferase tag (GST-tag), histidine tag (His-tag), an immunoglobulin domain or an immunoglobulin binding domain.

The term "isolated polypeptide" refers to a polypeptide, in certain embodiments prepared from recombinant DNA or RNA, or of synthetic origin or natural origin, or some combination thereof, which (1) is not associated with proteins that it is normally found with in nature, (2) is separated from the cell in which it normally occurs, (3) is free of other proteins from the same cellular source, (4) is expressed by a cell from a different species, or (5) does not occur in nature. It is possible for an isolated polypeptide exist but not qualify as a purified polypeptide.

The term "isolated nucleic acid" and "isolated polynucleotide" refers to a polynucleotide whether genomic DNA, cDNA, mRNA, tRNA, rRNA, iRNA, or a polynucleotide obtained from a cellular organelle (such as mitochondria and chloroplast), or whether from synthetic origin, which (1) is not associated with the cell in which the "isolated nucleic acid" is found in nature, or (2) is operably linked to a polynucleotide to which it is not linked in nature. It is possible for an isolated polynucleotide exist but not qualify as a purified polynucleotide.

The term "nucleic acid" and "polynucleotide" refers to a polymeric form of nucleotides, either ribonucleotides or deoxyribonucleotides or a modified form of either type of nucleotide. The terms should also be understood to include, as equivalents, analogs of either RNA or DNA made from nucleotide analogs, and, as applicable to the embodiment being described, single-stranded (such as sense or antisense) and double-stranded polynucleotides.

The term "nucleic acid of the invention" and "polynucleotide of the invention" refers to a nucleic acid encoding a polypeptide of the invention. A polynucleotide of the invention may comprise all, or a portion of, a subject nucleic acid sequence; a nucleotide sequence at least 60%, 70%, 80%, 90%, 95%, 96%, 97%, 98% or 99% identical to a subject nucleic acid sequence; a nucleotide sequence that hybridizes under stringent conditions to a subject nucleic acid sequence; nucleotide sequences encoding polypeptides that are functionally equivalent to polypeptides of the invention; nucleotide sequences encoding polypeptides at least about 60%, 70%, 80%, 85%, 90%, 95%, 98%, 99% homologous or identical with a subject amino acid sequence; nucleotide sequences encoding polypeptides having an activity of a polypeptide of the invention and having at least about 60%, 70%, 80%, 85%, 90%, 95%, 98%, 99% or more homology or identity with a subject amino acid sequence; nucleotide sequences that differ by 1 to about 2, 3, 5, 7, 10, 15, 20, 30, 50, 75 or more nucleotide substitutions, additions or deletions, such as allelic variants, of a subject nucleic acid sequence; nucleic acids derived from and evolutionarily related to a subject nucleic acid sequence; and complements of, and nucleotide sequences resulting from the degeneracy of the genetic code, for all of the foregoing and other nucleic acids of the invention. Nucleic acids of the invention also include homologs, *e.g.*, orthologs and paralogs, of a subject nucleic acid sequence and also variants of a subject nucleic acid sequence which have been codon optimized for expression in a particular organism (*e.g.,* host cell).

The term "operably linked", when describing the relationship between two nucleic acid regions, refers to a juxtaposition wherein the regions are in a relationship permitting them to function in their intended manner. For example, a control sequence "operably linked" to a coding sequence is ligated in such a way that expression of the coding sequence is achieved under conditions compatible with the control sequences, such as when the appropriate molecules, (*e.g.*, inducers and polymerases) are bound to the control or regulatory sequence(s).

The term "polypeptide", and the terms "protein" and "peptide" which are used interchangeably herein, refers to a polymer of amino acids. Exemplary polypeptides include gene products, naturally-occurring proteins, homologs, orthologs, paralogs, fragments, and other equivalents, variants and analogs of the foregoing.

The terms "polypeptide fragment" or "fragment", when used in reference to a reference polypeptide, refers to a polypeptide in which amino acid residues are deleted as compared to the reference polypeptide itself, but where the remaining amino acid sequence is usually identical to the corresponding positions in the reference polypeptide. Such deletions may occur at the amino-terminus or carboxy-terminus of the reference polypeptide, or alternatively both. Fragments typically are at least 5, 6, 8 or 10 amino acids long, at least 14 amino acids long, at least 20,30,40 or 50 amino acids long, at least 75 amino acids long, or at least 100, 150,200,300,500 or more amino acids long. A fragment can retain one or more of the biological activities of the reference polypeptide. In certain embodiments, a fragment may comprise a domain having the desired biological activity, and optionally additional amino acids on one or both sides of the domain, which additional amino acids may number from 5, 10, 15, 20, 30, 40, 50, or up to 100 or more residues. Further, fragments can include a sub-fragment of a specific region, which sub-fragment retains a function of the region from which it is derived. In another embodiment, a fragment may have immunogenic properties.

The term "polypeptide of the invention" refers to a polypeptide containing a subject amino acid sequence, or an equivalent or fragment thereof. Polypeptides of the invention include polypeptides containing all or a portion of a subject amino acid sequence; a subject amino acid sequence with I to about 2, 3, 5, 7,10,15 ,20, 30, 50, 75 or more conservative amino acid substitutions; an amino acid sequence that is at least 60%, 70%, 80%, 90%, 95%, 96%, 97%, 98%, or 99% identical to a subject amino acid sequence; and functional fragments thereof. Polypeptides of the invention also include homology, *e.g.*, orthologs and paralogs, of a subject amino acid sequence.

It is also possible to modify the structure of the polypeptides of the invention for such purposes as enhancing therapeutic or prophylactic efficacy, or stability (e.g., *ex vivo* shelf life, resistance to proteolytic degradation *in vivo, etc*.). Such modified polypeptides, when designed to retain at least one activity of the naturally-occurring form of the protein, are considered "functional equivalents" of the polypeptides described in more detail herein. Such modified polypeptides may be produced, for instance, by amino acid substitution, deletion, or addition, which substitutions may consist in whole or part by conservative amino acid substitutions.

For instance, it is reasonable to expect that an isolated conservative amino acid substitution, such as replacement of a leucine with an isoleucine or valine, an aspartate with a glutamate, a threonine with a serine, will not have a major affect on the biological activity of the resulting molecule. Whether a change in the amino acid sequence of a polypeptide results in a functional homology may be readily determined by assessing the ability of the variant polypeptide to produce a response similar to that of the wild-type protein. Polypeptides in which more than one replacement has taken place may readily be tested in the same manner.

The term "purified" refers to an object species that is the predominant species present (*i.e.*, on a molar basis it is more abundant than any other individual species in the composition). A "purified fraction" is a composition wherein the object species is at least about 50 percent (on a molar basis) of all species present. In making the determination of the purity or a species in solution or dispersion, the solvent or matrix in which the species is dissolved or dispersed is usually not included in such determination; instead, only the species (including the one of interest) dissolved or dispersed are taken into account. Generally, a purified composition will have one species that is more than about 80% of all species present in the composition, more than about 85%, 90%, 95%, 99% or more of all species present. The object species may be purified to essential homogeneity (contaminant species cannot be detected in the composition by conventional detection methods) wherein the composition is essentially a single species. A skilled artisan may purify a polypeptide of the invention using standard techniques for protein purification in light of the teachings herein. Purity of a polypeptide may be determined by a number of methods known to those of skill in the art, including for example, amino-terminal amino acid sequence analysis, gel electrophoresis, mass-spectrometry analysis and the methods described herein.

The terms "recombinant protein" or "recombinant polypeptide" refer to a polypeptide which is produced by recombinant DNA techniques. An example of such techniques includes the case when DNA encoding the expressed protein is inserted into a suitable expression vector which is in turn used to transform a host cell to produce the protein or polypeptide encoded by the DNA.

The term "regulatory sequence" is a generic term used throughout the specification to refer to polynucleotide sequences, such as initiation signals, enhancers, regulators and promoters, that are necessary or desirable to affect the expression of coding and non-coding sequences to which they are operably linked. Exemplary regulatory sequences are described in Goeddel; Gene Expression Technology: Methods in Enzymology, Academic Press, San Diego, CA (1990), and include, for example, the early and late promoters of SV40, adenovirus or cytomegalovirus immediate early promoter, the lac system, the trp system, the TAC or TRC system, T7 promoter whose expression is directed by T7 RNA polymerase, the major operator and promoter regions of phage lambda, the control regions for fd coat protein, the promoter for 3-phosphoglycerate kinase or other glycolytic enzymes, the promoters of acid phosphatase (*e.g.*, Pho5), the promoters of the yeast $\alpha$-mating factors, the polyhedron promoter of the baculovirus system and other sequences known to control the expression of genes of prokaryotic or eukaryotic cells or their viruses, and various combinations thereof. The nature and use of such control sequences may differ depending upon the host organism. In prokaryotes, such regulatory sequences generally include promoter, ribosomal binding site, and transcription termination sequences. The term "regulatory sequence" is intended to include, at a minimum, components whose presence may influence expression, and may also include additional components whose presence is advantageous, for example, leader sequences and fusion partner sequences. In certain embodiments, transcription of a polynucleotide sequence is under the control of a promoter sequence (or other regulatory sequence) which controls the expression of the polynucleotide in a cell-type in which expression is intended. It will also be understood that the polynucleotide can be under the control of regulatory sequences which are the same or different from those sequences which control expression of the naturally-occurring form of the polynucleotide.

The term "sequence homology" refers to the proportion of base matches between two nucleic acid sequences or the proportion of amino acid matches between two amino acid sequences. When sequence homology is expressed as a percentage, *e.g.*, 50%, the percentage denotes the proportion of matches over the length of sequence from a desired sequence that is compared to some other sequence. Gaps (in either of the two sequences) are permitted to maximize matching; gap lengths of 15 bases or less are usually used, 6 bases or less are used more frequently, with 2 bases or less used even more frequently. The term "sequence identity" means that sequences are identical (*i.e.*, on a nucleotide-by-nucleotide basis for nucleic acids or amino acid-by-amino acid basis for polypeptides) over a window of comparison. The term "percentage of sequence identity" is calculated by comparing two optimally aligned sequences over the comparison window, determining the number of positions at which the identical amino acids or nucleotides occurs in both sequences to yield the number of matched positions, dividing the number of matched positions by the total number of positions in the comparison window, and multiplying the result by 100 to yield the percentage of sequence identity. Methods to calculate sequence identity are known to those of skill in the art and described in further detail below.

The term "soluble" as used herein with reference to a polypeptide of the invention or other protein, means that upon expression in cell culture, at least some portion of the polypeptide or protein expressed remains in the cytoplasmic fraction of the cell and does not fractionate with the cellular debris upon lysis and centrifugation of the lysate. Solubility of a polypeptide may be increased by a variety of art recognized methods, including fusion to a heterologous amino acid sequence, deletion of amino acid residues, amino acid substitution (*e.g.*, enriching the sequence with amino acid residues having hydrophilic side chains), and chemical modification (e.g., addition of hydrophilic groups).

The solubility of polypeptides may be measured using a variety of art recognized techniques, including, dynamic light scattering to determine aggregation state, UV absorption, centrifugation to separate aggregated from non-aggregated material, and SDS gel electrophoresis (*e.g.*, the amount of protein in the soluble fraction is compared to the amount of protein in the soluble and insoluble fractions combined). When expressed in a host cell, the polypeptides of the invention may be at least about 1%, 2%, 5%, 10%, 20%, 30%, 40%, 50%, 60%, 70%, 80%, 90% or more soluble, e.g., at least about 1%, 2%, 5%, 10%, 20%, 30%, 40%, 50%, 60%, 70%, 80%, 90% or more of the total amount of protein expressed in the cell is found in the cytoplasmic fraction. In certain embodiments, a one liter culture of cells expressing polypeptide of the invention will produce at least about 0.1, 0.2, 0.5, 1, 2, 5, 10, 20, 30, 40, 50 milligrams of more of soluble protein. In an exemplary embodiment, a polypeptide of the invention is at least about 10% soluble and will produce at least about 1 milligram of protein from a one liter cell culture.

The term "specifically hybridizes" refers to detectable and specific nucleic acid binding. Polynucleotides, oligonucleotides and nucleic acids of the invention selectively hybridize to nucleic acid strands under hybridization and wash conditions that minimize appreciable amounts of detectable binding to nonspecific nucleic acids. Stringent conditions may be used to achieve selective hybridization conditions as known in the art and discussed herein. Generally, the nucleic acid sequence homology between the polynucleotides, oligonucleotides, and nucleic acids of the invention and a nucleic acid sequence of interest will be at least 30%, 40%, 50%, 60%, 70%, 80%, 85%, 90%, 95%, 98%, 99%, or more. In certain instances, hybridization and washing conditions are performed under stringent conditions according to conventional hybridization procedures and as described further herein.

The terms "stringent conditions" or "stringent hybridization conditions" refer to conditions which promote specific hybridization between two complementary polynucleotide strands so as to form a duplex: Stringent conditions may be selected to be about 5°C lower than the thermal melting point (Tm) for a given polynucleotide duplex at a defined ionic strength and pH. The length of the complementary polynucleotide strands and their GC content will determine the Tm of the duplex, and thus the hybridization conditions necessary for obtaining a desired specificity of hybridization. The Tm is the temperature (under defined ionic strength and pH) at which 50% of a polynucleotide sequence hybridizes to a perfectly matched complementary strand. In certain cases it may be desirable to increase the stringency of the hybridization conditions to be about equal to the Tm for a particular duplex.

A variety of techniques for estimating the Tm are available. Typically, G-C base pairs in a duplex are estimated to contribute about 3°C to the Tm, while A-T base pairs are estimated to contribute about 2°C, up to a theoretical maximum of about 80-100°C.

However, more sophisticated models of Tm are available in which G-C stacking interactions, solvent effects, the desired assay temperature and the like are taken into account. For example, probes can be designed to have a dissociation temperature (Td) of approximately 60°C, using the formula: Td = (((3 x #GC) + (2 x #AT)) x 37) - 562)/#bp) - 5; where #GC, #AT, and #bp are the number of guanine-cytosine base pairs, the number of adenise-thymine base pairs, and the number of total base pairs, respectively, involved in the formation of the duplex.

Hybridization may be carried out in 5x SSC, 4x SSC, 3x SSC, 2x SSC, 1x SSC or 0.2xSSC for at least about 1 hour, 2 hours, 5 hours, 12 hours, or 24 hours. The temperature of the hybridization may be increased to adjust the stringency of the reaction, for example, from about 25°C (room temperature), to about 45°C, 50°C, 55°C, 60°C, or 65°C. The hybridization reaction may also include another agent affecting the stringency, for example, hybridization conducted in the presence of 50% formamide increases the stringency of hybridization at a defined temperature.

The hybridization reaction may be followed by a single wash step, or two or more wash steps, which may be at the same or a different salinity and temperature. For example, the temperature of the wash may be increased to adjust the stringency from about 25°C (room temperature), to about 45°C, 50°C, 55°C, 60°C, 65°C, or higher. The wash step may be conducted in the presence of a detergent, *e.g.*, 0.1 or 0.2% SDS. For example, hybridization may be followed by two wash steps at 65°C each for about 20 minutes in 2x SSC, 0.1% SDS, and optionally two additional wash steps at 65°C each for about 20 minutes in 0.2x SSC, (0.1%SDS.

Exemplary stringent hybridization conditions include overnight hybridization at 65°C in a solution containing 50% formamide, lOx Denhardt (0.2% Ficoll, 0.2% polyvinylpyrrolidone, 0.2% bovine serum albumin) and 200 μg/ml of denatured carrier DNA, *e.g.*, sheared salmon sperm DNA, followed by two wash steps at 65°C each for about 20 minutes in 2x SSC, 0.1% SDS, and two wash steps at 65°C each for about 20 minutes in 0.2x SSC, 0.1% SDS.

Hybridization may consist of hybridizing two nucleic acids in solution, or a nucleic acid in solution to a nucleic acid attached to a solid support, *e.g.*, a filter. When one nucleic acid is on a solid support, a prehybridization step may be conducted prior to hybridization. Prehybridization may be carried out for at least about 1 hour, 3 hours or 10 hours in the same solution and at the same temperature as the hybridization solution (without the complementary polynucleotide strand).

Appropriate stringency conditions are known to those skilled in the art or may be determined experimentally by the skilled artisan. See, for example, Current Protocols in Molecular Biology, John Wiley & Sons, N.Y. (1989), 6.3.1-12.3.6; Sambrook et al., 1989, Molecular Cloning, A Laboratory Manual, Cold Spring Harbor Press, N.Y.; S. Agrawal (ed.) Methods in Molecular Biology, volume 20; Tijssen (1993) Laboratory Techniques in Biochemistry and Molecular Biology - Hybridization With Nucleic Acid Probes, e.g., part I chapter 2 "Overview of principles of hybridization and the strategy of nucleic acid probe assays", Elsevier, New York; and Tibanyenda, N. et al., Eur. J. Biochem. 139:19 (1984) and Ebel, S. et al., Biochem, 31:12083 (1992).

The term "vector" refers to a nucleic acid capable of transporting another nucleic acid to which it has been linked. One type of vector which may be used in accord with the invention is an episome, i.e., a nucleic acid capable of extra-chromosomal replication. Other vectors include those capable of autonomous replication and expression of nucleic acids to which they are linked. Vectors capable of directing the expression of genes to which they are operatively linked are referred to herein as "expression vectors" In general, expression vectors of utility in recombinant DNA techniques are often in the form of "plasmids" which refer to circular double stranded DNA molecules which, in their vector form are not bound to the chromosome. In the present specification, "plasmid" and "vector" are used interchangeably as the plasmid is the most commonly used form of vector. However, the invention is intended to include such other forms of expression vectors which serve equivalent functions and which become known in the art subsequently hereto.

The nucleic acids of the invention may be used as diagnostic reagents to detect the presence or absence of the target DNA or RNA sequence to which they specifically bind, such as for determining the level of expression of a nucleic acid of the invention. In one aspect, the present invention contemplates a method for detecting the presence of a nucleic acid of the invention or a portion thereof in a sample, the method of the steps of: (a) providing an oligonucleotide at least eight nucleotides in length, the oligonucleotide being complementary to a portion of a nucleic acid of the invention, (b) contacting the oligonucleotide with a sample containing at least one nucleic acid under conditions that permit hybridization of the oligonucleotide with a nucleic acid of the invention or a portion thereof, and (c) detecting hybridization of the oligonucleotide to a nucleic acid in the sample, thereby detecting the presence of a nucleic acid of the invention or a portion thereof in the sample. In another aspect, the present invention contemplates a method for detecting the presence of a nucleic acid of the invention or a portion thereof in a sample, by (a) providing a pair of single stranded oligonucleotides, each of which is at least eight nucleotides in length, complementary to sequences of a nucleic acid of the invention, and wherein the sequences to which the oligonucleotides are complementary are at least ten nucleotides apart; and (b) contacting the oligonucleotides with a sample containing at least one nucleic acid under hybridization conditions; (c) amplifying the nucleotide sequence between the two oligonucleotide primers; and (d) detecting the presence of the amplified sequence, thereby detecting the presence of a nucleic acid of the invention or a portion thereof in the sample.

In another aspect of the invention, the polynucleotide of the invention is provided in an expression vector containing a nucleotide sequence encoding a polypeptide of the invention and operably linked to at least one regulatory sequence. It should be understood that the design of the expression vector may depend on such factors as the choice of the host cell to be transformed and/or the type of protein desired to be expressed. The vector's copy number, the ability to control that copy number and the expression of any other protein encoded by the vector, such as antibiotic markers, should be considered.

An expression vector containing the polynucleotide of the invention can then be used as a pharmaceutical agent to treat an animal infected with *B. hyodysenteriae* or as a vaccine (also a pharmaceutical agent) to prevent an animal from being infected with *B. hyodysenteriae*, or to reduce the symptoms and course of the disease if the animal does become infected. One manner of using an expression vector as a pharmaceutical agent is to administer a nucleic acid vaccine to the animal at risk of being infected or to the animal after being infected. Nucleic acid vaccine technology is well-described in the art. Some descriptions can be found in U.S. Patent 6,562,376 (Hooper et al.); U.S. Patent 5,589,466 (Felgner, et al.); U.S. Patent 6,673,776 (Felgner, et al.); and U.S. Patent 6,710,035 (Feigner, et al.). Nucleic acid vaccines can be injected into muscle or intradermally, can be electroporated into the animal (see WO 01/23537, King et al.; and WO 01/68889, Malone et al.), via lipid compositions (see U.S. Patent 5,703,055, Felgner, et al), or other mechanisms known in the art field.

Expression vectors can also be transfected into bacteria which can be administered to the target animal to induce an immune response to the protein encoded by the nucleotides of this invention contained on the expression vector. The expression vector can contain eukaryotic expression sequences such that the nucleotides of this invention are transcribed and translated in the host animal. Alternatively, the expression vector can be transcribed in the bacteria and then translated in the host animal. The bacteria used as a carrier of the expression vector should be attenuated but still invasive. One can use *Shigella spp., Salmonella spp., Escherichia spp*., and *Aeromonas spp*., just to name a few, that have been attenuated but still invasive. Examples of these methods can be found in U.S. Patent 5,824,538 (Branstrom et al); U.S. Patent 5,877,159 (Powell, et al.); U.S. Patent 6,150,170 (Powell, et al.); U,S. Patent 6,500,419 (Hone, et al. ); and U.S. Patent 6,682,729 (Powell, et al.).

Alternatively, the polynucleotides of this invention can be placed in certain viruses which act a vector. Viral vectors can either express the proteins of this invention on the surface of the virus, or carry polynucleotide of this invention into an animal cell where the polynucleotide is transcribed and translated into a protein. The animal infected with the viral vectors can develop an immune response to the proteins encoded by the polynucleotides of this invention. Thereby one can alleviate or prevent an infection by *B. hyodysenteriae* in the animal which received the viral vectors. Examples of viral vectors can be found U.S. Patent 5,283,191 (Morgan et al.); U.S. Patent 5,554,525 (Sondermeijer et al) and U.S. Patent 5,712,118 (Murphy).

The polynucleotide of the invention may be used to cause expression and over-expression of a polypeptide of the invention in cells propagated in culture, *e.g.* to produce proteins or polypeptides, including fusion proteins or polypeptides.

This invention pertains to a host cell transfected with a recombinant gene in order to express a polypeptide of the invention. The host cell may be any prokaryotic or eukaryotic cell. For example, a polypeptide of the invention may be expressed in bacterial cells, such as *E. coli*, insect cells (baculovirus), yeast, plant, or mammalian cells. In those instances when the host cell is human, it may or may not be in a live subject. Other suitable host cells are known to those skilled in the art. Additionally, the host cell may be supplemented with tRNA molecules not typically found in the host so as to optimize expression of the polypeptide. Alternatively, the nucleotide sequence may be altered to optimize expression in the host cell, yet the protein produced would have high homology to the originally encoded protein. Other methods suitable for maximizing expression of the polypeptide will be known to those in the art.

The present invention further pertains to methods of producing the polypeptides of the invention. For example, a host cell transfected with an expression vector encoding a polypeptide of the invention may be cultured under appropriate conditions to allow expression of the polypeptide to occur. The polypeptide may be secreted and isolated from a mixture of cells and medium containing the polypeptide. Alternatively, the polypeptide may be retained cytoplasmically and the cells harvested, lysed and the protein isolated.

A cell culture includes host cells, media and other byproducts. Suitable media for cell culture are well known in the art. The polypeptide may be isolated from cell culture medium, host cells, or both using techniques known in the art for purifying proteins, including ion-exchange chromatography, gel filtration chromatography, ultrafiltration, electrophoresis, and immunoaffinity purification with antibodies specific for particular epitopes of a polypeptide of the invention.

Thus, a nucleotide sequence encoding all or a selected portion of polypeptide of the invention, may be used to produce a recombinant form of the protein via microbial or eukaryotic cellular processes. Ligating the sequence into a polynucleotide construct, such as an expression vector, and transforming or transfecting into hosts, either eukaryotic (yeast, avian, insect or mammalian) or prokaryotic (bacterial cells), are standard procedures. Similar procedures, or modifications thereof, may be employed to prepare recombinant polypeptides of the invention by microbial means or tissue- culture technology.

Suitable vectors for the expression of a polypeptide of the invention include plasmids of the types: pTreHis-derived plasmids, pET-derived plasmids, pBR322-derived plasmids, pEMBL-derived plasmids, pEX-derived plasmids, pBTac-derived plasmids and pUC-derived plasmids for expression in prokaryotic cells, such as *E. coli.* The various methods employed in the preparation of the plasmids and transformation of host organisms are well known in the art. For other suitable expression systems for both prokaryotic and eukaryotic cells, as well as general recombinant procedures, see Molecular Cloning, A Laboratory Manual, 2nd Ed., ed. by Sambrook, Fritsch and Maniatis (Cold Spring Harbor Laboratory Press, 1989) Chapters 16 and 17.

Coding sequences for a polypeptide of interest may be incorporated as a patt of a fusion gene including a nucleotide sequence encoding a different polypeptide. The present invention contemplates an isolated polynucleotide containing a nucleic acid of the invention and at least one heterologous sequence encoding a heterologous peptide linked in frame to the nucleotide sequence of the nucleic acid of the invention so as to encode a fusion protein containing the heterologous polypeptide. The heterologous polypeptide may be fused to (a) the C-terminus of the polypeptide of the invention, (b) the N-terminus of the polypeptide of the invention, or (c) the C-terminus and the N-terminus of the polypeptide of the invention. In certain instances, the heterologous sequence encodes a polypeptide permitting the detection, isolation, solubilization and/or stabilization of the polypeptide to which it is fused. In still other embodiments, the heterologous sequence encodes a polypeptide such as a poly His tag, myc, HA, GST, protein A, protein G, cahnodulin-binding peptide, thioredoxin, maltose-binding protein, poly arginine, poly His-Asp, FLAG, a portion of an immunoglobulin protein, and a transcytosis peptide.

Fusion expression systems can be useful when it is desirable to produce an immunogenic fragment of a polypeptide of the invention. For example, the VP6 capsid protein of rotavirus may be used as an immunologic carrier protein for portions of polypeptide, either in the monomeric form or in the form of a viral particle. The nucleic acid sequences corresponding to the portion of a polypeptide of the invention to which antibodies are to be raised may be incorporated into a fusion gene construct which includes coding sequences for a late vaccinia virus structural protein to produce a set of recombinant viruses expressing fusion proteins comprising a portion of the protein as part of the virion. The Hepatitis B surface antigen may also be utilized in this role as well. Similarly, chimeric constructs coding for fusion proteins containing a portion of a polypeptide of the invention and the poliovirus capsid protein may be created to enhance immunogenicity (see, for example, EP Publication NO: 0259149; and Evans et al., (1989) Nature 339:385; Huang et al., (1988) J. Virol. 62:3855; and Schlienger et al., (1992) J. Virol. 66:2).

Fusion proteins may facilitate the expression and/or purification of proteins. For example, a polypeptide of the invention may be generated as a glutathione-S-transferase (GST) fusion protein. Such GST fusion proteins may be used to simplify purification of a polypeptide of the invention, such as through the use of glutathione-derivatized matrices (see, for example, Current Protocols in Molecular Biology, eds. Ausubel et al (N.Y.: John Wiley & Sons, 1991)). In another embodiment, a fusion gene coding for a purification leader sequence, such as a poly-(His)/enterokinase cleavage site sequence at the N-terminus of the desired portion of the recombinant protein, may allow purification of the expressed fusion protein by affinity chromatography using a $Ni^{2+}$ metal resin. The purification leader sequence may then be subsequently removed by treatment with enterokinase to provide the purified protein (e.g., see Hochuli et al., (1987) J. Chromatography 411: 177; and Janknecht et al., PNAS USA 88:8972).

Techniques for making fusion genes are well known. Essentially, the joining of various DNA, fragments coding for different polypeptide sequences is performed in accordance with conventional techniques, employing blunt-ended or stagger-ended termini for ligation, restriction enzyme digestion to provide for appropriate termini, filling-in of cohesive ends as appropriate, alkaline phosphatase treatment to avoid undesirable jointing, and enzymatic ligation. In another embodiment, the fusion gene may be synthesized by conventional techniques including automated DNA synthesizers. Alternatively, PCR amplification of gene fragments may be carried out using anchor primers which give rise to complementary overhangs between two consecutive gene fragments which may subsequently be annealed to generate a chimeric gene sequence (see, for example, Current Protocols in Molecular Biology, eds. Ausubel et al., John Wiley & Sons: 1992).

In other embodiments, the invention provides for nucleic acids of the invention immobilized onto a solid surface, including, plates, microtiter plates, slides, beads, particles, spheres, films, strands, precipitates, gels, sheets, tubing, containers, capillaries, pads, slices, *etc.* The nucleic acids of the invention may be immobilized onto a chip as part of an array. The array may contain one or more polynucleotides of the invention as described herein. In one embodiment, the chip contains one or more polynucleotide of the invention as part of an array of polynucleotide sequences from the same pathogenic species as such polynucleotide(s).

In a preferred form of the invention there is provided isolated *B. hyodysenteriae* polypeptides as herein described, and also the polynucleotide sequences encoding these polypeptides. More desirably the *B. hyodysenteriae* polypeptides are provided in substantially purified form.

Preferred polypeptides of the invention will have one or more biological properties (e.g., *in vivo, in vitro* or immunological properties) of the native full-length polypeptide. Non-functional polypeptides are also included within the scope of the invention because they may be useful, for example, as antagonists of the functional polypeptides. The biological properties of analogues, fragments, or derivatives relative to wild type may be determined, for example, by means of biological assays.

Polypeptides, including analogues, fragments and derivatives, can be prepared synthetically (*e.g.*, using the well known techniques of solid phase or solution phase peptide synthesis). Preferably, solid phase synthetic techniques are employed. Alternatively, the polypeptides of the invention can be prepared using well known genetic engineering techniques, as described *infra.* In yet another embodiment, the polypeptides can be purified (*e.g.*, by immunoaffinity purification) from a biological fluid, such as but not limited to plasma, faeces, serum, or urine from animals, including, but not limited to, pig, chicken, goose, duck, turkey, parakeet, human, monkey, dog, cat, horse, hamster, gerbil, rabbit, ferret, horse, cattle, and sheep. An animal can be any mammal or bird.

The *B. hyodysenteriae* polypeptide analogues include those polypeptides having the amino acid sequence, wherein one or more of the amino acids are substituted with another amino acid which substitutions do not substantially alter the biological activity of the molecule.

According to the invention, the polypeptides of the invention produced recombinantly or by chemical synthesis and fragments or other derivatives or analogues thereof, including fusion proteins, may be used as an immunogen to generate antibodies that recognize the polypeptides.

A molecule is "antigenic" when it is capable of specifically interacting with an antigen recognition molecule of the immune system, such as an immunoglobulin (antibody) or T cell antigen receptor. An antigenic amino acid sequence contains at least about 5, and preferably at least about 10, amino acids. An antigenic portion of a molecule can be the portion that is immunodominant for antibody or T cell receptor recognition, or it can be a portion used to generate an antibody to the molecule by conjugating the antigenic portion to a carrier molecule for immunization. A molecule that is antigenic need not be itself immunogenic, *i.e.*, capable of eliciting an immune response without a carrier.

An "antibody" is any immunoglobulin, including antibodies and fragments thereof, that binds a specific epitope. The term encompasses polyclonal, monoclonal, and chimeric antibodies, the last mentioned described in further detail in U.S. Patent Nas. 4,816,397 and 4,816,567, as well as antigen binding portions of antibodies, including Fab, $F(ab')_2$ and F(v) (including single chain antibodies). Accordingly, the phrase "antibody molecule" in its various grammatical forms as used herein contemplates both an intact immunoglobulin molecule and an immunologically active portion of an immunoglobulin molecule containing the antibody combining site. An "antibody combining site" is that structural portion of an antibody molecule comprised of heavy and light chain variable and hypervariable regions that specifically binds an antigen.

Exemplary antibody molecules are intact immunoglobulin molecules, substantially intact immunoglobulin molecules and those portions of an immunoglobulin molecule that contain the paratope, including those portions known in the art as Fab, Fab', $F(ab')_2$ and F(v), which portions are preferred for use in the therapeutic methods described herein.

Fab and $F(ab')_2$ portions of antibody molecules are prepared by the proteolytic reaction of papain and pepsin, respectively, on substantially intact antibody molecules by methods that are well-known. See for example, U.S. Patent No. 4,342,566 to Theofilopolous et al. Fab' antibody molecule portions are also well-known and are produced from $F(ab')_2$ portions followed by reduction with mercaptoethanol of the disulfide bonds linking the two heavy chain portions, and followed by alkylation of the resulting protein mercaptan with a reagent such as iodoacetamide. An antibody containing intact antibody molecules is preferred herein.

The phrase "monoclonal antibody" in its various grammatical forms refers to an antibody having only one species of antibody combining site capable of immunoreacting with a particular antigen. A monoclonal antibody thus typically displays a single binding affinity for any antigen with which it immunoreacts. A monoclonal antibody may therefore contain an antibody molecule having a plurality of antibody combining sites, each immunospecific for a different antigen; *e.g.*, a bispecific (chimeric) monoclonal antibody.

The term "adjuvant" refers to a compound or mixture that enhances the immune response to an antigen. An adjuvant can serve as a tissue depot that slowly releases the antigen and also as a lymphoid system activator that non-specifically enhances the immune response [Hood et al., in Immunology, p. 384, Second Ed., Benjamin/Cummings, Menlo Park, California (1984)]. Often, a primary challenge with an antigen alone, in the absence of an adjuvant, will fail to elicit a humoral or cellular immune response. Adjuvants include, but are not limited to, complete Freund's adjuvant, incomplete Freund's adjuvant, saponin, mineral gels such as aluminium hydroxide, surface active substances such as lysolecithin, pluronic polyols, polyanions, peptides, oil or hydrocarbon emulsions, keyhole limpet hemocyanins, dinitrophenol, and potentially useful human adjuvants such as BCG (*bacille Calmette-Guerin*) and *Corynebacterium parvum.* Preferably, the adjuvant is pharmaceutically acceptable.

Various procedures known in the art may be used for the production of polyclonal antibodies to the polypeptides of the invention. For the production of antibody, various host animals can be immunised by injection with the polypeptide of the invention, including but not limited to rabbits, mice, rats, sheep, goats, *etc.* In one embodiment, a polypeptide of the invention can be conjugated to an immunogenic carrier, *e.g.*, bovine serum albumin (BSA) or keyhole limpet hemocyanin (KLH). Various adjuvants may be used to increase the immunological response, depending on the host species, including but not limited to Freund's (complete and incomplete), mineral gels such as aluminum hydroxide, surface active substances such as lysolecithin, pluronic polyols, polyanions, peptides, oil emulsions, keyhole limpet hemocyanins, dinitrophenol, and potentially useful human adjuvants such as BCG (*bacille Calmette-Guerin*) and *Corynebacterium parvum.*

For preparation of monoclonal antibodies directed toward a polypeptide of the invention, any technique that provides for the production of antibody molecules by continuous cell lines in culture may be used. These include but are not limited to the hybridoma technique originally developed by Kohler et al., (1975) Nature, 256:495-497, the trioma technique, the human B-cell hybridoma technique [Kozbor et al., (1983) Immunology Today, 4:72], and the EBV-hybridoma technique to produce human monoclonal antibodies [Cole et al., (1985) in Monoclonal Antibodies and Cancer Therapy, pp. 77-96, Alan R. Liss, Inc.]. Immortal, antibody-producing cell lines can be created by techniques other than fusion, such as direct transformation of B lymphocytes with oncogenic DNA, or transfection with Epstein-Barr virus. *See, e.g.*, U.S. Patent Nos. 4,341,761; 4,399,121; 4,427,783; 4,444,887; 4,451,570; 4,466,917; 4,472,500; 4,491,632; and 4,493,890.

In an additional embodiment of the invention, monoclonal antibodies can be produced in germ-free animals utilising recent technology. According to the invention, chicken or swine antibodies may be used and can be obtained by using chicken or swine hybridomas or by transforming B cells with EBV virus *in vitro.* In fact, according to the invention, techniques developed for the production of "chimeric antibodies" [Morrison et al., (1984) J. Bacteriol., 159-870; Neuberger et al., (1984) Nature, 312:604-608; Takeda et al., (1985) Nature, 314:452-454] by splicing the genes from a mouse antibody molecule specific for a polypeptide of the invention together with genes from an antibody molecule of appropriate biological activity can be used; such antibodies are within the scope of this invention. Such chimeric antibodies are preferred for use in therapy of intestinal diseases or disorders (described *infra*), since the antibodies are much less likely than xenogenic antibodies to induce an immune response, in particular an allergic response, themselves.

According to the invention, techniques described for the production of single chain antibodies (U.S. Patent 4,946,778) can be adapted to produce single chain antibodies specific for an polypeptide of the invention. An additional embodiment of the invention utilises the techniques described for the construction of Fab expression libraries [Huse et al., (1989) Science, 246:1275-128] to allow rapid and easy identification of monoclonal Fab fragments with the desired specificity for a polypeptide of the invention.

Antibody fragments, which contain the idiotype of the antibody molecule, can be generated by known techniques. For example, such fragments include but are not limited to: the $F(ab')_2$ fragment which can be produced by pepsin digestion of the antibody molecule; the Fab' fragments which can be generated by reducing the disulfide bridges of the $F(ab')_2$ fragment, and the Fab fragments which can be generated by treating the antibody molecule with papain and a reducing agent.

In the production of antibodies, screening for the desired antibody can be accomplished by techniques known in the art, *e.g.*, radioimmunoassay, ELISA, "sandwich" immunoassays, immunoradiometric assays, gel diffusion precipitin reactions, immunodiffusion assays, *in situ* immunoassays (using colloidal gold, enzyme or radioisotope labels, for example), Western blots, precipitation reactions, agglutination assays (*e.g.*, gel agglutination assays, hemagglutination assays), complement fixation assays, immunofluorescence assays, protein A assays, immunoelectrophoresis assays, *etc.* In one embodiment, antibody binding is detected by detecting a label on the primary antibody. In another embodiment, the primary antibody is detected by detecting binding of a secondary antibody or reagent to the primary antibody. In a further embodiment, the secondary antibody is labelled. Many means are known in the art for detecting binding in an immunoassay and are within the scope of the present invention. For example, to select antibodies that recognise a specific epitope of a polypeptide of the invention, one may assay generated hybridomas for a product that binds to a fragment of a polypeptide of the invention containing such epitope.

The invention also covers diagnostic and prognostic methods to detect the presence of *B. hyodysenteriae* using a polypeptide of the invention and/or antibodies which bind to the polypeptide of the invention and kits useful for diagnosis and prognosis of *B. hyodysenteriae* infections.

Diagnostic and prognostic methods will generally be conducted using a biological sample obtained from an animal, such as chicken or swine. A "sample" refers to an animal's tissue or fluid suspected of containing a *Brachyspira* species, such as *B. hyodysenteriae,* or its polynucleotides or its polypeptides. Examples of such tissue or fluids include, but not limited to, plasma, serum, faecal material, urine, lung, heart, skeletal muscle, stomach, intestines, and *in vitro* cell culture constituents.

The invention provides methods for detecting the presence of a polypeptide of the invention in a sample, with the following steps: (a) contacting a sample suspected of containing a polypeptide of the invention with an antibody (preferably bound to a solid support) that specifically binds to the polypeptide of the invention under conditions which allow for the formation of reaction complexes comprising the antibody and the polypeptide of the invention; and (b) detecting the formation of reaction complexes comprising the antibody and polypeptide of the invention in the sample, wherein detection of the formation of reaction complexes indicates the presence of the polypeptide of the invention in the sample.

Preferably, the antibody used in this method is derived from an affinity-purified polyclonal antibody, and more preferably a monoclonal antibody. In addition, it is preferable for the antibody molecules used herein be in the form of Fab, Fab', $F(ab')_2$ or F(v) portions or whole antibody molecules.

Particularly preferred methods for detecting *B. hyodysenteriae* based on the above method include enzyme linked immunosorbent assays, radioimmunoassays, immunoradiometric assays and immunoenzymatic assays, including sandwich assays using monoclonal and/or polyclonal antibodies.

Three such procedures that are especially useful utilise either polypeptide of the invention (or a fragment thereof) labelled with a detectable label, antibody $Ab_1$ labelled with a detectable label, or antibody $Ab_2$ labelled with a detectable label. The procedures may be summarized by the following equations wherein the asterisk indicates that the particle is labelled and "AA" stands for the polypeptide of the invention:

$$\text{A. } AA^* + Ab_1 = AA^*Ab_1$$

$$\text{B. } AA + Ab^*_1 = AA\ Ab_1^*$$

$$\text{C. } AA + Ab_1 + Ab_2^* = Ab_1\ AA\ Ab_2^*$$

The procedures and their application are all familiar to those skilled in the art and accordingly may be utilised within the scope of the present invention. The "competitive" procedure, Procedure A, is described in U.S. Patent Nos. 3,654,090 and 3,850,752. Procedure B is representative of well-known competitive assay techniques. Procedure C, the "sandwich" procedure, is described in U.S. Patent Nos. RE 31,006 and 4,016,043. Still other procedures are known, such as the "double antibody" or "DASP" procedure, and can be used.

In each instance, the polypeptide of the invention form complexes with one or more antibody(ies) or binding partners and one member of the complex is labelled with a detectable label. The fact that a complex has formed and, if desired, the amount thereof, can be determined by known methods applicable to the detection of labels.

It will be seen from the above, that a characteristic property of $Ab_2$ is that it will react with $Ab_1$. This reaction is because $Ab_1$, raised in one mammalian species, has been used in another species as an antigen to raise the antibody, $Ab_2$. For example, $Ab_2$ may be raised in goats using rabbit antibodies as antigens. $Ab_2$ therefore would be anti-rabbit antibody raised in goats. For purposes of this description and claims, $Ab_1$ will be referred to as a primary antibody, and $Ab_2$ will be referred to as a secondary or anti-$Ab_1$ antibody.

The labels most commonly employed for these studies are radioactive elements, enzymes, chemicals that fluoresce when exposed to ultraviolet light, and others. Examples of fluorescent materials capable of being utilised as labels include fluorescein, rhodamine and auramine. A particular detecting material is anti-rabbit antibody prepared in goats and conjugated with fluorescein through an isothiocyanate. Examples of preferred isotope include $^{3}H$, $^{14}C$, $^{32}P$, $^{35}S$, $^{36}Cl$, $^{51}Cr$, $^{57}Co$, $^{58}Co$, $^{59}Fe$, $^{90}Y$, $^{125}I$, $^{131}I$, and $^{186}Re$. The radioactive label can be detected by any of the currently available counting procedures. While many enzymes can be used, examples of preferred enzymes are peroxidase, β-glucuronidase, β-D-glucosidase, β-D-galactosidase, urease, glucose oxidase plus peroxidase and alkaline phosphatase. Enzyme are conjugated to the selected particle by reaction with bridging molecules such as carbodiimides, diisocyanates, glutaraldehyde and the like. Enzyme labels can be detected by any of the presently utilized colorimetric, spectrophotometric, fluorospectrophotometric, amperometric or gasometric techniques. U.S. Patent Nos. 3,654,090; 3,850,752; and 4,016,043 are referred to by way of example for their disclosure of alternate labelling material and methods.

The invention also provides a method of detecting antibodies to a polypeptide of the invention in biological samples, using the following steps: (a) providing a polypeptide of the invention or a fragment thereof; (b) incubating a biological sample with said polypeptide of the invention under conditions which allow for the formation of an antibody-antigen complex; and (c) determining whether an antibody-antigen complex with the polypeptide of the invention is formed.

In another embodiment of the invention there are provided *in vitro* methods for evaluating the level of antibodies to a polypeptide of the invention in a biological sample using the following steps: (a) detecting the formation of reaction complexes in a biological sample according to the method noted above; and (b) evaluating the amount of reaction complexes formed, which amount of reaction complexes corresponds to the level of polypeptide of the invention in the biological sample.

Further there are provided *in vitro* methods for monitoring therapeutic treatment of a disease associated with *B. hyodysenteriae* in an animal host by evaluating, as describe above, the levels of antibodies to a polypeptide of the invention in a series of biological samples obtained at different time points from an animal host undergoing such therapeutic treatment.

The present invention further provides methods for detecting the presence or absence of *B. hyodysenteriae* in a biological sample by: (a) bringing the biological sample into contact with a polynucleotide probe or primer of polynucleotide of the invention under suitable hybridizing conditions; and (b) detecting any duplex formed between the probe or primer and nucleic acid in the sample.

According to one embodiment of the invention, detection *of B. hyodysenteriae* may be accomplished by directly amplifying polynucleotide sequences from biological sample, using known techniques and then detecting the presence of polynucleotide of the invention sequences.

In one form of the invention, the target nucleic acid sequence is amplified by PCR and then detected using any of the specific methods mentioned above. Other useful diagnostic techniques for detecting the presence of polynucleotide sequences include, but are not limited to: 1) allele-specific PCR; 2) single stranded conformation analysis; 3) denaturing gradient gel electrophoresis; 4) RNase protection assays; 5) the use of proteins which recognize nucleotide mismatches, such as the *E. coli* mutS protein; 6) allele-specific oligonucleotides; and 7) fluorescent *in situ* hybridisation.

In addition to the above methods polynucleotide sequences may be detected using conventional probe technology. When probes are used to detect the presence of the desired polynucleotide sequences, the biological sample to be analysed, such as blood or serum, may be treated, if desired, to extract the nucleic acids. The sample polynucleotide sequences may be prepared in various ways to facilitate detection of the target sequence; *e.g.* denaturation, restriction digestion, electrophoresis or dot blotting. The targeted region of the sample polynucleotide sequence usually must be at least partially single-stranded to form hybrids with the targeting sequence of the probe. If the sequence is naturally single-stranded, denaturation will not be required. However, if the sequence is double-stranded, the sequence will probably need to be denatured. Denaturation can be carried out by various techniques known in the art.

Sample polynucleotide sequences and probes are incubated under conditions that promote stable hybrid formation of the target sequence in the probe with the putative desired polynucleotide sequence in the sample. Preferably, high stringency conditions are used in order to prevent false positives.

Detection, if any, of the resulting hybrid is usually accomplished by the use of labelled probes. Alternatively, the probe may be unlabeled, but may be detectable by specific binding with a ligand that is labelled, either directly or indirectly. Suitable labels and methods for labelling probes and ligands are known in the art, and include, for example, radioactive labels which may be incorporated by known methods (e.g., nick translation, random priming or kinasing), biotin, fluorescent groups, chemiluminescent groups (e.g., dioxetanes, particularly triggered dioxetanes), enzymes, antibodies and the like. Variations of this basic scheme are known in the art, and include those variations that facilitate separation of the hybrids to be detected from extraneous materials and/or that amplify the signal from the labelled moiety.

It is also contemplated within the scope of this invention that the nucleic acid probe assays of this invention may employ a cocktail of nucleic acid probes capable of detecting the desired polynucleotide sequences of this invention. Thus, in one example to detect the presence of polynucleotide sequences of this invention in a cell sample, more than one probe complementary to a polynucleotide sequences is employed and in particular the number of different probes is alternatively 2, 3, or 5 different nucleic acid probe sequences.

The polynucleotide sequences described herein (preferably in the form of probes) may also be immobilised to a solid phase support for the detection of *Brachyspira* species, including but not limited to *B. hyodysenteriae, B. intermedia, B. alvinipulli, B. aalborgi, B. innocens, B. murdochii, and B. pilosicoli.* Alternatively the polynucleotide sequences described herein will form part of a library of DNA molecules that may be used to detect simultaneously a number of different genes from *Brachyspira* species, such as *B. hyodysenteriae.* In a further alternate form of the invention polynucleotide sequences described herein together with other polynucleotide sequences (such as from other bacteria or viruses) may be immobilised on a solid support in such a manner permitting identification of the presence of a *Brachyspira* species, such as *B. hyodysenteriae* and/or any of then other polynucleotide sequences bound onto the solid support.

Techniques for producing immobilised libraries of DNA molecules have been described in the art. Generally, most prior art methods describe the synthesis of single-stranded nucleic acid molecule libraries, using for example making techniques to build up various permutations of sequences at the various discrete positions on the solid substrate. U.S. Patent No. 5,837,832 describes an improved method for producing DNA arrays immobilised to silicon substrates based on very large scale integration technology. In particular, U.S. Patent No. 5,837,832 describes a strategy called "tiling" to synthesize specific sets of probes at spatially defined locations on a substrate that may be used to produced the immobilised DNA libraries of the present invention. U.S. Patent No. 5,837,832 also provides references for earlier techniques that may also be used. Thus polynucleotide sequence probes may be synthesised *in situ* on the surface of the substrate.

Alternatively, single-stranded molecules may be synthesised off the solid substrate and each pre-formed sequence applied to a discrete position on the solid substrate. For example, polynucleotide sequences may be printed directly onto the substrate using robotic devices equipped with either pins or pizo electric devices.

The library sequences are typically immobilised onto or in discrete regions of a solid substrate. The substrate may be porous to allow immobilisation within the substrate or substantially non-porous, in which case the library sequences are typically immobilised on the surface of the substrate. The solid substrate may be made of any material to which polypeptides can bind, either directly or indirectly. Examples of suitable solid substrates include flat glass, silicon wafers, mica, ceramics and organic polymers such as plastics, including polystyrene and polymethacrylate. It may also be possible to use semi-permeable membranes such as nitrocellulose or nylon membranes, which are widely available. The semi-permeable membranes may be mounted on a more robust solid surface such as glass. The surfaces may optionally be coated with a layer of metal, such as gold, platinum or other transition metal.

Preferably, the solid substrate is generally a material having a rigid or semi-rigid surface. In preferred embodiments, at least one surface of the substrate will be substantially flat, although in some embodiments it may be desirable to physically separate synthesis regions for different polymers with, for example, raised regions or etched trenches. It is also preferred that the solid substrate is suitable for the high density application of DNA sequences in discrete areas of typically from 50 to 100 $\mu$m, giving a density of 10000 to 40000 dots/cm$^{-2}$.

The solid substrate is conveniently divided up into sections. This may be achieved by techniques such as photoetching, or by the application of hydrophobic inks, for example teflon-based inks (Cel-line, USA).

Discrete positions, in which each different member of the library is located may have any convenient shape, e.g., circular, rectangular, elliptical, wedge-shaped, etc.

Attachment of the polynucleotide sequences to the substrate may be by covalent or noncovalent means. The polynucleotide sequences may be attached to the substrate via a layer of molecules to which the library sequences bind. For example, the polynucleotide sequences may be labelled with biotin and the substrate coated with avidin and/or streptavidin. A convenient feature of using biotinylated polynucleotide sequences is that the efficiency of coupling to the solid substrate can be determined easily. Since the polynucleotide sequences may bind only poorly to some solid substrates, it is often necessary to provide a chemical interface between the solid substrate (such as in the case of glass) and the nucleic acid sequences. Examples of suitable chemical interfaces include hexaethylene glycol. Another example is the use of polylysine coated glass, the polylysine then being chemically modified using standard procedures to introduce an affinity ligand. Other methods for attaching molecules to the surfaces of solid substrate by the use of coupling agents are known in the art, see for example WO98/49557.

Binding of complementary polynucleotide sequences to the immobilised nucleic acid library may be determined by a variety of means such as changes in the optical characteristics of the bound polynucleotide sequence (*i.e.* by the use of ethidium bromide) or by the use of labelled nucleic acids, such as polypeptides labelled with fluorophores. Other detection techniques that do not require the use of labels include optical techniques such as optoacoustics, reflectometry, ellipsometry and surface plasmon resonance (see WO97/49989).

Thus, the present invention provides a solid substrate having immaobilized thereon at least one polynucleotide of the present invention, preferably two or more different polynucleotide sequences of the present invention.

The present invention also can be used as a prophylactic or therapeutic, which may be utilised for the purpose of stimulating humoral and cell mediated responses in animals, such as chickens and swine, thereby providing protection against colonisation with *Brachyspira* species, including but not limited to *B. hyodysenteriae, B. intermedia, B. alvinipulli, B. aalborgi, B. innocens, B. murdochii, and B. pilosicoli.* Natural infection with a *Brachyspira* species, such as *B. hyodysenteriae* induces circulating antibody titres against the proteins described herein. Therefore, the amino acid sequences described herein or parts thereof, have the potential to form the basis of a systemically or orally administered prophylactic or therapeutic to provide protection against intestinal spirochaetosis.

Accordingly, in one embodiment the present invention provides the amino acid sequences described herein or fragments thereof or antibodies that bind the amino acid sequences or the polynucleotide sequences described herein in a therapeutically effective amount admixed with a pharmaceutically acceptable carrier, diluent, or excipient.

The phrase "therapeutically effective amount" is used herein to mean an amount sufficient to reduce by at least about 15%, preferably by at least 50%, more preferably by at least 90%, and most preferably prevent, a clinically significant deficit in the activity, function and response of the animal host. Alternatively, a therapeutically effective amount is sufficient to cause an improvement in a clinically significant condition in the animal host.

The phrase "pharmaceutically acceptable'" refers to molecular entities and compositions that are physiologically tolerable and do not typically produce an allergic or similarly untoward reaction, such as gastric upset and the like, when administered to an animal. The term "carrier" refers to a diluent, adjuvant, excipient, or vehicle with which the compound is administered. Such pharmaceutical carriers can be sterile liquids, such as water and oils, including those of petroleum, animal, vegetable or synthetic origin, such as peanut oil, soybean oil, mineral oil, sesame oil and the like. Water or saline solutions and aqueous dextrose and glycerol solutions are preferably employed as carriers, particularly for injectable solutions. Suitable pharmaceutical carriers are described in Martin, Remington's Pharmaceutical Sciences, 18th Ed., Mack Publishing Co., Easton, PA, (1990).

In a more specific form of the invention there are provided pharmaceutical compositions comprising therapeutically effective amounts of the amino acid sequences described herein or an analogue, fragment or derivative product thereof or antibodies thereto together with pharmaceutically acceptable diluents, preservatives, solubilizes, emulsifiers, adjuvants and/or carriers. Such compositions include diluents of various buffer content (*e.g.*, Tris-HCl, acetate, phosphate), pH and ionic strength and additives such as detergents and solubilizing agents (e.g., Tween 80, Polysorbate 80), anti-oxidants (*e.g.*, ascorbic acid, sodium metabisulfite), preservatives (*e.g.*, Thimersol, benzyl alcohol) and bulking substances (*e.g.*, lactose, mannitol). The material may be incorporated into particulate preparations of polymeric compounds such as polylactic acid, polyglycolic acid, etc. or into liposomes. Hylauronic acid may also be used. Such compositions may influence the physical state, stability, rate of *in vivo* release, and rate of *in vivo* clearance of the present proteins and derivatives. *See, e.g.*, Martin, Remington's Pharmaceutical Sciences, 18th Ed. (1990, Mack Publishing Co., Easton, PA 18042) pages 1435-1712 that are herein incorporated by reference. The compositions may be prepared in liquid form, or may be in dried powder, such as lyophilised form.

Alternatively, the polynucleotides of the invention can be optimized for expression in plants (*e.g.*, corn). The plant may be transformed with plasmids containing the optimized polynucleotides. Then the plant is grown, and the proteins of the invention are expressed in the plant, or the plant- optimized version is expressed. The plant is later harvested, and the section of the plant containing the proteins of the invention is processed into feed for the animal. This animal feed will impart immunity against *B. hyodysenteriae* when eaten by the animal. Examples of prior art detailing these methods can be found in U.S. Patent 5,914,123 (Arntzen, et al.); U.S. Patent 6,034,298 (Lam, et al.); and U.S. Patent 6,136,320 (Arntzen, et al.).

It will be appreciated that pharmaceutical compositions provided accordingly to the invention may be administered by any means known in the art. Preferably, the pharmaceutical compositions for administration are administered by injection, orally, or by the pulmonary, or nasal route. The amino acid sequences described herein or antibodies derived therefrom are more preferably delivered by intravenous, intraarterial, intraperitoneal, intramuscular, or subcutaneous routes of administration. Alternatively, the amino acid sequence described herein or antibodies derived therefrom, properly formulated, can be administered by nasal or oral administration.

In a specific embodiment of the invention, a vaccine composition may be a "combination vaccine" with one or more polynucleotide or polypeptide sequences of the invention which have a similar functional role that are combined in a vaccine. For example, in the present invention, four different functional groups whose components can be combined have been identified in Table 5.

A prerequisite for combination vaccines is a lack of competition (i.e. between antigens) and a high compatibility with respect to the subject to be immunized. According to international standard applied in connection with immunization, a combination vaccine should confer a protection which is comparable to that achieved by separate vaccinations. However, the combination of antigens is a complicated process which is associated with a number of problems and uncertainties.

As the individual components of a combination vaccine are not inert substances, the combination of various components into one mixture can negatively influence the immunogenicity of the individual antigenic components. For example, interactions between the different antigens or between the antigens and other components typically used in such vaccines can occur due to differences in charge, chemical residues, detergents, formaldehyde, concentration of ions etc. These changes can occur instantaneously after contacting the different antigens with each other or with other substances usually present in vaccine formulations. Moreover, these changes can also occur with a significant delay after mixing, for example during storage, shipping, etc. However, it cannot be predicted to which extent the immunogenicity of individual antigens may be influenced by mixing them to one combination. As a consequence, a situation can occur in which one or more antigens of the vaccine only confer an insufficient seroprotection against one or more of diseases which renders the product unsuitable for medical practice.

The production of effective combination vaccines is a complex matter which depends on multidimensional interactions between the individual components of the vaccine and docs not allow to extrapolate any effect of the components observed when administered separately.

It has been found that the combination vaccines according to the present invention may be combined with one or more further antigens and remain effective and stable.

The term "stable" as used in the context of the present invention means that the combination vaccine formulation can be kept for a period of eight days or more preferably 14 days at room temperature without any substantial loss with respect to immunogenity and stability of its distinct antigen compounds. In order to enhance stability, stabilizing agents such as saccharose can be added to the vaccine composition. Instead of saccharose or in addition thereto, other stabilizers, e.g. human serum albumin, mannose, trehalose, mannite, or polygeline can also be added as stabilizing agents. The vaccines of the present invention exhibit a pH value of preferably between 5.0 to 8.0, more preferably between 6.0 to 7.0, wherein a pH value of 6.8 to 7.8 is most preferred.

The term "effective" as used herein, refers to the fact that the sequence of the invention upon single or repeated administration to a subject (for example, a mammal) confers protection against a specific disease.

As used herein the term "conferring protection" means that the sequence of the invention, upon administeration, induces an immunological response in the vaccinated subject which response is capable to protect said subject from the symptoms of subsequent infection.

Also encompassed by the present invention is the use of polynucleotide sequences of the invention, as well as antisense and ribozyme polynucleotide sequences hybridisable to a polynucleotide sequence encoding an amino acid sequence according to the invention, for manufacture of a medicament for modulation of a disease associated *B. hyodysenteriae.*

Polynucleotide sequences encoding antisense constructs or ribozymes for use in therapeutic methods are desirably administered directly as a naked nucleic acid construct. Uptake of naked nucleic acid constructs by bacterial cells is enhanced by several known transfection techniques, for example those including the use of transfection agents. Example of these agents include cationic agents (for example calcium phosphate and DEAE-dextran) and lipofectants. Typically, nucleic acid constructs are mixed with the transfection agent to produce a composition.

Alternatively the antisense construct or ribozymes may be combined with a pharmaceutically acceptable carrier or diluent to produce a pharmaceutical composition. Suitable carriers and diluents include isotonic saline solutions, for example phosphate-buffered saline. The composition may be formulated for parenteral, intramuscular, intravenous, subcutaneous, intraocular, oral or transdermal administration. The routes of administration described are intended only as a guide since a skilled practitioner will be able to determine readily the optimum route of administration and any dosage for any particular animal and condition.

The invention also includes kits for screening animals suspected of being infected with a *Brachyspira* species, such as *B. hyodysenteriae* or to confirm that an animal is infected with a *Brachyspira* species, such as *B. hyodysenteriae.* In a further embodiment of this invention, kits suitable for use by a specialist may be prepared to determine the presence or absence of *Brachyspira* species, including but not limited to *B. hyodysenteriae, B. intermedia, B. alvinipulli. B. aalborgi, B. innocens, B. murdochii, and B. pilosicoli* in suspected infected animals or to quantitatively measure a *Brachyspira* species, including but not limited to *B. hyodysenteriae, B. intermedia, B. alvinipulli, B. aalborgi and B. pilosicoli* infection. In accordance with the testing techniques discussed above, such kits can contain at least a labelled version of one of thc amino acid sequences described herein or its binding partner, for instance an antibody specific thereto, and directions depending upon the method selected, e.g., "competitive," "sandwich," "DASP" and the like. Alternatively, such kits can contain at least a polynucleotide sequence complementary to a portion of one of the polynucleotide sequences described herein together with instructions for its use. The kits may also contain peripheral reagents such as buffers, stabilizers, *etc.*

Accordingly, a test kit for the demonstration of the presence of a *Brachyspira* species, including but not limited to *B. hyodysenteriae, B. intermedia, B. alvinipulli, B. aalborgi, B. innocens, B. murdochii, and B. pilosicoli,* may contain the following:

(a) a predetermined amount of at least one labelled immnunochemically reactive component obtained by the direct or indirect attachment of one of the amino acid sequences described herein or a specific binding partner thereto, to a detectable label;
(b) other reagents; and
(c) directions for use of said kit.

More specifically, the diagnostic test kit may contain:

(a) a known amount of one of the amino acid sequences described herein as described above (or a binding partner) generally bound to a solid phase to form an immunosorbent, or in the alternative, bound to a suitable tag, or there are a plural of such end products, etc;
(b) if necessary, other reagents; and

(c) directions for use of said test kit.

In a further variation, the test kit may contain:

(a) a labelled component which has been obtained by coupling one of the amino acid sequences described herein to a detectable label;
(b) one or more additional immunochemical reagents of which at least one reagent is a ligand or an immobilized ligand, which ligand is selected from the group consisting of:

(i) a ligand capable of binding with the labelled component (a);
(ii) a ligand capable of binding with a binding partner of the labelled component (a);
(iii) a ligand capable of binding with at least one of the component(s) to be determined; or
(iv) a ligand capable of binding with at least one of the binding partners of at least one of the component(s) to be determined; and

(c) directions for the performance of a protocol for the detection and/or determination of one or more components of an immunochemical reaction between one of the amino acid sequences described herein and a specific binding partner thereto.
The following examples are provided for the purposes of illustration and are not intended to limit the scope of the invention.

**Examples**

**Example 1: Genome sequencing**

An Australian porcine field isolate of B. hyodysenteriae (strain WA1) is shotgun sequenced. This strain has been well-characterised and shown to be virulent following experimental challenge of pigs. The spirochaete is grown in anaerobic trypticase soy broth culture and 100 $\mu$g DNA was extracted using a cetyltrimethylammonium bromide (CTAB) method to prepare high quality chromosomal DNA suitable for preparation of genomic DNA libraries. The genomic DNA is sheared using a GeneMachines Hydroshear, and the fragmented DNA processed for cloning as per the protocol recommended by the suppliers of the pSMART vector system (Lucigen). A small insert (2-3 kb) library and a medium insert (3-10 kb) library are constructed into the low copy version of the pSMART vector.

**Example 2: Annotation**

Partial genome sequences for B. hyodysenteriae are assembled and annotated by the Australian Genome Research Facility (AGRF) in Queensland and at Murdoch University by the Centre for Bioinformatics and Biological Computing (CBBC). The CBBC uses up-to-date mirrors of the major international databases and have developed leading edge bioinformatics software and strategies for annotation of features in large genome sequences. A range of public domain bioinformatics tools are used to analyse and re-analyse the sequences as part of a quality assurance procedure on data analysis. Open reading frames (ORFs) are predicted using a variety of programs including GeneMark, GLIMMER, ORPHEUS, SELFID and GetORF. Putative ORFs are examined for homology (DNA and protein) with existing international databases using searches including BLAST and FASTA. All the predicted ORFs are analysed to determine their cellular localisation using programs such as PSI-BLAST, FASTA, MOTIFS, FINDPATTERNS, PHD, SIGNALP and PSORT. Databases including Interpro, Prosite, ProDom, Pfam and Blocks are used to predict surface associated proteins such as transmembrane domains, leader peptides, homologies to know surface proteins, lipoprotein signature, outer membrane anchoring motifs and host cell binding domains. Phylogenetic and other molecular evolution analysis is conducted with the identified genes and with other species to assist in the assignment of function. The in silico analysis of both partially sequenced genomes has produced a comprehensive list of all the predicted ORFs present in the sequence data available. Each ORF is interrogated for descriptive information such as predicted molecular weight, isoelectric point, hydrophobicity, and subcellular localisation to enable correlation with the in vitro properties of the native gene product. Predicted genes which encode proteins similar to surface localized components and virulence factors in other pathogenic bacteria are selected as potential vaccine targets.

**Example 3- Analysis of gene distribution using polymerase chain reaction (PCR)**

One or two primer pairs which anneal to different regions of the target gene coding region are designed and optimised for PCR detection. Distribution analysis of the B. hyodysenteriae target genes is performed on 23 strains of B. hyodysenteriae, including two strains which have been shown to be avirulent. Primer sets used in the distribution analysis are shown in Table 2. PCR analysis is performed in a 25 μl total volume using Taq DNA polymerase. The amplification mixture consisted of 1x PCR buffer (containing 1.5 mM of $MgCl_2$), 1 U of Taq DNA polymerase, 0.2 mM of each dNTP, 0.5 μM of the primer pair, and 1 μl purified chromosomal template DNA. Cycling conditions involved an initial template denaturation step of 5 min at 94˚C, followed by 30 cycles of denaturation at 94˚C for 30 s, annealing at 50˚C for 15 s, and primer extension at 72˚C for 1 min. The PCR products are subjected to electrophoresis in 1% (w/v) agarose gels in 1x TAE buffer (40 mM Tris-acetate, 1 mM EDTA), stained with a 1 μg/ml ethidium bromide solution and viewed over ultraviolet (UV) light.

**Example 4- Bioinformatics**

Shot-gun sequencing of the B. hyodysenteriae genome resulted in 73% (2,347.8 kb out of a predicted 2,300 kb) of the genome to be sequenced. These sequences are comprised of 171 contigs with an average contig size of 13.7 kb. From the 171 contigs, 1,860 open-reading frames (ORFs) are predicted. Comparison of the predicted ORFs with genes present in the nucleic acid and protein databases indicated that approximately 70% in each species have homology with genes contained in the databases. The remaining 30% of the predicted ORFs from each genome have no known identity.

**Example 5 -Vaccine candidates**

ORFs showing strong homology with putative virulence-associated proteins present in the public databases are selected as vaccine targets. Table 1 shows the genes selected as vaccine targets and their similarity with other known genes.

**Example 6-Gene Distribution**

The overall gene distribution of each ORF is summarized in Table 4. A total of 23 B. hyodysenteriae strains are analysed. The distribution is determined from the cumulative result of PCR using up to two different primer sets. All of the ORFs are present in 87-100% of the B. hyodysenteriae strains tested.

**Example 7- Plasmid Extraction**

Escherichia coli JM109 clones harbouring the pET-19b plasmid (Novagen) are streaked out from glycerol stock storage onto Luria-Bertani (LB) agar plates supplemented with 100 mg/l ampicillin and incubated at 37˚C for 16 h. A single colony is used to inoculate 10 ml of LB broth supplemented with 100 mg/l ampicillin and the broth culture was incubated at 37˚C for 12 h with shaking. The entire overnight culture is centrifuged at 5,000 x g for 10 min and the plasmid contained in the cells extracted using the QIAprep Spin Miniprep Kit (Qiagen) according to the manufacturer's instructions. The purified plasmid is quantified using a fluorometer and the DNA concentration adjusted to 100 μg/ml by dilution with TE (10 mM Tris-HCl, 1 mM EDTA, pH 8.0) buffer. The purified pET-19b plasmid is stored at -20˚C.

**Example 8- Vector Preparation**

Two μg of the purified pET-19b plasmid is digested at 37˚C for 1-4 h in a total volume of 50 μl containing 50 mM Tris-HCl (pH 7.5), 100 mM NaCl, 10 mM MgCl2, 0.1 mg/ml bovine serum albumin (BSA) and 5 U of NdeI and BamHI (Fermentas). The linearised vectors are verified by electrophoresing 1 μl of the digestion reaction through a 1% (w/v) agarose gel in 1x TAE buffer at 90V for 1 h. The electrophoresed DNA is stained with 1 μg/ml ethidium bromide and viewed over UV light.

Linearised pET-19b vectors are purified using the UltraClean PCR Clean-up Kit (Mo Bio Laboratories) according to the manufacturer's instructions. The purified linear vectors are quantified using the NanoDrop ND1000 spectrophotemeter and the DNA concentration adjusted to 50 μg/ml by dilution with TE buffer. The linearised vectors are stored at -20˚C.

**Example 9- Insert Preparation**

**Primer design**

Primer pairs are designed to amplify as much of the coding region of the target gene as possible. All primers sequences include terminal restriction enzyme recognition sites to enable cohesive-end ligation of the resultant amplicon into the linearised pET-19b vector. The primer sequences used for cloning are shown in Table 3. The primers are tested using Amplify 3.0 (University of Wisconsin) and the theoretical amplicon sequence is inserted into the appropriate position in the pET-19b vector sequence. Deduced translation of the chimeric pET-19b expression cassette is performed using Victor NTI Advance version 10 (Invitrogen) to confirm that the gene inserts would be in the correct reading frame.

**Example 10- Amplification of the gene inserts**

All target gene inserts are amplified by PCR in a 100 μl total volume using Taq DNA polymerase and Pfu DNA polymerase. Briefly, the amplification mixture consists of 1 X PCR buffer (containing 1.5 mM of MgCl2), 2 U of Taq DNA polymerase, 0.01 U Pfu DNA polymerase, 0.2 mM of each dNTP, 0.5 μM of the appropriate primer pair and 1 μl of purified chromosomal DNA. The chromosomal DNA is prepared from the same B. hyodysenteriae strain used for genome sequencing. Cycling conditions involve an initial template denaturation step of 5 min at 94˚C, followed by 30 cycles of denaturation at 94˚C for 30 s, annealing at 50˚C for 15 s, and primer extension at 72˚C for 1 min. The PCR products are subjected to electrophoresis in 1% (w/v) agarose gels in 1x TAE buffer, stained with a 1 μg/ml ethidium bromide solution and viewed over UV light. After verifying the presence of the correct size PCR product, the PCR reaction is purified using the UltraClean PCR Clean-up Kit (Mo Bio Laboratories).

**Example 11- Restriction enzyme digestion of the gene inserts**

Thirty μl of the purified PCR product is digested in a 50 μl total volume with 1 U of NdeI and 1 U of BamHI. The digested insert DNA are purified using the UltraClean PCR Clean-up Kit (Mo Bio Laboratories). Purified digested insert DNA are quantified using the NanoDrop ND1000 spectrophotemeter and the DNA concentration adjusted to 10 μg/ml by dilution with TE buffer. The purified restricted insert DNA are used immediately for vector ligation.

**Example 12- Ligation of the gene inserts into the pET-19b vector**

Ligation reactions are all performed in a total volume of 20 μl. Twenty five ng of linearised pTrcHis is incubated with an equi-molar amount of restricted insert at 16˚C for 16 h in 30 mM Tris-HCl (pH 7.8), 10 mM MgCl2, 10 mM DTT and 1 mM ATP containing 1 U of T4 DNA ligase (Fermentas). An identical ligation reaction containing no insert DNA is also included as a vector rc-eircularisation negative control.

**Example 13 -Transformation of pET-19b ligations into E. coli JM109 cells**

Competent E. coli JM109 cells are thawed from -80˚C storage on ice and then 50 μl of the cells are transferred into ice-cold 1.5 ml microfuge tubes containing 5 μl of the overnight ligation reactions. The tubes are mixed by gently tapping the bottom of each tube on the bench and left on ice for 30 min. The cells are then heat-shocked by placing the tubes into a 42˚C waterbath for 45 s before returning the tube to ice for 2 min. The transformed cells are recovered in 1 ml LB broth for l h at 37˚C with gentle mixing. The recovered cells are harvested at 2,500 x g for 5 min and the cells resuspended in 50 μl of fresh LB broth. The entire 50 μl of resuspended cells are spread evenly onto a LB agar plate containing 100 mg/l ampicillin using a sterile glass rod. Plates are incubated at 37˚C for 16 h.

**Example 14- Detection of recombinant pET-19b constructs in E. coli by PCR**

Twelve single transformant colonies for each construct are streaked onto fresh LB agar plates containing 100 mg/l ampicillin and incubated at 37˚ C for 16 h. A single colony from each transformation event is resuspended in 50 μl of TE buffer and boiled for 1 min. Two μl of boiled cells are used as template for PCR. The amplification mixture consists of 1x PCR buffer (containing 1.5 mM of MgCl2), 1 U of Taq DNA polymerase, 0.2 mM of each dNTP, 0.5 μM of the pET-19b-F primer (5'-GGAATTGTGAGCGGATAAC-3') and 0.5 μM of the pET-19b-R primer (5'-GCAAAAAACCCCTCAAGAC-3'). Cycling conditions involve an initial template denaturation step of 5 min at 94˚C, followed by 30 cycles of denaturation at 94˚C for 30 s, annealing at 60˚C for 15 s, and a primer extension at 72˚C for 1 min. The PCR products are subjected to electrophoresis in 1% (w/v) agarose gels in 1x TAE buffer, stained with a 1 μg/ml ethidium bromide solution and viewed over UV light.

**Example 15- Purification of recombinant pET-19b plasmids**

An E. coli J1 09 clone harbouring the successfully ligated pET-19b plasmids are inoculated into 5 ml of LB broth supplemented with 100 mg/l ampicillin and the broth culture is incubated at 37˚C for 12 h with shaking. The entire overnight culture is centrifuged at 5,000 x g for 10 min and the plasmid contained in the cells extracted using the QIAprsp Spin Miniprep Kit (Qiagen) according to the manufacturer's instructions. The purified recombinant pET-19b plasmid is stored at -20˚C.

**Example 16- Transformation of recombinant pET-19b plasmids into E. coli BL21 (DE3) cells**

Competent E. coli BL21 (DE3) cells are thawed from -80˚C storage on ice and then 20 μl of the cells are transferred into ice-cold 1.5 ml microfuge tubes containing I μl of the recombinant purified pET-19b plasmid. The tubes are mixed by gently tapping the bottom of each tube on the bench and left on ice for 30 min. The cells are then heat-shocked by placing the tubes into a 42˚C waterbath for 45 s before returning the tube to ice for 2 min. The transformed cells are recovered in 1 ml LB broth for 1 h at 37˚C with gentle mixing. Fifty μl of the recovered cells are spread evenly onto a LB agar plate containing 100 mg/l ampicillin using a sterile glass rod. Plates are incubated at 37˚C for 16 h.

**Example 17 -Expression of recombinant His-tagged proteins**

Four isolated colonies of recombinant pET-19b plasmid in E. coli BL21 (DE3) is inoculated into 3 ml LB broth in a 10 ml centrifuge tube containing 100 mg/l ampicillin and 1 mM IPTG and incubated at 37˚C for 16 h with shaking. The cells are harvested by centrifugation at 5,000 x g for 10 min at 4˚C. The supernatant is discarded and each pellet is resuspended with 300 μl of 1 x SDS-PAGE loading buffer (250 mM Tris-HCl pH 6.0, 8% w/v SDS, 200 mM DTT, 40% v/v glycerol and 0.04 % w/v bromophenol blue). After boiling the tube for 5 min, the cellular debris is pelleted by centrifugation at 10,000 x g for 10 min at 4˚C. The supernatant is transferred to a new tube and stored at -20˚C until analysis.

**Example 18 -Sodium dodecyl sulfate polyacrylamide gel electrophoresis (SDS-PAGE)**

SDS-PAGE analysis of protein involved electrophoretic separation using a discontinuous Tris-glycine buffer system. Ten μl of the prepared cell lysate is loaded into the wells of a polyacrylamide gel. The gel is comprised of a stacking gel (125 mM Tris-HCl ph 6.8,4% w/v acylamide, 0.15% w/v bis-acrylamide and 0.1% w/v SDS) and a separating gel (375 mM Tris-HCl ph 8.8, 12% w/v acylamide, 0.31% w/v bis-acrylamide and 0.1% w/v SDS). These gels are polymerised by the addition of 0.1% (v/v) TEMED and 0.05% (w/v) freshly prepared ammonium sulphate solution and cast into the mini-Protean dual slab cell (Bio-Rad). Samples are run at 150 V at room temperature (RT) until the bromophenol blue dye-front reached the bottom of the gel. Pre-stained molecular weight standards are electrophoresed in parallel with the samples in order to allow molecular weight estimations. After electrophoresis, the gel is immediately stained using Coomassie Brilliant Blue G250 or subjected to electro-transfer onto nitrocellulose membrane for Western blotting.

**Example 19 -Western Blot Analysis**

Electrophoretic transfer of separated proteins from the SDS-PAGE gel to nitrocellulose membrane is performed using the Towbin transfer buffer system. After electrophoresis, the gel is equilibrated in transfer buffer (25 mM Tris, 192 mM glycine, 20% v/v methanol, pH 8.3) for 15 min. The proteins in the gel are transferred to nitrocellulose membrane using the mini-Protean transblot apparatus (Bio-Rad). After assembly of the gel holder according to the manufacturer's instructions, electrophoretic transfer is performed at 30 V overnight at 4˚C, The freshly transferred nitrocellulose membrane containing the separated proteins is blocked with 10 ml of tris-buffered saline (TBS; 20 mM Tris-HCl, 500 mM NaCl, pH 7.5) containing 5% (w/v) skim milk powder for 1 h at RT. The membrane is washed with TBS containing 0.1% (v/v) Tween 20 (TBST) and then incubated with 10 ml mouse anti-his antibody (diluted 5,000-fold with TBST) for 1 h at RT. After washing three times for 5 min with TBST, the membrane is incubated with 10 mL goat anti-mouse IgG (whole molecule)-AP diluted 5,000-fold in TBST for 1 h at RT. The membrane is developed using the Alkaline Phosphatase Substrate Kit (Bio-Rad). The development reaction is stopped by washing the membrane with distilled water. The membrane is then dried and scanned for presentation.

**Example 20 -Expression and purification of recombinant His-tagged proteins**

A single colony of the recombinant pET-19b plasmid in E. coli BL21 (DE3) is inoculated into 20 ml LB broth in a 50 ml centrifuge tube containing 100 mg/l ampicillin and incubated at 37C for 16 h with shaking. A 21 conical flask containing 500 ml of LB broth supplemented with 100 mg/l ampicillin is inoculated with 10 ml of the overnight culture and incubated at 37˚C until the optical density of the cells at 600 nm is 0.5 (approximately 3-4 h). The culture is then induced by adding IPTG to a final concentration of 1 mM and the cells returned to 37˚C with shaking. After 6 h of induction, the culture is transferred to two 250 ml centrifuge bottles and the bottles are centrifuged at 5,000 x g for 10 min at 25˚C. The supernatant is discarded and each pellet is resuspended with 4 ml of NI-NTA native lysis buffer (50 mM NaH2PO4, 300 mM NaCl, 10 mM imidazole, pH 8.0). The resuspended cells are pooled and stored at -20˚C overnight.

The cell suspension is removed from -20˚C storage and thawed on ice. Five μl of DNAse I (10 mg/ml) is added to the thawed lysate and incubated on ice for l h. The lysate is centrifuged at 20,000 x g for 15 min at 4˚C. The supernatant is transferred to a 10 ml column containing a 1 ml bed volume of Ni-NTA agarose resin (Qiagen). The recombinant his-tagged protein is allowed to bind to the resin for 1 h at 4˚C with end-over-end mixing. The resin is then ished with 30 ml of Ni-NTA native ish buffer (50 mM NaH2PO4, 300 mM NaCl, 20 mM imidazole, pH 8.0) before elution with 9 ml of Ni-NTA native elution buffer (50 mM NaH2PO4, 300 mM NaCl, 250 mM imidazole, pH 8.0). Three 3 ml fractions of the eluate are collected and stored at 4˚C. Thirty μl of each eluate is treated with 10 μl of 4 x sample treatment buffer and boiled for 5 min. The samples are subjected to SDS-PAGE and stained with Coomassie Brilliant Blue G250. The stained gel is equilibrated in distilled water for 1 h and dried between two sheets of cellulose overnight at RT.

**Example 21- Dialysis and lyophilisation of the purified recombinant His-tagged protein**

The eluted proteins are pooled and transferred into a hydrated dialysis tube with a molecular weight cut-off (MWCO) of 3,500 Da. A 200 μl aliquot of the pooled eluate is taken and quantified using a commercial Protein Assay (Bio-Rad). The proteins are dialysed against 2 l of distilled water at 4˚C with stirring. The dialysis buffer is changed 8 times at 12-hourly intervals. The dialysed proteins are transferred from the dialysis tube into a 50 ml centrifuge tubes (40 ml maximum volume) and the tubes are placed at -80˚C overnight. Tubes are placed into a freeze-drier and lyophilised to dryness. The lyophilised proteins are then re-hydrated with PBS to a calculated concentration of 2 mg/ml and stored at -20˚C.

**Example 22- Serology using purified recombinant protein**

Ten μg of purified recombinant protein is diluted in 10 ml of carbonate buffer and 100 μl is added to each well of a 96-well microtitre plate. The protein is allowed to coat overnight at 4˚C. The plate is blocked with 150 μl of PBS-BSA (1% w/v) in each well for 1 hour at room temperature (RT) with mixing and then washed three times with 150 μl of PBST (0.05% v/v). Pig sera are diluted 1:100 in 100 μl of PBST-BSA (0.1% w/v) and incubated at RT for 2 hours with mixing. Plates are washed before adding 100 μl of goat anti-swine IgG (whole molecule)-HRP diluted 1:5,000 in PBST. After incubating for 1 hr at RT, the plates are washed and 100 μl of TMB substrate added. Colour development is allowed to occur for 10 minutes at RT before being stopped with the addition of 50 μl of 1 M sulphuric acid. The optical density of each well is read at 450 nm.

**Example 23- Construction of the recombinant pET-19b constructs**

Cloning of the various inserts into the pET-19b expression vector produced recombinant constructs of various sizes. Nucleotide sequencing of the pET-19b constructs verified that the expression cassette is in the correct frame for all the constructs. The predicted translation of the pET-19b expression cassette indicated that all the recombinant his-tagged proteins and the deduced amino acid sequence of the native spirochaete proteins were identical.

**Example 24- Expression and purification of recombinant proteins**

Expression of the selected recombinant E. coli clones is performed in medium-scale to generate sufficient recombinant protein for vaccination of mice. All genes cloned produce recombinant proteins possessing the hexa-histidine fusion with an apparent molecular weight similar to the predicted molecular weight of the native protein as shown in Table 3. All recombinant proteins were highly reactive in western blotting using the anti-his antibody. Purification of the his-tagged recombinant proteins is by affinity chromatography under denaturing conditions. SDS-PAGE and Coomassie Blue staining of all recombinant proteins show purification of the proteins.

**Example 25- Vaccination of mice and pigs using the purified recombinant his-tagged proteins**

Five mice are immunised with the purified recombinant his-tagged protein. The recombinant protein is emulsified with an equal volume of Freunds' Incomplete adjuvant and injected intramuscularly into the quadracep of five mice (Balb/cJ: 5 weeks old males). All mice receive 50 μg of protein in a total volume of 100 μl. Three weeks after the first vaccination, all mice receive a second intramuscular vaccination identical to the first vaccination. All mice are killed two weeks after the second vaccination. Sera are obtained from the heart at post-mortem and tested in ELISA analysis for antibodies against cellular extracts of the parent spirochaete and demonstrate that the recombinant protein is immunogenic in a mouse model.

**Example 26 -Vaccination of pigs using the purified recombinant his-tagged proteins**

Four purified recombinant his-tagged proteins (0.5 mg of each) are pooled in a total volume of 1 ml. Four vaccines are made:

(A) Hemolysin recombinant consisting of: NAV-H32, NAV-H33, NAV-H43 and NAV-H68;
(B) Toxin recombinant consisting of: NAV-H40, NAV-H66, NAV-H72 and NAV-H177;
(C) Lipase/Peptidase recombinant consisting of NAV-H46, NAV-H184, NAV-H186 and NAV-H191;
(D) Protease recombinant consisting of: NAV-H179, NAV-H185, NAV-H188 and NAV-H197. Four groups of ten sero-negative pigs (5 weeks old) are injected intramuscularly with 2 mg of vaccine consisting of 1 ml of the pooled antigen (1mg total protein) emulsified with 1 ml of a water-in-oil adjuvant. The pigs are vaccinated twice intramuscularly into the back of the neck at 5 weeks of age and at 8 weeks of age. A fifth group of ten sero-negative pigs is used as negative controls and are left unvaccinated. All pigs are challenged with 100 ml of an active B. hyodysenteriae culture (~109 cells/ml) at ten weeks of age, and the pigs are observed for clinical signs of swine dysentery during the experiment (up to six weeks post-challenge) and at post-mortem examination. Twice weekly, all pigs are rectally swabbed for selective bacteriological culture.

**Table 1: Function of *B. hyodysenteriae* genes based on similarity with the amino acid sequence of similar bacterial proteins obtained from the SWISS-PROT database**

| Gene | Function | Identity (amino acids) | Similarity (amino acids) |
|---|---|---|---|
| NAV-H32 | **Hemolysin**<br>hemolytic protein (HlpA) of *Nostoc* sp.<br>Nucleotide seq. SEQ ID No.25<br>Amino acid seq. SEQ ID No.26 | 49/137 (35%) | 77/137 (56%) |
| NAV-H33 | **Hemolysin**<br>hemolytic protein of *Prevotella intermedia*<br>Nucleotide seq. SEQ ID No.27<br>Amino acid seq. SEQ ID No.28 | 64/117 (54%) | 83/117 (70%) |
| NAV-H40 | **Toxin**<br>lytic murein transglycosylase (contains LysM/invasin domains)<br>Nucleotide seq. SEQ ID No.19<br>Amino acid seq. SEQ ID No.20 | 120/449 (26%) | 185/449 (41%) |
| NAV-H43 | **Hemolysin**<br>hemolysins and related proteins of *Anabaena variabilis*<br>Nucleotide seq. SEQ ID No.29<br>Amino acid seq. SEQ ID No.30 | 150/425 (35%) | 242/425 (56%) |

(continued)

| Gene | Function | Identity (amino acids) | Similarity (amino acids) |
|---|---|---|---|
| NAV-H46 | **Lipase**<br>Lysophospholipase<br>Nucleotide seq. SEQ ID No.15<br>Amino acid seq. SEQ ID No.16 | 116/299 (38%) | 188/299 (62%) |
| NAV-H66 | **Toxin**<br>toxin (YoeB) of *Escherichia coli*<br>Nucleotide seq. SEQ ID No.21<br>Amino acid seq. SEQ ID No.22 | 42/85 (58%) | 60/85 (76%) |
| NAV-H68 | **Hemolysin**<br>hemolysin-related protein of *Dehalococcoides* sp.<br>Nucleotide seq. SEQ ID No.31<br>Amino acid seq. SEQ ID No.32 | 121/415 (29%) | 204/415 (49%) |
| NAV-H72 | **Toxin**<br>lytic murein transglycosylate (possibly outer membrane-bound)<br>Nucleotide seq. SEQ ID No.23<br>Amino acid seq. SEQ ID No.24 | 51/141 (36%) | 6/141 (53%) |
| NAV-H177 | **Toxin**<br>toxin (YoeB) of *Actinobacillus pleuropneumoniae*<br>Nucleotide seq. SEQ ID No.17<br>Amino acid seq. SEQ ID No.18 | 44/86 (51%) | 64/86 (74%) |
| NAV- H179 | **Protease** | 227/388 (58%) | 291/388 (75%) |
| | ATP-dependent ClpX protease<br>Nucleotide seq. SEQ ID No.1<br>Amino acid seq. SEQ ID No.2 | | |
| NAV- H184 | **Peptidase**<br>Peptidase<br>Nucleotide seq. SEQ ID No.9<br>Amino acid seq. SEQ ID No.10 | 148/445 (33%) | 241/445 (54%) |

(continued)

| Gene | Function | Identity (amino acids) | Similarity (amino acids) |
|---|---|---|---|
| NAV- H185 | **Protease**<br>membrane associated zinc metalloprotease<br>Nucleotide seq. SEQ ID No.3<br>Amino acid seq. SEQ ID No.4 | 199/455 (43%) | 289/455 (63%) |
| NAV- H186 | **Peptidase**<br>Zn-dependent peptidase<br>Nucleotide seq. SEQ ID No.11<br>Amino acid seq. SEQ ID No.12 | 132/406 (32%) | 234/406 (57%) |
| NAV- H188 | **Protease**<br>adenylate cyclase<br>Nucleotide seq. SEQ ID No.5<br>Amino acid seq. SEQ ID No.6 | 147/509 (28%) | 269/509 (52%) |
| NAV- H191 | **Lipase**<br>Lipase<br>Nucleotide seq. SEQ ID No.13<br>Amino acid seq. SEQ ID No.14 | 70/223 (31%) | 117/223 (52%) |
| NAV- H197 | **Protease**<br>trypsin-like serine protease<br>Nucleotide seq. SEQ ID No.7<br>Amino acid seq. SEQ ID No.8 | 184/445 (41%) | 267/445 (60%) |

**Table 2: Oligonucleotide primers used in the PCR distribution analysis of the *B. hyodysenteriae* vaccine candidate genes**

| Gene | Primer name | Primer Sequence (5'-3') | Seq ID No. |
|---|---|---|---|
| NAV-H32 | H32-F4 | CATATTTCTGGTGATTCTC | SEQ ID No.33 |
| | H32-R564 | TTTTTTGATAAATAAGTTTTTTATTTG | SEQ ID No.34 |
| NAV-H33 | H33-F4 | TTTAATACTCCTATATTATTAATTATTT | SEQ ID No.35 |
| | H33-R396 | AAGGAGAATCACCAGAAA | SEQ ID No.36 |
| NAV-H40 | H40-F16 | AAATATGCTTCCATTATAGG | SEQ ID No.37 |
| | H40-R1815 | ACTTTTAGGAAGAAGTTTAAC | SEQ ID No.38 |
| NAV-H43 | H43-F4 | GATATAATTATAATAATAGTGTTAATAC | SEQ ID No.39 |
| | H43-R1296 | TTCAGTGTCTGAATCATTC | SEQ ID No.40 |
| NAV-H46 | H46-F327 | TATGATGGGGCATAGTATGG | SEQ ID No.41 |

(continued)

| Gene | Primer name | Primer Sequence (5'-3') | Seq ID No. |
|---|---|---|---|
| | H46-R769 | TATCTCCTACAGGGTCTTTCG | SEQ ID No.42 |
| NAV-H66 | H66-F17 | ATGATAAAGCTTGGGAAGATTATC | SEQ ID No.43 |
| | H66-R255 | ATAATGTGTTTTACATCCAAC | SEQ ID No.44 |
| NAV-H68 | H68-F53 | TTATGCTCTCTGCTTTATTTTCTGG | SEQ ID No.45 |
| | H68-R596 | TCTTTTATTATTCCTTCTTTATGCC | SEQ ID No.46 |
| NAV-H72 | H72-F128 | CTATGGCAGAAAACAAAGTC | SEQ ID No.47 |
| | H72-R614 | GCAGATAAAGATAAATCAAGTC | SEQ ID No.48 |
| NAV-H177 | H177-F20 | AAAGAGCTTGGCAG | SEQ ID No.49 |
| | H177-R258 | CTTATCATAATGGTATTTACAAGATG | SEQ ID No.50 |
| NAV-H179 | H179-F420 | TGCTATTGCTGATGCTACTAC | SEQ ID No.51 |
| | H179-R705 | ATTATCTTGATGAGGATGCTTTC | SEQ ID No.52 |
| NAV- | H184-F314 | TAGATAAAGATGATGATGAAAAAAG | SEQ ID No.53 |
| H184 | | | |
| | H184-R762 | AAATAAACCTAAATAAGCACC | SEQ ID No.54 |
| NAV-H185 | H185-F294 | TGGGTTTAGAATAGGTGCAGG | SEQ ID No.55 |
| | H185-R796 | ATTTTAATGTTTGTCTTTTTGC | SEQ ID No.56 |
| NAV-H186 | H186-F44 | TAGAAAAGATGCCTATGCTG | SEQ ID No.57 |
| | H186-R718 | TTACAAGAGAAAAATAAACCTG | SEQ ID No.58 |
| NAV-H188 | H188-F293 | TGGGGAATCATACAAATAGAAG | SEQ ID No.59 |
| | H188-R565 | GGCTCATCAAAGGAAGAACC | SEQ ID No.60 |
| NAV-H191 | H191-F8 | ATAATAAAAAAGAAGAAAAAGAG | SEQ ID No.61 |
| | H191-R403 | GTCTGTATCCTAATAAAATAGTATG | SEQ ID No.62 |
| NAV-H197 | H197-F39 | TCTTGGGCTTACATTTACTTTG | SEQ ID No.63 |
| | H197-R354 | TGATTTCTGACTTCTTTTTTG | SEQ ID No.64 |

**Table 3: Oligonucleotide primers used in the cloning of the *B. hyodysenteriae* genes into the pET-19b vector. The apparent molecular weight is determined from SDS-PAGE**

| Gene | Primer name | Primer Sequence (5'-3') | Predicted MW of native protein (Da) | Apparent MW of recombinant protein (kDa) |
|---|---|---|---|---|
| NAV-H32 | H32-F-Nde1 | TATGCATATGTCTGGTGATTCTCCTTTAG (SEQ ID No.65) | 25,326 | 20.5 |
| | H32-R-BamH1 | TGCGGATCCTTTTTTGATAAATAAG (SEQ ID No.66) | | |
| NAV-H33 | H33-F-Nde1 | GGAAGATAACATATGTTTAATACTC (SEQ ID No.67) | 18,465 | 13.9 |
| | H33-R-BamH1 | TCCTAGGATCCTATCTAAAGGAGAATCACC AG | | |

(continued)

| Gene | Primer name | Primer Sequence (5'-3') | Predicted MW of native protein (Da) | Apparent MW of recombinant protein (kDa) |
|---|---|---|---|---|
| | | (SEQ ID No.68) | | |
| NAV-H40 | H40-F-Nde1 | AAATATGCATATGTTATAGGATGCACTTTT GCCTC (SEQ ID No.69) | 69,479 | 61.4 |
| | H40-R-BamH1 | CTTTTAGGATCCAGTTTAACAGGATCTATTG ATC (SEQ ID No.70) | | |
| NAV-H43 | H43-F-Nde1 | AATCCATATGGATATAATTATAATAATAGT GTTAATAC (SEQ ID No.71) | 51,176 | 48.4 |
| | H43-R-BamH1 | ATCTGGATCCTATTCAGTGTCTGAATCATTC TTTTCATCAG (SEQ ID No.72) | | |
| NAV-H46 | H46-F-Nde1 | CTTCATATGGAAATGCTTAATAGAGTTTTA ATATCAG (SEQ ID No.73) | 38,153 | 32.2 |
| | H46-R-BamH1 | AATAGGATCCTATATGAGCATTAAGCCAAG CAATTATC (SEQ ID No.74) | | |
| NAV-H66 | H66-F-Nde1 | ATTTCATATGATAAAGCTTGGGAAGATTAT C (SEQ ID No.75) | 12,678 | 10.9 |
| | H66-R-BamH1 | AATTGGATCCATAATGTGTTTTACATCCAA C (SEQ ID No.76) | | |
| NAV-H68 | H68-F-Nde1 | ATTACATATGGAAATACTACTAGTTTATTTA TTTG (SEQ ID No.77) | 55,123 | 50.4 |
| | H68-R-BamH1 | CATTGGATCCTGAATCAGATGTCTCTTGTAT AT (SEQ ID No. 78) | | |

(continued)

| Gene | Primer name | Primer Sequence (5'-3') | Predicted MW of native protein (Da) | Apparent MW of recombinant protein (kDa) |
|---|---|---|---|---|
| NAV-H72 | H72-F-Nde1 | GATACATATGATACAAAGAATACATGCCAG AATAG (SEQ ID No.79) | 28,201 | 24.6 |
| | H72-R-BamH1 | ATGTGGATCCCATAGTTTTGAGTTTCCTC (SEQ ID No.80) | | |
| NAV-H177 | H177-F-Nde1 | GTTTCATATGAAAGAGCTTGGCAG (SEQ ID No. 81) | 12,997 | 10.8 |
| | H177-R-BamH1 | ATATGGATCCCTTATCATAATGGTATTTACA AGATG (SEQ ID No.82) | | |
| NAV-H179 | H179-F-Nde1 | TTAACATATGGTTCTCTTTGCGGAAGAG (SEQ ID No.83) | 48,109 | 54.6 |
| | H179-R-BamH1 | TTAGGATCCCATCAATACCTAATTTTTCATT AAC (SEQ ID No.84) | | |
| NAV-H184 | H184-F-Nde1 | TTAACATATGTCAGAGAATAGTTTTAAAAA TAGAATAAAAG (SEQ ID No.85) | 53,101 | 57.2 |
| | H184-R-BamH1 | TTAAGGATCCGAAGCAAATGAAAGTTCTTT AATC (SEQ ID No.86) | | |
| NAV-H185 | H185-F-Nde1 | TTTTCATATGGCACAAACTAATGAATCAGC TATATT (SEQ ID No.87) | 58,175 | 67.8 |
| | H185-R-BamH1 | TTAAGGATCCGTTAAGCAGCACCAGAGAAT TATAATC (SEQ ID No.88) | | |
| NAV-H186 | H186-F-Nde1 | CTTACATATGGGAACAAGAGTTGTTTTAGA AAAGATG (SEQ ID No.89) | 49.786 | 56.1 |
| | H186-R-BamH1 | ACTGGGATCCGACTTGCTTATATCCTTAGTA CCG (SEQ ID No. 90) | | |

(continued)

| Gene | Primer name | Primer Sequence (5'-3') | Predicted MW of native protein (Da) | Apparent MW of recombinant protein (kDa) |
|---|---|---|---|---|
| NAV-H188 | H188-F-Nde1 | TAATCATATGATGTACAGTCAGGTTATGAT AG (SEQ ID No.91) | 64,413 | 68.8 |
| | H188-R- | ACTGGGATCCTGTACCGACATTCTCTTCAC | | |
| | BamH1 | (SEQ ID No.92) | | |
| NAV-H191 | H191-F-Nde1 | AAAGCATATGGAAGAAAAAAAATCCAAATT GGAAAG (SEQ ID No.93) | 36,953 | 37.8 |
| | H191-R-BamH1 | ATACGGATCCAATATATGTTCACTGTCCAT AGCCTC (SEQ ID No.94) | | |
| NAV-H197 | H197-F-Nde1 | CTTTCATATGCTTGGGCTTACATTTACTTTG GTTGG (SEQ ID No.95) | 56,670 | 66.6 |
| | H197-R-BamH1 | ATAAGGATCCCATAAGTCATTCTTGCTCGTT TTATAG (SEQ ID No.96) | | |

**Table 4: Gene distribution of the *B. hyodysenteriae* vaccine candidates analysed by PCR using a panel of 23 different strains**

| Gene | Distribution (%) |
|---|---|
| NAV-H32 | 91 |
| NAV-H33 | 91 |
| NAV-H40 | 100 |
| NAV-H43 | 91 |
| NAV-H46 | 100 |
| NAV-H66 | 100 |
| NAV-H68 | 91 |
| NAV-H72 | 87 |
| NAV-H177 | 91 |
| NAV-H179 | 100 |
| NAV-H184 | 100 |
| NAV-H185 | 100 |
| NAV-H186 | 100 |

(continued)

| Gene | Distribution (%) |
|---|---|
| NAV-H188 | 100 |
| NAV-H191 | 100 |
| NAV-H197 | 100 |

**Table 5: Vaccine combinations**

| **Group: Proteases** | | |
|---|---|---|
| **Gene** | **Polynucleotide sequence SEQ ID No.** | **Polypeptide sequence SEQ ID No.** |
| NAV-H197 | SEQ ID No.1 | SEQ ID No.2 |
| NAV-H188 | SEQ ID No.3 | SEQ ID No.4 |
| NAV-H185 | SEQ ID No.5 | SEQ ID No.6 |
| NAV-H179 | SEQ ID No.7 | SEQ ID No.8 |
| **Group: Lipases/Peptidases** | | |
| **Gene** | **Polynucleotide sequence SEQ ID No.** | **Polypeptide sequence SEQ ID No.** |
| NAV-H191 | SEQ ID No.9 | SEQ ID No.10 |
| NAV-H186 | SEQ ID No.11 | SEQ ID No.12 |
| NAV-H184 | SEQ ID No.13 | SEQ ID No.14 |
| NAV-H46 | SEQ ID No.15 | SEQ ID No.16 |
| **Group: Toxins** | | |
| **Gene** | **Polynucleotide sequence SEQ ID No.** | **Polypeptide sequence SEQ ID No.** |
| NAV-H177 | SEQ ID No.17 | SEQ ID No.18 |
| NAV-H72 | SEQ ID No.19 | SEQ ID No.20 |
| NAV-H66 | SEQ ID No.21 | SEQ ID No.22 |
| NAV-H40 | SEQ ID No.23 | SEQ ID No.24 |
| **Group: Hemolysins** | | |
| **Gene** | **Polynucleotide sequence SEQ ID No.** | **Polypeptide sequence SEQ ID No.** |
| NAV-H68 | SEQ ID No.25 | SEQ ID No.26 |
| NAV-H43 | SEQ ID No.27 | SEQ ID No.28 |
| NAV-H33 | SEQ ID No.29 | SEQ ID No.30 |
| NAV-H32 | SEQ ID No.31 | SEQ ID No.32 |

## Claims

1. A polynucleotide comprising the sequence selected from the group consisting of SEQ ID NO: 1, 3, 5, 7, 9, 11, 13, 15, 17, 19, 21, 23, 25, 27, 29, and 31.

2. A plasmid comprising the polynucleotide of Claim 1.

3. The plasmid of Claim 2 wherein said plasmid is an expression vector.

4. A cell containing the plasmid of Claim 2.

5. A cell containing the plasmid of Claim 3.

6. An immunogenic composition comprising the plasmid of Claim 3.

7. A vaccine composition for the treatment or prevention of swine dysentery associated with *Brachyspira hyodysenteriae* comprising the expression vector of Claim 3.

8. A vaccine composition for the treatment or prevention of swine dysentery associated with *Brachyspira hyodysenteriae* comprising the cell of Claim 4.

9. A vaccine composition comprising at least two polynucleotides encoding protease molecules selected from the group consisting of SEQ ID No.1, 3, 5 and 7 for the treatment or prevention of swine dysentery associated with *Brachyspira hyodysenteriae.*

10. A vaccine composition comprising at least three polynucleotides encoding protease molecules selected from the group consisting of SEQ ID No.1, 3, 5 and 7 for the treatment or prevention of swine dysentery associated with *Brackyspira hyodysenteriae.*

11. A vaccine composition consisting of polynucleotides encoding protease molecules selected from the group consisting of: SEQ ID No.1, 3, 5 and 7 for the treatment or prevention of swine dysentery associated with *Brachyspira hyodysenteriae.*

12. A vaccine composition comprising at least two polynucleotides encoding lipase and/or peptidase molecules selected from the group consisting of SEQ ID No.9, 11, 13 and 15 for the treatment or prevention of swine dysentery associated with *Brachyspira hyodysenteriae.*

13. A vaccine composition comprising at least three polynucleotides encoding lipase and/or peptidase molecules selected from the group consisting of SEQ ID No.9, 11, 13 and 15 for the treatment or prevention of swine dysentery associated with *Brachyspira hyadysenteriae.*

14. A vaccine composition consisting of polynucleotides encoding lipase and/or peptidase molecules selected from the group consisting of: SEQ ID No.9, 11, 13 and 15 for the treatment or prevention of swine dysentery associated with *Brachyspira hyodysenteriae.*

15. A vaccine composition comprising at least two polynucleotides encoding toxin molecules selected from the group consisting of SEQ ID No.17, 19, 21 and 23 for the treatment or prevention of swine dysentery associated with *Brachyspira hyodysenteriae*.

**16.** A vaccine composition comprising at least three polynucleotides encoding toxin molecules selected from the group consisting of SEQ ID No.17, 19, 21 and 23 for the treatment or prevention of swine dysentery associated with *Brachyspira hyodysenteriae.*

**17.** A vaccine composition consisting of polynucleotides encoding toxin molecules selected from the group consisting of: SEQ ID No.17, 19, 21 and 23 for the treatment or prevention of swine dysentery associated with *Brachyspira hyodysenteriae.*

**18.** A vaccine composition comprising at least two polynucleotides encoding hemolysin molecules selected from the group consisting of SEQ ID No.25, 27, 29 and 31 for the treatment or prevention of swine dysentery associated with *Brachyspira hyodysenteriae.*

**19.** A vaccine composition comprising at least three polynucleotides encoding hemolysin molecules selected from the group consisting of SEQ ID No.25, 27, 29 and 31 for the treatment or prevention of swine dysentery associated with *Brachyspira hyodysenteriae.*

**20.** A vaccine composition consisting of polynucleotides encoding hemolysin molecules selected from the group consisting of: SEQ ID No.25, 27, 29 and 31 for the treatment or prevention of swine dysentery associated with *Brachyspira hyodysenteriae.*

**21.** A vaccine composition comprising:

(i) at least one polynucleotide encoding a protease molecule selected from the group of: SEQ ID No.1, 3, 5 and 7
(ii) at least one polynucleotide encoding a lipase and/or peptidase molecule selected from the group of: SEQ ID No. 9, 11, 13 and 15
(iii) at least one polynucleotide encoding a toxin molecule selected from the group of: SEQ ID No. 17, 19, 21 and 23
(iv) at least one polynucleotide encoding a hemolysin molecule selected from the group of: SEQ ID No. 25, 27, 29 and 31 for the treatment or prevention of swine dysentery associated with *Brachyspira hyodysenteriae.*

**22.** A DNA molecule comprising a sequence that is at least 70% homologous to a polynucleotide of Claim 1.

**23.** A plasmid comprising the polynucleotide of Claim 22.

**24.** A DNA molecule of Claim 22 wherein said DNA molecule is at least 80% homologous to a polynucleotide of Claim 1.

**25.** A plasmid comprising the polynucleotide of Claim 24.

**26.** A DNA molecule of Claim 22 wherein said DNA molecule is at least 90% homologous to a polynucleotide of Claim 1.

**27.** A plasmid comprising the polynucleotide of Claim 26.

**28.** A polypeptide comprising the sequence selected from the group consisting of SEQ ID Nos: 2, 4, 6, 8, 10, 12, 14, 16, 18, 20, 22, 24, 26, 28, 30 and 32.

**29.** A polynucleotide comprising a sequence which encodes a polypeptide of Claim 28.

**30.** A plasmid comprising a polynucleotide of Claim 29.

**31.** A plasmid of Claim 30 wherein said plasmid is an expression vector.

**32.** A cell containing the plasmid of Claim 30.

**33.** An immunogenic composition comprising the cell of Claim 32.

**34.** A protein comprising a sequence that is at least 70% homologous to the polypeptide of Claim 28.

**35.** The protein of Claim 34 wherein said protein is at least 80% homologous to the polypeptide of Claim 28.

**36.** The protein of Claim 35 wherein said protein is at least 90% homologous to the polypeptide of Claim 28.

**37.** An immunogenic composition comprising the protein of Claim 36.

**38.** An immunogenic composition comprising the polypeptide of Claim 28.

**39.** A vaccine composition for the treatment or prevention of swine dysentery associated with *Brachyspira hyodysenteriae* comprising a polypeptide of Claim 28.

**40.** A vaccine composition comprising at least two protease polypeptides selected from the group consisting of SEQ ID No.2, 4, 6 and 8 for the treatment or prevention of swine dysentery associated with *Brachyspira hyodysenteriae.*

**41.** A vaccine composition comprising at least three protease polypeptides selected from the group consisting of SEQ ID No.2, 4, 6 and 8 for the treatment or prevention of swine dysentery associated with *Brachyspira hyodysenteriae.*

**42.** A vaccine composition consisting of protease polypeptides SEQ ID No.2, 4, 6 and 8 for the treatment or prevention of swine dysentery associated with *Brachyspira hyodysenteriae.*

**43.** A vaccine composition comprising at least two lipase and/or peptidase polypeptides selected from the group consisting of SEQ ID No.10, 12, 14 and 16 for the treatment or prevention of swine dysentery associated with *Brachyspira hyodysenteriae.*

**44.** A vaccine composition comprising at least three lipase and/or peptidase polypeptides selected from the group consisting of SEQ ID No.10, 12, 14 and 16 for the treatment or prevention of swine dysentery associated with *Brachyspira hyodysenteriae.*

**45.** A vaccine composition consisting of lipase and/or peptidase polypeptides from the group consisting of: SEQ ID No. 10, 12, 14 and 16 for the treatment or prevention of swine dysentery associated with *Brachyspira hyodysenteriae.*

**46.** A vaccine composition comprising at least two toxin polypeptides selected from the group consisting of SEQ ID No. 18, 20, 22 and 24 for the treatment or prevention of swine dysentery associated with *Brachyspira hyodysenteriae.*

**47.** A vaccine composition comprising at least three toxin polypeptides selected from the group consisting of SEQ ID No.18, 20, 22 and 24 for the treatment or prevention of swine dysentery associated with *Brachyspira hyodysenteriae*.

**48.** A vaccine composition consisting of toxin polypeptides selected from the group consisting of: SEQ ID No.18, 20, 22 and 24 for the treatment or prevention of swine dysentery associated with *Brachyspira hyodysenteriae.*

**49.** A vaccine composition comprising at least two hemolysin polypeptides selected from the group consisting of SEQ ID No.26, 28, 30 and and 32 for the treatment or prevention of swine dysentery associated with *Brachyspira hyodysenteriae.*

**50.** A vaccine composition comprising at least three hemolysin polypeptides selected from the group consisting of SEQ ID No.26, 28, 30 and 32 for the treatment or prevention of swine dysentery associated with *Brachyspira hyodysenteriae.*

**51.** A vaccine composition consisting of hemolysin polypeptides SEQ ID No.26, 28, 30 and 32 for the treatment or prevention of swine dysentery associated with *Brachyspira hyodysenteriae.*

**52.** A vaccine composition comprising:

(i) at least one protease polypeptide selected from the group of: SEQ ID No.2, 4, 6, or 8
(ii) at least one lipase and/or peptidase polypeptide selected from the group of: SEQ ID No. 10, 12, 14, or 16
(iii) at least one toxin selected from the group of: SEQ ID No. 18, 20, 22 or 24
(iv) at least one hemolysin selected from the group of: SEQ ID No. 26, 28, 30 or 32 for the treatment or prevention of swine dysentery associated with *Brachyspira hyodysenteriae.*

**53.** A monoclonal antibody that specifically binds to a polypeptide of Claim 28.

**54.** A kit for the diagnosis of presence of swine dysentery associated with *Brachyspira hyodysenteriae* in an animal, said kit comprising a monoclonal antibody of Claim 53.

**55.** A kit for the diagnosis of presence of swine dysentery associated with *Brachyspira hyodysenteriae* in an animal, said kit comprising the polypeptides of Claim 28.

**56.** A kit for the diagnosis of presence of swine dysentery associated with *Brachyspira hyodysenteriae* in an animal, said kit comprising the polynucleotides of Claim 1.

**57.** A method of generating an immune response to swine dysentery associated with *Brachyspira hyodysenteriae* in an animal comprising administering to said animal the immunogenic composition of Claim 37 or Claim 38.

**58.** A method of generating an immune response to swine dysentery associated with *Brachyspira hyodysenteriae* in an animal comprising administering to said animal the immunogenic composition of Claim 6 or Claim 33.

**59.** A method of treating or preventing a disease caused by swine dysentery associated with *Brachyspira hyodysenteriae* in an animal in need of said treatment comprising administering to said animal a therapeutically effective amount of the vaccine composition of according to any one of Claims 7 - 21 or Claims 39-52.

European Patent Office

# PARTIAL EUROPEAN SEARCH REPORT

which under Rule 63 of the European Patent Convention shall be considered, for the purposes of subsequent proceedings, as the European search report

Application Number
EP 08 15 2093

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2007/107323 A (NOVARTIS AG [CH]; UNIV MURDOCH [AU]; HAMPSON DAVID JOHN [AU]; BELLGARD) 27 September 2007 (2007-09-27)<br>* abstract *<br>----- | 1-11, 21-42, 52-59 | INV.<br>C07K14/20 |
| A | DATABASE UniProt [Online]<br>29 May 2007 (2007-05-29), "ATP-dependent Clp protease ATP-binding subunit clpX."<br>XP002476722<br>retrieved from EBI accession no. UNIPROT:A4XHW1<br>Database accession no. A4XHW1<br>* the whole document *<br>----- | 1-11, 21-42, 52-59 | |
| A | DATABASE UniProt [Online]<br>20 March 2007 (2007-03-20), "ATP-dependent Clp protease ATP-binding subunit clpX."<br>XP002476723<br>retrieved from EBI accession no. UNIPROT:A3DJ11<br>Database accession no. A3DJ11<br>* the whole document *<br>-----<br>-/-- | 1-11, 21-42, 52-59 | |

TECHNICAL FIELDS SEARCHED (IPC)

C07K

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

3

| Place of search | Date of completion of the search | Examiner |
|---|---|---|
| Munich | 22 July 2008 | Griesinger, Irina |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04E07)

European Patent Office

# PARTIAL EUROPEAN SEARCH REPORT

Application Number
EP 08 15 2093

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MOVAHEDI ABDOLREZA ET AL: "Distribution of the clpX gene in Brachyspira species and reactivity of recombinant Brachyspira pilosicoli ClpX with sera from mice and humans" JOURNAL OF MEDICAL MICROBIOLOGY, vol. 56, no. 7, July 2007 (2007-07), pages 930-936, XP002476721 ISSN: 0022-2615 * abstract * | 1-11, 21-42, 52-59 | |
| X | WO 2008/017636 A (NOVARTIS AG [CH]; UNIV MURDOCH [AU]; BELLGARD MATTHEW [AU]; HAMPSON DA) 14 February 2008 (2008-02-14) * sequences 29,30,51-54,61,62 * | 1-8, 18-39, 49-59 | TECHNICAL FIELDS SEARCHED (IPC) |
| A | US 5 176 910 A (MCCAMAN MICHAEL [US] ET AL) 5 January 1993 (1993-01-05) | 1-8, 18-39, 49-59 | |

3

EPO FORM 1503 03.82 (P04C10)

European Patent Office

INCOMPLETE SEARCH
SHEET C

Application Number
EP 08 15 2093

Although claims 57-59 are directed to a method of treatment of the human/animal body (Article 53(c) EPC), the search has been carried out and based on the alleged effects of the compound/composition.

-----

European Patent Office

Application Number

EP 08 15 2093

## CLAIMS INCURRING FEES

The present European patent application comprised at the time of filing claims for which payment was due.

- [ ] Only part of the claims have been paid within the prescribed time limit. The present European search report has been drawn up for those claims for which no payment was due and for those claims for which claims fees have been paid, namely claim(s):

- [ ] No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

- [ ] All further search fees have been paid within the fixed time limit. The present European search report has been drawn up for all claims.

- [ ] As all searchable claims could be searched without effort justifying an additional fee, the Search Division did not invite payment of any additional fee.

- [X] Only part of the further search fees have been paid within the fixed time limit. The present European search report has been drawn up for those parts of the European patent application which relate to the inventions in respect of which search fees have been paid, namely claims:

  1-11,18-42,49-59

- [ ] None of the further search fees have been paid within the fixed time limit. The present European search report has been drawn up for those parts of the European patent application which relate to the invention first mentioned in the claims, namely claims:

- [ ] The present supplementary European search report has been drawn up for those parts of the European patent application which relate to the invention first mentioned in the claims (Rule 164 (1) EPC).

European Patent Office

**LACK OF UNITY OF INVENTION SHEET B**

Application Number
EP 08 15 2093

The Search Division considers that the present European patent application does not comply with the requirements of unity of invention and relates to several inventions or groups of inventions, namely:

Invention 1: claims 1-11,21-42,52-59 (all partially)

Nucleic acids, plasmids, cells, immunogenic composition and vaccines comprising the nucleic acids and up to 70% identical sequences thereof, when relating to SEQ ID NO: 1. Proteins, immunogenic compositions and vaccines comprising the proteins and up to 70% homologous thereof as well as antibodies thereto, when relating to SEQ ID NO: 2. Furthermore, the corresponding medical uses, diagnostic methods, vaccines and kits.

---

Inventions 2-4: claims 1-11, 21-42, 52-59 (all partially)

Nucleic acids, plasmids, cells, immunogenic composition and vaccines comprising the nucleic acids and up to 70% identical sequences thereof, when relating to SEQ ID NO: 3 or 5 or 7 respectively. Proteins, immunogenic compositions and vaccines comprising the proteins and up to 70% homologous thereof as well as antibodies thereto, when relating to SEQ ID NO: 4 or 6 or 8 respectively. Furthermore, the corresponding medical uses, diagnostic methods, vaccines and kits.

---

Inventions 5-8: claims 1-8, 12-14,21-39,43-45,52-59 (all partially)

Nucleic acids, plasmids, cells, immunogenic composition and vaccines comprising the nucleic acids and up to 70% identical sequences thereof, when relating to SEQ ID NO: 9 or 11 or 13 or 15 respectively. Proteins, immunogenic compositions and vaccines comprising the proteins and up to 70% homologous thereof as well as antibodies thereto, when relating to SEQ ID NO: 10 or 12 or 14 or 16 respectively. Furthermore, the corresponding medical uses, diagnostic methods, vaccines and kits.

---

Inventions 9-12: claims 1-8,15-17,21-39,46-48,52-59 (all partially)

Nucleic acids, plasmids, cells, immunogenic composition and vaccines comprising the nucleic acids and up to 70% identical sequences thereof, when relating to SEQ ID NO: 17 or 19 or 21 or 23 respectively. Proteins, immunogenic compositions and vaccines comprising the proteins and up to 70% homologous thereof as well as antibodies thereto, when relating to SEQ ID NO: 18 or 20 or 22 or 24 respectively. Furthermore, the corresponding medical uses, diagnostic methods, vaccines and kits.

---

European Patent Office

**LACK OF UNITY OF INVENTION SHEET B**

Application Number
EP 08 15 2093

The Search Division considers that the present European patent application does not comply with the requirements of unity of invention and relates to several inventions or groups of inventions, namely:

Inventions 13-16: claims 1-8, 18-39,49-59 (all partially)

Nucleic acids, plasmids, cells, immunogenic composition and vaccines comprising the nucleic acids and up to 70% identical sequences thereof, when relating to SEQ ID NO: 25 or 27 or 29 or 31 respectively. Proteins, immunogenic compositions and vaccines comprising the proteins and up to 70% homologous thereof as well as antibodies thereto, when relating to SEQ ID NO: 26 or 28 or 30 or 32 respectively. Furthermore, the corresponding medical uses, diagnostic methods, vaccines and kits.

---

## ANNEX TO THE EUROPEAN SEARCH REPORT ON EUROPEAN PATENT APPLICATION NO. EP 08 15 2093

This annex lists the patent family members relating to the patent documents cited in the above-mentioned European search report.
The members are as contained in the European Patent Office EDP file on
The European Patent Office is in no way liable for these particulars which are merely given for the purpose of information.

22-07-2008

| Patent document cited in search report | | Publication date | Patent family member(s) | Publication date |
|---|---|---|---|---|
| WO 2007107323 | A | 27-09-2007 | NONE | |
| WO 2008017636 | A | 14-02-2008 | NONE | |
| US 5176910 | A | 05-01-1993 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6562376 B, Hooper
- US 5589466 A, Felgner
- US 6673776 B, Felgner
- US 6710035 B, Feigner
- WO 0123537 A, King
- WO 0168889 A, Malone
- US 5703055 A, Felgner
- US 5824538 A, Branstrom
- US 5877159 A, Powell
- US 6150170 A, Powell
- US 6500419 B, Hone
- US 6682729 B, Powell
- US 5283191 A, Morgan
- US 5554525 A, Sondermeijer
- US 5712118 A, Murphy
- US 4816397 A
- US 4816567 A
- US 4342566 A, Theofilopolous
- US 4341761 A
- US 4399121 A
- US 4427783 A
- US 4444887 A
- US 4451570 A
- US 4466917 A
- US 4472500 A
- US 4491632 A
- US 4493890 A
- US 4946778 A
- US 3654090 A
- US 3850752 A
- US RE31006 E
- US 4016043 E
- US 4016043 A
- US 5837832 A
- WO 9849557 A
- WO 9749989 A
- US 5914123 A, Arntzen
- US 6034298 A, Lam
- US 6136320 A, Arntzen


### Non-patent literature cited in the description


- **Gabe, JD ; Chang, RJ ; Slomiany, R ; Andrews, WH ; McCaman, MT.** Isolation of extracytoplasmic proteins from Serpulina hyodysenteriae B204 and molecular cloning of the flaB1 gene encoding a 38-kilodalton flagellar protein. *Infection and Immunity,* 1995, vol. 63, 142-148
- **La, T ; Phillips, ND ; Reichel, MP ; Hampson, DJ.** Protection of pigs from swine dysentery by vaccination with recombinant BmpB, a 29.7 kDa outer-membrane lipoprotein of Brachyspira hyodysenteriae. *Veterinary Microbiology,* 2004, vol. 102, 97-109
- **Davis, A.J. ; Smith, S.C. ; Moore, R.J.** The Brachyspira hyodysenteriae ftnA gene: DNA vaccination and real-time PCR quantification of bacteria in a mouse model of disease. *Current Microbiology,* 2005, vol. 50, 285-291
- **Goeddel.** Gene Expression Technology: Methods in Enzymology. Academic Press, 1990
- Current Protocols in Molecular Biology. John Wiley & Sons, 1989, 6.3.1-12.3.6
- **Sambrook et al.** Molecular Cloning, A Laboratory Manual. Cold Spring Harbor Press
- Methods in Molecular Biology. vol. 20
- Overview of principles of hybridization and the strategy of nucleic acid probe assays. **Tijssen.** Laboratory Techniques in Biochemistry and Molecular Biology - Hybridization With Nucleic Acid Probes. Elsevier, 1993
- **Tibanyenda, N. et al.** *Eur. J. Biochem.,* 1984, vol. 139, 19
- **Ebel, S. et al.** *Biochem,* 1992, vol. 31, 12083
- Molecular Cloning, A Laboratory Manual. Cold Spring Harbor Laboratory Press, 1989
- **Evans et al.** *Nature,* 1989, vol. 339, 385
- **Huang et al.** *J. Virol.,* 1988, vol. 62, 3855
- **Schlienger et al.** *J. Virol.,* 1992, vol. 66, 2
- Current Protocols in Molecular Biology. John Wiley & Sons, 1991
- **Hochuli et al.** *J. Chromatography,* 1987, vol. 411, 177
- **Janknecht et al.** *PNAS USA,* vol. 88, 8972
- Current Protocols in Molecular Biology. John Wiley & Sons, 1992
- **Hood et al.** Immunology. Benjamin/Cummings, Menlo Park, 1984, 384
- **Kohler et al.** *Nature,* 1975, vol. 256, 495-497
- **Kozbor et al.** *Immunology Today,* 1983, vol. 4, 72

- **Cole et al.** Monoclonal Antibodies and Cancer Therapy. Alan R. Liss, Inc, 1985, 77-96
- **Morrison et al.** *J. Bacteriol.,* 1984, 159-870
- **Neuberger et al.** *Nature,* 1984, vol. 312, 604-608
- **Takeda et al.** *Nature,* 1985, vol. 314, 452-454
- **Huse et al.** *Science,* 1989, vol. 246, 1275-128
- **Martin.** Remington's Pharmaceutical Sciences. Mack Publishing Co, 1990
- **Martin.** Remington's Pharmaceutical Sciences. Mack Publishing Co, 1990, 1435-1712

SEQUENCE LISTING

<110> Murdoch University

<120> Novel Sequences of Brachyspira, Immunogenic Compositions, Methods for Preparation and Use Thereof

<130> 52578

<160> 96

<170> PatentIn version 3.3

<210> 1
<211> 1230
<212> DNA
<213> Brachyspira hyodysenteriae

<400> 1
atgtctaaaa agaataatga taagaaagaa gtttgttctc tttgcggaag agaattaaaa 60

gaattaagcc gatatattga aggcaatgaa ggatatttgt gttcggattg ttcttccata 120

gcaaatgact attttaatat gaatatgcag aagaaaaaat ctaaagaaaa agattatgat 180

ataaagatat tgcctccaaa acaaataaaa tctttacttg atgaatatgt tgtaggtcag 240

gattatgcca aaaaggtatt atctgtatct gtttataatc attataaaag aatcactcag 300

aatgctgaag ataaaaatga tgttgaaata gagaagtcta atgtactttt aataggacct 360

acaggaagcg gtaaaacatt attggcaaga actttggcta aggctttaaa tgtacctttt 420

gctattgctg atgctactac tgtaacagag gctggttatg taggcgatga tgttgagaat 480

atacttttaa gacttataca gaatgctgat ggagatataa aattagctga aaaaggcata 540

atatatattg atgagataga taaaatttca cgtaaaagcg aatctcgttc tataacaaga 600

gatgtatcag gtgaaggagt tcagcaggct ttattaaaaa ttgttgaagg tactgttgct 660

actgttcctc ctcatggcgg aagaaagcat cctcatcaag ataatcttca tatagacact 720

tctaatatac tttttatatg cggaggtgct ttcattgata tagaaaaatc tattgctgaa 780

agaacttcta agaaaggttt aggatttgct gctgaagtta attctatgga taatctaaat
840

tatgatgaga taatgaaaaa tattgctact gaggatttag ttaaatacgg gcttatacct
900

gaacttatag gaaggcttcc tgttatagct actttagaag agcttaaaga agaagagctt
960

ttgaaaatat taaaagagcc taagaatgct ttgactaagc agtatcagaa attattctct
1020

tatgataata tagaattaaa aatagatgat gatgcattaa aatcaatagt taaaaagact
1080

atggaagagc atactggagc tagaggactt agagctttat ttgagagagt tatgcttgat
1140

gctatgtatg atatgcctaa tgaaagtaaa gaaaaacaag tgtttgagct tgataaaaaa
1200

tatgttaatg aaaaattagg tattgatgat
1230

<210> 2
<211> 410
<212> PRT
<213> Brachyspira hyodysenteriae

<400> 2

Met Ser Lys Lys Asn Asn Asp Lys Lys Glu Val Cys Ser Leu Cys Gly
1 5 10 15

Arg Glu Leu Lys Glu Leu Ser Arg Tyr Ile Glu Gly Asn Glu Gly Tyr
20 25 30

Leu Cys Ser Asp Cys Ser Ser Ile Ala Asn Asp Tyr Phe Asn Met Asn
35 40 45

Met Gln Lys Lys Lys Ser Lys Glu Lys Asp Tyr Asp Ile Lys Ile Leu
50 55 60

Pro Pro Lys Gln Ile Lys Ser Leu Leu Asp Glu Tyr Val Val Gly Gln
65 70 75 80

Asp Tyr Ala Lys Lys Val Leu Ser Val Ser Val Tyr Asn His Tyr Lys
85 90 95

Arg Ile Thr Gln Asn Ala Glu Asp Lys Asn Asp Val Glu Ile Glu Lys
100 105 110

Ser Asn Val Leu Leu Ile Gly Pro Thr Gly Ser Gly Lys Thr Leu Leu
115 120 125

Ala Arg Thr Leu Ala Lys Ala Leu Asn Val Pro Phe Ala Ile Ala Asp
130 135 140

Ala Thr Thr Val Thr Glu Ala Gly Tyr Val Gly Asp Asp Val Glu Asn
145 150 155 160

Ile Leu Leu Arg Leu Ile Gln Asn Ala Asp Gly Asp Ile Lys Leu Ala
165 170 175

Glu Lys Gly Ile Ile Tyr Ile Asp Glu Ile Asp Lys Ile Ser Arg Lys
180 185 190

Ser Glu Ser Arg Ser Ile Thr Arg Asp Val Ser Gly Glu Gly Val Gln
195 200 205

Gln Ala Leu Leu Lys Ile Val Glu Gly Thr Val Ala Thr Val Pro Pro
210 215 220

His Gly Gly Arg Lys His Pro His Gln Asp Asn Leu His Ile Asp Thr
225 230 235 240

Ser Asn Ile Leu Phe Ile Cys Gly Gly Ala Phe Ile Asp Ile Glu Lys
245 250 255

Ser Ile Ala Glu Arg Thr Ser Lys Lys Gly Leu Gly Phe Ala Ala Glu
260 265 270

Val Asn Ser Met Asp Asn Leu Asn Tyr Asp Glu Ile Met Lys Asn Ile
275 280 285

Ala Thr Glu Asp Leu Val Lys Tyr Gly Leu Ile Pro Glu Leu Ile Gly
290 295 300

Arg Leu Pro Val Ile Ala Thr Leu Glu Glu Leu Lys Glu Glu Glu Leu
305 310 315 320

Leu Lys Ile Leu Lys Glu Pro Lys Asn Ala Leu Thr Lys Gln Tyr Gln
325 330 335

Lys Leu Phe Ser Tyr Asp Asn Ile Glu Leu Lys Ile Asp Asp Asp Ala
340 345 350

Leu Lys Ser Ile Val Lys Lys Thr Met Glu Glu His Thr Gly Ala Arg
355 360 365

Gly Leu Arg Ala Leu Phe Glu Arg Val Met Leu Asp Ala Met Tyr Asp
370 375 380

Met Pro Asn Glu Ser Lys Glu Lys Gln Val Phe Glu Leu Asp Lys Lys
385 390 395 400

Tyr Val Asn Glu Lys Leu Gly Ile Asp Asp
405 410

<210> 3
<211> 1503
<212> DNA
<213> Brachyspira hyodysenteriae

<400> 3
gtgttaagga aattgatata tattattttt ttgcattcaa ttctttttaa tttttttatt
60

tttgcacaaa ctaatgaatc agctatatta aattacactc aatatatgga aagaataaaa
120

tccataatac cagaaatgaa attaactgca tctcaagaaa gcaatgccta taataattta
180

acaaaagcaa aaagctccgg agacgttaaa tttgatttgc aggctggtgc tataggaatg
240

caaagccatt ttgatgaata caattttttta gcaacatcag attttaatta taatgggttt
300

agaataggtg caggattcag cggacttgta ccatactctg gaactagatg gtctgtagaa
360

attaaacatg acagtttttt cggcgacttt aatacaggag atatttcatt accggttgat
420

acgcctctag gtagaataaa tggaaaactt ccaaatttaa gtactaatga ttttaaatac
480

tattatccaa gcataaaaat tcaaatagct cagcctattt taagggattt ttttggtaaa
540

ttggatagat accctataaa agatgcagaa tatcagctta ccatagcaaa attaaaaaga
600

ataatagacg acaacagcgt attaacatct tatcagaaaa tttattatca atggataatg
660

gcaagaaaat taatagattt atatgatgat atgataagag aagcaagaag ttttgaaaat
720

caagtataca gaagatatac aagcggagtt atagataatg actcatatca gaatgcaaaa
780

agacaaacat taaaatatat agaagcaaga gataaatctg aattaatgct taaaaaaata
840

atgagaaata ttcaattctt tatacctgaa gaaaatatac agccaaatga agatgattgg
900

aatcagacat tagaaacctc tataaatgct aaaatagata tagtaccatt tttagaaagt
960

gctcagggac aaatggctta tcaattaaaa ttaagaagcg aatatgctat ttcagtaatg
1020

aaaaataatg ctctgcctga tttatctata gtaggaagcg tatcattatc aagtttagat
1080

gacagcggat attttaaatc tttttctacc atgactaatg ttgattattt tgtagggctt
1140

atgttttcct accccatagg cggacgtgat gctaaagcta aaatggaaga tgcttatgct
1200

gctttgaatg ctgttacggc tgattttgat agggtgaaca gagattttga cgttcagata
1260

ggtacttatt atgatgagtt tgaggcatac aaaaaaatgc tagaaaataa aaaattggaa
1320

gttaatgcta tagtatcaag aataaatacg caaaatgcta aattcagaca aggaagactc
1380

cctatagatg aaataataaa tgcaaggctt gatttagcac aggcaagagc agaacttctt
1440

aatttgcagt atttaataat aagcactgtt atggattata attctctggt gctgcttaac
1500

aat
1503

<210> 4
<211> 501
<212> PRT
<213> Brachyspira hyodysenteriae

<400> 4

Val Leu Arg Lys Leu Ile Tyr Ile Ile Phe Leu His Ser Ile Leu Phe
1 5 10 15

Asn Phe Phe Ile Phe Ala Gln Thr Asn Glu Ser Ala Ile Leu Asn Tyr
20 25 30

```
Thr Gln Tyr Met Glu Arg Ile Lys Ser Ile Ile Pro Glu Met Lys Leu
        35                  40                  45

Thr Ala Ser Gln Glu Ser Asn Ala Tyr Asn Asn Leu Thr Lys Ala Lys
    50                  55                  60

Ser Ser Gly Asp Val Lys Phe Asp Leu Gln Ala Gly Ala Ile Gly Met
65                  70                  75                  80

Gln Ser His Phe Asp Glu Tyr Asn Phe Leu Ala Thr Ser Asp Phe Asn
                85                  90                  95

Tyr Asn Gly Phe Arg Ile Gly Ala Gly Phe Ser Gly Leu Val Pro Tyr
            100                 105                 110

Ser Gly Thr Arg Trp Ser Val Glu Ile Lys His Asp Ser Phe Phe Gly
        115                 120                 125

Asp Phe Asn Thr Gly Asp Ile Ser Leu Pro Val Asp Thr Pro Leu Gly
    130                 135                 140

Arg Ile Asn Gly Lys Leu Pro Asn Leu Ser Thr Asn Asp Phe Lys Tyr
145                 150                 155                 160

Tyr Tyr Pro Ser Ile Lys Ile Gln Ile Ala Gln Pro Ile Leu Arg Asp
                165                 170                 175

Phe Phe Gly Lys Leu Asp Arg Tyr Pro Ile Lys Asp Ala Glu Tyr Gln
            180                 185                 190

Leu Thr Ile Ala Lys Leu Lys Arg Ile Ile Asp Asp Asn Ser Val Leu
        195                 200                 205

Thr Ser Tyr Gln Lys Ile Tyr Tyr Gln Trp Ile Met Ala Arg Lys Leu
    210                 215                 220

Ile Asp Leu Tyr Asp Asp Met Ile Arg Glu Ala Arg Ser Phe Glu Asn
225                 230                 235                 240

Gln Val Tyr Arg Arg Tyr Thr Ser Gly Val Ile Asp Asn Asp Ser Tyr
                245                 250                 255

Gln Asn Ala Lys Arg Gln Thr Leu Lys Tyr Ile Glu Ala Arg Asp Lys
            260                 265                 270
```

Ser Glu Leu Met Leu Lys Lys Ile Met Arg Asn Ile Gln Phe Phe Ile
275 280 285

Pro Glu Glu Asn Ile Gln Pro Asn Glu Asp Asp Trp Asn Gln Thr Leu
290 295 300

Glu Thr Ser Ile Asn Ala Lys Ile Asp Ile Val Pro Phe Leu Glu Ser
305 310 315 320

Ala Gln Gly Gln Met Ala Tyr Gln Leu Lys Leu Arg Ser Glu Tyr Ala
325 330 335

Ile Ser Val Met Lys Asn Asn Ala Leu Pro Asp Leu Ser Ile Val Gly
340 345 350

Ser Val Ser Leu Ser Ser Leu Asp Asp Ser Gly Tyr Phe Lys Ser Phe
355 360 365

Ser Thr Met Thr Asn Val Asp Tyr Phe Val Gly Leu Met Phe Ser Tyr
370 375 380

Pro Ile Gly Gly Arg Asp Ala Lys Ala Lys Met Glu Asp Ala Tyr Ala
385 390 395 400

Ala Leu Asn Ala Val Thr Ala Asp Phe Asp Arg Val Asn Arg Asp Phe
405 410 415

Asp Val Gln Ile Gly Thr Tyr Tyr Asp Glu Phe Glu Ala Tyr Lys Lys
420 425 430

Met Leu Glu Asn Lys Lys Leu Glu Val Asn Ala Ile Val Ser Arg Ile
435 440 445

Asn Thr Gln Asn Ala Lys Phe Arg Gln Gly Arg Leu Pro Ile Asp Glu
450 455 460

Ile Ile Asn Ala Arg Leu Asp Leu Ala Gln Ala Arg Ala Glu Leu Leu
465 470 475 480

Asn Leu Gln Tyr Leu Ile Ile Ser Thr Val Met Asp Tyr Asn Ser Leu
485 490 495

Val Leu Leu Asn Asn
500

<210> 5
<211> 1773
<212> DNA
<213> Brachyspira hyodysenteriae

<400> 5
atgattaaga acatatttta taaatcatta ctcattttga tattatcaag tgctttaatg 60

tttataggta tactgtttac tgtgcaatat aataatatta tgtacagtca ggttatgata 120

gtaaatatta tggaaatgct tgaaaaagag attgatttat atgatgtgca aactgaagat 180

gcttttagaa aatttgtact gcagtcagat aaagaagaat taagaatcag cataatatct 240

acaaatgggg ttgtagtagc tgatacgatg acagatacct ctaaaaatcc tatggggaat 300

catacaaata gaagcgatgt tatagaggca ttgcatagta tggaaaataa gccggctttt 360

tctataaata cctctattag tcagaaaact ccttatatgt atgtatccaa aaaaattaaa 420

gctgatgata aaagagatta tatacttaga gtttcaattc ctatagagtc agttaataaa 480

tatttgataa gcttcttatt tacagccgta atggttatag gtatagttat tataattatg 540

gctttggttc ttcctttgat gagccgtaat attatgattc ctttctatat gataaaagaa 600

accctggata atatatataa taataaatct ccgacaccta aaaatttaac gggatacaat 660

gatataaata ctattcttta tgatataaat gaacttgctg ttagtttgaa tgaaaatatc 720

acaggttatc agactgaaag agaaaagctt aattatgtac ttgagaatat agcacagggt 780

attatagctg ttaataaaaa taaggagata ttttttatta atcagtatgc tttagatttg 840

cttgaaattt ctgacagcaa tataagaaaa ctcaatgaaa taataaaaga tgatgatgta 900

atagaaaaaa tagataatgc catagagttc agcagatttt caaaatttga tgttaaagaa 960

catactatgg atatagaaat aagcatcata cctataagaa ataatgaaaa tatatctgca 1020

ttaataaaat ttgaagatgt tacagatata agaaaggttg agatagaaaa gcaggacttc
1080

tttataaatg cttcccatga attaaaaact cctctcactt ctataatagg atattctgaa
1140

cttcttattg ctacaggagg cggaaagaat caggcagatt ttataaaaag aataaataat
1200

gaagctttaa gaatgaaaga tttagttatt gatatgctta cattgagcag aatagaagct
1260

aattggaaag aaactgttga tgaagaaata gacataaaag atattgttct tgatgtattt
1320

gaaagcaata gaataaaatc cgagcagaga aatatagatg tagaattaga tatagaatct
1380

gctgttataa tggctaataa agagaaaatt actgaagtag ttaataattt agttgataat
1440

gctataaaat atactgatga tgacggtaaa gtaaagatat atttaaagaa taattctgat
1500

aaagctatat tttcagtaaa agatacagga tgcggtatcg cacctcagta tttgaatagg
1560

gtttttgaaa gatttttcag agttaaaaat aataaatatt tgaaagttca tggtacaggt
1620

cttggactta caatagtaaa aaatatatgt aattattata atgctgacat tcatattaaa
1680

agtgaagaga atgtcggtac agaaatgact gttgtattta atttgtataa agaagaagaa
1740

tctaaaaata ttgaaaaaat agaaaaaaat aat
1773

<210> 6
<211> 591
<212> PRT
<213> Brachyspira hyodysenteriae

<400> 6

Met Ile Lys Asn Ile Phe Tyr Lys Ser Leu Leu Ile Leu Ile Leu Ser
1 5 10 15

Ser Ala Leu Met Phe Ile Gly Ile Leu Phe Thr Val Gln Tyr Asn Asn
20 25 30

Ile Met Tyr Ser Gln Val Met Ile Val Asn Ile Met Glu Met Leu Glu
35 40 45

```
Lys Glu Ile Asp Leu Tyr Asp Val Gln Thr Glu Asp Ala Phe Arg Lys
    50                  55                  60

Phe Val Leu Gln Ser Asp Lys Glu Glu Leu Arg Ile Ser Ile Ile Ser
65                  70                  75                  80

Thr Asn Gly Val Val Val Ala Asp Thr Met Thr Asp Thr Ser Lys Asn
                85                  90                  95

Pro Met Gly Asn His Thr Asn Arg Ser Asp Val Ile Glu Ala Leu His
            100                 105                 110

Ser Met Glu Asn Lys Pro Ala Phe Ser Ile Asn Thr Ser Ile Ser Gln
        115                 120                 125

Lys Thr Pro Tyr Met Tyr Val Ser Lys Lys Ile Lys Ala Asp Asp Lys
    130                 135                 140

Arg Asp Tyr Ile Leu Arg Val Ser Ile Pro Ile Glu Ser Val Asn Lys
145                 150                 155                 160

Tyr Leu Ile Ser Phe Leu Phe Thr Ala Val Met Val Ile Gly Ile Val
                165                 170                 175

Ile Ile Ile Met Ala Leu Val Leu Pro Leu Met Ser Arg Asn Ile Met
            180                 185                 190

Ile Pro Phe Tyr Met Ile Lys Glu Thr Leu Asp Asn Ile Tyr Asn Asn
        195                 200                 205

Lys Ser Pro Thr Pro Lys Asn Leu Thr Gly Tyr Asn Asp Ile Asn Thr
    210                 215                 220

Ile Leu Tyr Asp Ile Asn Glu Leu Ala Val Ser Leu Asn Glu Asn Ile
225                 230                 235                 240

Thr Gly Tyr Gln Thr Glu Arg Glu Lys Leu Asn Tyr Val Leu Glu Asn
                245                 250                 255

Ile Ala Gln Gly Ile Ile Ala Val Asn Lys Asn Lys Glu Ile Phe Phe
            260                 265                 270

Ile Asn Gln Tyr Ala Leu Asp Leu Leu Glu Ile Ser Asp Ser Asn Ile
        275                 280                 285
```

```
Arg Lys Leu Asn Glu Ile Ile Lys Asp Asp Asp Val Ile Glu Lys Ile
    290                 295                 300

Asp Asn Ala Ile Glu Phe Ser Arg Phe Ser Lys Phe Asp Val Lys Glu
305                 310                 315                 320

His Thr Met Asp Ile Glu Ile Ser Ile Ile Pro Ile Arg Asn Asn Glu
                325                 330                 335

Asn Ile Ser Ala Leu Ile Lys Phe Glu Asp Val Thr Asp Ile Arg Lys
            340                 345                 350

Val Glu Ile Glu Lys Gln Asp Phe Phe Ile Asn Ala Ser His Glu Leu
        355                 360                 365

Lys Thr Pro Leu Thr Ser Ile Ile Gly Tyr Ser Glu Leu Leu Ile Ala
    370                 375                 380

Thr Gly Gly Gly Lys Asn Gln Ala Asp Phe Ile Lys Arg Ile Asn Asn
385                 390                 395                 400

Glu Ala Leu Arg Met Lys Asp Leu Val Ile Asp Met Leu Thr Leu Ser
                405                 410                 415

Arg Ile Glu Ala Asn Trp Lys Glu Thr Val Asp Glu Glu Ile Asp Ile
            420                 425                 430

Lys Asp Ile Val Leu Asp Val Phe Glu Ser Asn Arg Ile Lys Ser Glu
        435                 440                 445

Gln Arg Asn Ile Asp Val Glu Leu Asp Ile Glu Ser Ala Val Ile Met
    450                 455                 460

Ala Asn Lys Glu Lys Ile Thr Glu Val Val Asn Asn Leu Val Asp Asn
465                 470                 475                 480

Ala Ile Lys Tyr Thr Asp Asp Asp Gly Lys Val Lys Ile Tyr Leu Lys
                485                 490                 495

Asn Asn Ser Asp Lys Ala Ile Phe Ser Val Lys Asp Thr Gly Cys Gly
            500                 505                 510

Ile Ala Pro Gln Tyr Leu Asn Arg Val Phe Glu Arg Phe Phe Arg Val
        515                 520                 525
```

Lys Asn Asn Lys Tyr Leu Lys Val His Gly Thr Gly Leu Gly Leu Thr
    530                 535                 540

Ile Val Lys Asn Ile Cys Asn Tyr Tyr Asn Ala Asp Ile His Ile Lys
545                 550                 555                 560

Ser Glu Glu Asn Val Gly Thr Glu Met Thr Val Val Phe Asn Leu Tyr
                565                 570                 575

Lys Glu Glu Glu Ser Lys Asn Ile Glu Lys Ile Glu Lys Asn Asn
            580                 585                 590

<210> 7
<211> 1509
<212> DNA
<213> Brachyspira hyodysenteriae

<400> 7
atggagcaaa aaaaacgttc atttatgttc tttttaaatc ttgggcttac atttactttg
60

gttggtataa ttatttcttt tttcattttt tcatatgaaa aaaattataa aaaagatgag
120

ttcttggttc atgcccaggc agcacctgaa aaaacatctt ttacaggttc attagatggt
180

gccttagctg tagaaaatgc tataagagat gttgtggata aaaatatgcc tgctgtagtt
240

aatatatcta cagaaataga agcaggacaa actgaagaag atagatatgc cgatgaattc
300

tttagatttt tcttcggaga tcaaatgcct agacaaaaaa gaagtcagaa atcattagga
360

agcggtttca taatcaatga agaaggatat gtgctttcta attaccatgt tgtaaaaggc
420

gctactaaaa taatgattac gctttatgga gaagacggag agcttcctgc aaaattaata
480

ggttatgatg aggcttatga cttagcatta ctaaaaatag aggatgaaaa tagaactttc
540

ccttatgttg ctttaggaga cagtgatgct atagagcctg gtgaatttgc tattgctata
600

ggaaaccctt acggacttaa taatacagtt actttcggta ttgtaagtgc taaaggaaga
660

agcgatgttg gtgctaataa atatcaaaga tatatacaaa ctgatgttgc tataaaccct
720

ggtaactcag gaggaccttt attcaatata catggacagg ttataggaat aaacacattg 780

atatattcta cttctggcgg aagcataggt ataggatttg ctactcctat taatcttgct 840

acttcagtaa tgactgattt aaaagaaaat ggaagagtta caagaggata tttaggtata 900

tatttacaag atatcgatgg aaatctttca agaggtttaa atgttaagca aaacagcgga 960

gtatatgtaa gtgaggttgt acctgattca cctgctgcaa aaggcggatt acaggacgga 1020

gatattataa tagagtatga tggtgaaaaa atgactaaat caggtgattt attcaataaa 1080

gtagccacta ctaaagtagg aaaagaagtt actgttaaat atttaagaaa tggaagagaa 1140

agaagcacta aaatcactat agaagctaga gttgaagatg aagaggttgt acctacaaga 1200

caatctcaga ataactcaca aggaagtact cgttcttgga tgggattaga tgtttctaat 1260

atcactcctg aaatatctca aagacttcag ataagaagca atgagcgagg tgttgtagta 1320

cttaatatga ctcaaaactc aaaggcatat caaaatggac ttagaccagg agatgttata 1380

aaagctatta atggtataac aatatcaaat actgatgatt atgataattt tgtaaaatct 1440

tatggtaatg ataagtcctt tacaataact ataaaacgag caagaatgac ttatgttata 1500

attatagaa 1509

<210> 8
<211> 503
<212> PRT
<213> Brachyspira hyodysenteriae

<400> 8

Met Glu Gln Lys Lys Arg Ser Phe Met Phe Phe Leu Asn Leu Gly Leu
1 5 10 15

Thr Phe Thr Leu Val Gly Ile Ile Ile Ser Phe Phe Ile Phe Ser Tyr
20 25 30

```
Glu Lys Asn Tyr Lys Lys Asp Glu Phe Leu Val His Ala Gln Ala Ala
        35                  40                  45

Pro Glu Lys Thr Ser Phe Thr Gly Ser Leu Asp Gly Ala Leu Ala Val
    50                  55                  60

Glu Asn Ala Ile Arg Asp Val Val Asp Lys Asn Met Pro Ala Val Val
65                  70                  75                  80

Asn Ile Ser Thr Glu Ile Glu Ala Gly Gln Thr Glu Glu Asp Arg Tyr
                85                  90                  95

Ala Asp Glu Phe Phe Arg Phe Phe Phe Gly Asp Gln Met Pro Arg Gln
            100                 105                 110

Lys Arg Ser Gln Lys Ser Leu Gly Ser Gly Phe Ile Ile Asn Glu Glu
        115                 120                 125

Gly Tyr Val Leu Ser Asn Tyr His Val Val Lys Gly Ala Thr Lys Ile
    130                 135                 140

Met Ile Thr Leu Tyr Gly Glu Asp Gly Glu Leu Pro Ala Lys Leu Ile
145                 150                 155                 160

Gly Tyr Asp Glu Ala Tyr Asp Leu Ala Leu Leu Lys Ile Glu Asp Glu
                165                 170                 175

Asn Arg Thr Phe Pro Tyr Val Ala Leu Gly Asp Ser Asp Ala Ile Glu
            180                 185                 190

Pro Gly Glu Phe Ala Ile Ala Ile Gly Asn Pro Tyr Gly Leu Asn Asn
        195                 200                 205

Thr Val Thr Phe Gly Ile Val Ser Ala Lys Gly Arg Ser Asp Val Gly
    210                 215                 220

Ala Asn Lys Tyr Gln Arg Tyr Ile Gln Thr Asp Val Ala Ile Asn Pro
225                 230                 235                 240

Gly Asn Ser Gly Gly Pro Leu Phe Asn Ile His Gly Gln Val Ile Gly
                245                 250                 255

Ile Asn Thr Leu Ile Tyr Ser Thr Ser Gly Gly Ser Ile Gly Ile Gly
            260                 265                 270
```

```
Phe Ala Thr Pro Ile Asn Leu Ala Thr Ser Val Met Thr Asp Leu Lys
        275                 280                 285

Glu Asn Gly Arg Val Thr Arg Gly Tyr Leu Gly Ile Tyr Leu Gln Asp
    290                 295                 300

Ile Asp Gly Asn Leu Ser Arg Gly Leu Asn Val Lys Gln Asn Ser Gly
305                 310                 315                 320

Val Tyr Val Ser Glu Val Val Pro Asp Ser Pro Ala Ala Lys Gly Gly
                325                 330                 335

Leu Gln Asp Gly Asp Ile Ile Ile Glu Tyr Asp Gly Glu Lys Met Thr
            340                 345                 350

Lys Ser Gly Asp Leu Phe Asn Lys Val Ala Thr Thr Lys Val Gly Lys
        355                 360                 365

Glu Val Thr Val Lys Tyr Leu Arg Asn Gly Arg Glu Arg Ser Thr Lys
    370                 375                 380

Ile Thr Ile Glu Ala Arg Val Glu Asp Glu Glu Val Val Pro Thr Arg
385                 390                 395                 400

Gln Ser Gln Asn Asn Ser Gln Gly Ser Thr Arg Ser Trp Met Gly Leu
                405                 410                 415

Asp Val Ser Asn Ile Thr Pro Glu Ile Ser Gln Arg Leu Gln Ile Arg
            420                 425                 430

Ser Asn Glu Arg Gly Val Val Val Leu Asn Met Thr Gln Asn Ser Lys
        435                 440                 445

Ala Tyr Gln Asn Gly Leu Arg Pro Gly Asp Val Ile Lys Ala Ile Asn
    450                 455                 460

Gly Ile Thr Ile Ser Asn Thr Asp Asp Tyr Asp Asn Phe Val Lys Ser
465                 470                 475                 480

Tyr Gly Asn Asp Lys Ser Phe Thr Ile Thr Ile Lys Arg Ala Arg Met
                485                 490                 495

Thr Tyr Val Ile Ile Ile Glu
            500
```

<210> 9
<211> 1353
<212> DNA
<213> Brachyspira hyodysenteriae

<400> 9
atgtcagaga atagttttaa aaatagaata aaagaaatat ataatgatat aaaaaataag 60

gcaaaggata ttgatgaaat agaaatttat atgtcagtaa gcggagaaga ggagtttact 120

gtaagagaaa aagatttaga taaatatact tatgctgaat ctggaggaat aggtttaaga 180

ttgattaaag acggtaaagt tggaataagt tttacagaga aaataagctc tgatataaat 240

atagatgatc ttattaataa tgcaaaaact tcattacatt atgctgactc tgaacctgaa 300

tataataaac ttatagataa agatgatgat gaaaaaagtt atgatatgat taataaagat 360

atagttaatc tgtctaatga taaacttaaa gaaatttcat tatcaataga agataaatta 420

tattctcttg ataatagaat tgtaaatgta ccatctgcag gattagcaag atataatttt 480

aataaatgta taataaacag caatggtata tgcaaagaag aaaagaaaaa cagtatagca 540

tattatggtg aagtaatagc aaaagaaaat gatatagtaa aaactttttt tgatgtttat 600

tcttcaacag atgctgtatt tgatgttgct tcttttacaa aaaatattgt tgataatgtt 660

gtcaataaat tatctgcaaa gcctatagaa agcggaaaat ataaaactgt atttacaaat 720

aaagctatga gaataattat aggtgcttat ttaggtttat tttcttcaga agcagttcag 780

aaaaaattat cacttcttca gggaaaattg aatcagaaaa tagcaagcag tatcataaat 840

attaaagata ttcctattta tgataaagga ttagcaaata caaattttga cggagaaggt 900

tctaaaacta aagatttaaa tataataact aacggggttt taaatagcta tctttataat 960

aattatacag caaaaaaaga cggcataaaa acaacttcgc atgcttcaag aggatttaaa 1020

acttcaatag gaatatcttg tcataatttt attttggaga atggaaataa tactcaggaa
1080

gaattgattt cccaaattca aaatggagta ttagtaaatt ctctaaccgg aactcatgca
1140

ggagtaaatg ctataagcgg agatttttct ttacaggcag aaggcataaa aatagaaaat
1200

ggaaaattat catatacagc aaatcctttc attgtttcag gtaatatatt agatctttta
1260

agcaatgtag aaatgcttgc caatgatact gattatcatc attcatctat atatgcacct
1320

agtgctttga ttaaagaact ttcatttgct tca
1353

<210> 10
<211> 451
<212> PRT
<213> Brachyspira hyodysenteriae

<400> 10

Met Ser Glu Asn Ser Phe Lys Asn Arg Ile Lys Glu Ile Tyr Asn Asp
1 5 10 15

Ile Lys Asn Lys Ala Lys Asp Ile Asp Glu Ile Glu Ile Tyr Met Ser
20 25 30

Val Ser Gly Glu Glu Glu Phe Thr Val Arg Glu Lys Asp Leu Asp Lys
35 40 45

Tyr Thr Tyr Ala Glu Ser Gly Gly Ile Gly Leu Arg Leu Ile Lys Asp
50 55 60

Gly Lys Val Gly Ile Ser Phe Thr Glu Lys Ile Ser Ser Asp Ile Asn
65 70 75 80

Ile Asp Asp Leu Ile Asn Asn Ala Lys Thr Ser Leu His Tyr Ala Asp
85 90 95

Ser Glu Pro Glu Tyr Asn Lys Leu Ile Asp Lys Asp Asp Asp Glu Lys
100 105 110

Ser Tyr Asp Met Ile Asn Lys Asp Ile Val Asn Leu Ser Asn Asp Lys
115 120 125

Leu Lys Glu Ile Ser Leu Ser Ile Glu Asp Lys Leu Tyr Ser Leu Asp

130 135 140

Asn Arg Ile Val Asn Val Pro Ser Ala Gly Leu Ala Arg Tyr Asn Phe
145 150 155 160

Asn Lys Cys Ile Ile Asn Ser Asn Gly Ile Cys Lys Glu Glu Lys Lys
165 170 175

Asn Ser Ile Ala Tyr Tyr Gly Glu Val Ile Ala Lys Glu Asn Asp Ile
180 185 190

Val Lys Thr Phe Phe Asp Val Tyr Ser Ser Thr Asp Ala Val Phe Asp
195 200 205

Val Ala Ser Phe Thr Lys Asn Ile Val Asp Asn Val Val Asn Lys Leu
210 215 220

Ser Ala Lys Pro Ile Glu Ser Gly Lys Tyr Lys Thr Val Phe Thr Asn
225 230 235 240

Lys Ala Met Arg Ile Ile Ile Gly Ala Tyr Leu Gly Leu Phe Ser Ser
245 250 255

Glu Ala Val Gln Lys Lys Leu Ser Leu Leu Gln Gly Lys Leu Asn Gln
260 265 270

Lys Ile Ala Ser Ser Ile Ile Asn Ile Lys Asp Ile Pro Ile Tyr Asp
275 280 285

Lys Gly Leu Ala Asn Thr Asn Phe Asp Gly Glu Gly Ser Lys Thr Lys
290 295 300

Asp Leu Asn Ile Ile Thr Asn Gly Val Leu Asn Ser Tyr Leu Tyr Asn
305 310 315 320

Asn Tyr Thr Ala Lys Lys Asp Gly Ile Lys Thr Thr Ser His Ala Ser
325 330 335

Arg Gly Phe Lys Thr Ser Ile Gly Ile Ser Cys His Asn Phe Ile Leu
340 345 350

Glu Asn Gly Asn Asn Thr Gln Glu Glu Leu Ile Ser Gln Ile Gln Asn
355 360 365

Gly Val Leu Val Asn Ser Leu Thr Gly Thr His Ala Gly Val Asn Ala
    370                 375                 380

Ile Ser Gly Asp Phe Ser Leu Gln Ala Glu Gly Ile Lys Ile Glu Asn
385                 390                 395                 400

Gly Lys Leu Ser Tyr Thr Ala Asn Pro Phe Ile Val Ser Gly Asn Ile
                405                 410                 415

Leu Asp Leu Leu Ser Asn Val Glu Met Leu Ala Asn Asp Thr Asp Tyr
            420                 425                 430

His His Ser Ser Ile Tyr Ala Pro Ser Ala Leu Ile Lys Glu Leu Ser
        435                 440                 445

Phe Ala Ser
    450

<210> 11
<211> 1263
<212> DNA
<213> Brachyspira hyodysenteriae

<400> 11
atggtaaaaa gattaacctt aaaaaatgga acaagagttg ttttagaaaa gatgcctatg
60

ctggatactg tatctatagg ttttatattt ttaacaggaa gtgctaatga gaaaaaagaa
120

gaaaatggat atactcattt catagagcat atgcttttta aaggcactga caaaattagt
180

gcaaaagagc ttattagaaa tattgaaggt gtaggcggta tatttaatgc atttacttca
240

agacatttaa cttcttttta tatcaatata atatctaaat attttgacag agctgttaat
300

gctttagaaa atatcatgct tcattctgca ttcagagaag atgatataaa cagagaaaaa
360

aaagttatta tcgaagagct taaaatgtca aatgatactc ctgaagaaat atcagctaat
420

caattttttg ctgctgctta taaaggtact tctatgagct tccctatagg aggaaatatt
480

aataatataa aaaaaataag cagagataaa atttattctt attttaaaga gcattttaat
540

tctaataatt taatcatatc aatagctgga aattttgata ttgattatgc tgttgaaaga
600

ttagaaaaaa taaaattaga aaaaagaaat aaaacagtta atgatgatct tcctttttat
660

tataaaacta taactaaaga aaaacaggaa attaatcagg tttatttttc tcttgtaaca
720

ccttcatata atgcatgcga taataaaaaa agatacgcta tgaatatagt taatgatata
780

ttcggaggaa gttcttattc aagattattt cagtcaataa gagaaaataa aggactttgt
840

tataatatat atagttataa ttctagtttt ataaacggag gtacatttga gatacatggt
900

tctactagtt tggataggta tgcagaaaca atagaaagca tatattgtga aatagaaaaa
960

ttggttaatg aaagaatatc tgaagaagaa attgaagaaa ctaaagagag ttataaaggt
1020

tctatggcat ttagtaaatt cagtgcagaa tttataatga ataaaaatgc aaggcatgaa
1080

ttatatttat ctaaatatgt ttcatttaaa gagctttaca atataataga taaaatagat
1140

ttaaaacttg ttaatgaagt tatagacgaa aaattgatga ataaaaaatt ctttttaaca
1200

tctgtaggag caagcggtac taaggatata agcaagtctt taagcagtaa acttaaattg
1260

aat
1263

<210> 12
<211> 421
<212> PRT
<213> Brachyspira hyodysenteriae

<400> 12

Met Val Lys Arg Leu Thr Leu Lys Asn Gly Thr Arg Val Val Leu Glu
1 5 10 15

Lys Met Pro Met Leu Asp Thr Val Ser Ile Gly Phe Ile Phe Leu Thr
20 25 30

Gly Ser Ala Asn Glu Lys Lys Glu Glu Asn Gly Tyr Thr His Phe Ile
35 40 45

Glu His Met Leu Phe Lys Gly Thr Asp Lys Ile Ser Ala Lys Glu Leu
50 55 60

```
Ile Arg Asn Ile Glu Gly Val Gly Gly Ile Phe Asn Ala Phe Thr Ser
65                  70                  75                  80

Arg His Leu Thr Ser Phe Tyr Ile Asn Ile Ile Ser Lys Tyr Phe Asp
                85                  90                  95

Arg Ala Val Asn Ala Leu Glu Asn Ile Met Leu His Ser Ala Phe Arg
            100                 105                 110

Glu Asp Asp Ile Asn Arg Glu Lys Lys Val Ile Ile Glu Glu Leu Lys
        115                 120                 125

Met Ser Asn Asp Thr Pro Glu Glu Ile Ser Ala Asn Gln Phe Phe Ala
    130                 135                 140

Ala Ala Tyr Lys Gly Thr Ser Met Ser Phe Pro Ile Gly Gly Asn Ile
145                 150                 155                 160

Asn Asn Ile Lys Lys Ile Ser Arg Asp Lys Ile Tyr Ser Tyr Phe Lys
                165                 170                 175

Glu His Phe Asn Ser Asn Asn Leu Ile Ile Ser Ile Ala Gly Asn Phe
            180                 185                 190

Asp Ile Asp Tyr Ala Val Glu Arg Leu Glu Lys Ile Lys Leu Glu Lys
        195                 200                 205

Arg Asn Lys Thr Val Asn Asp Asp Leu Pro Phe Tyr Tyr Lys Thr Ile
    210                 215                 220

Thr Lys Glu Lys Gln Glu Ile Asn Gln Val Tyr Phe Ser Leu Val Thr
225                 230                 235                 240

Pro Ser Tyr Asn Ala Cys Asp Asn Lys Lys Arg Tyr Ala Met Asn Ile
                245                 250                 255

Val Asn Asp Ile Phe Gly Gly Ser Ser Tyr Ser Arg Leu Phe Gln Ser
            260                 265                 270

Ile Arg Glu Asn Lys Gly Leu Cys Tyr Asn Ile Tyr Ser Tyr Asn Ser
        275                 280                 285

Ser Phe Ile Asn Gly Gly Thr Phe Glu Ile His Gly Ser Thr Ser Leu
```

290 295 300

Asp Arg Tyr Ala Glu Thr Ile Glu Ser Ile Tyr Cys Glu Ile Glu Lys
305 310 315 320

Leu Val Asn Glu Arg Ile Ser Glu Glu Glu Ile Glu Glu Thr Lys Glu
325 330 335

Ser Tyr Lys Gly Ser Met Ala Phe Ser Lys Phe Ser Ala Glu Phe Ile
340 345 350

Met Asn Lys Asn Ala Arg His Glu Leu Tyr Leu Ser Lys Tyr Val Ser
355 360 365

Phe Lys Glu Leu Tyr Asn Ile Ile Asp Lys Ile Asp Leu Lys Leu Val
370 375 380

Asn Glu Val Ile Asp Glu Lys Leu Met Asn Lys Lys Phe Phe Leu Thr
385 390 395 400

Ser Val Gly Ala Ser Gly Thr Lys Asp Ile Ser Lys Ser Leu Ser Ser
405 410 415

Lys Leu Lys Leu Asn
420

<210> 13
<211> 972
<212> DNA
<213> Brachyspira hyodysenteriae

<400> 13
atggaagata ataaaaaaga agaaaaagag caaataaaaa aagaaaaaga acaaaagaaa
60

cttgaagaaa aaaaatccaa attggaaaga aaacagaaaa aaatagagga aaagataaga
120

agaaaaaatt tgcttggcga gtatctcact tatccaagta tggctgttaa ttttataaaa
180

gctgataaga aaataaagcc tgaaaagtat atatttgata aaaataaggc ccaatatata
240

ttatattttg cacctcagtt aaaagaaaat gaaagtccaa aaaaacaggt tatagtttat
300

ttatatggcg gaggctggag agagggtaat gctaatttat acagattcgt tggaagaaga
360

tttgcaaaag agaaatttca tactatttta ttaggataca gactcagtaa aaaatataaa 420

taccccgctc aaatagaaga tgtatttgca ggatttaata aggcactaga agttttgaaa 480

aataagaata ttgattattc tgatattata gtaataggtt catcagcagg tgcacatttg 540

ggagctttgc ttgtatacca taaggaaatg cataagaaat acaatataga ttctaatatt 600

tttaaaggat atgtatcgct tggaggacca actgatttaa atgtatgtac taatgatata 660

ataactccta tgcttgatca gctatttgaa gagggatata atagagaaga tgctaatcct 720

tataatcata tagacggaag cgagaaaaca aaggtattat gcgttcattc agaattggat 780

cctatttgtg atgctgataa ttcaattaat ttttctaata aggttaatag ctttcatgac 840

ggcttggctg agtgtattat atttgataat aaaaatattt atcataataa tttagttaat 900

ggtatattct ttgaggctat ggacagtgaa catatattgg ataaagtatt taaatggatt 960

gaaaagttaa at 972

<210> 14
<211> 324
<212> PRT
<213> Brachyspira hyodysenteriae

<400> 14

Met Glu Asp Asn Lys Lys Glu Glu Lys Glu Gln Ile Lys Lys Glu Lys
1 5 10 15

Glu Gln Lys Lys Leu Glu Glu Lys Lys Ser Lys Leu Glu Arg Lys Gln
20 25 30

Lys Lys Ile Glu Glu Lys Ile Arg Arg Lys Asn Leu Leu Gly Glu Tyr
35 40 45

Leu Thr Tyr Pro Ser Met Ala Val Asn Phe Ile Lys Ala Asp Lys Lys
50 55 60

Ile Lys Pro Glu Lys Tyr Ile Phe Asp Lys Asn Lys Ala Gln Tyr Ile
65 70 75 80

```
Leu Tyr Phe Ala Pro Gln Leu Lys Glu Asn Glu Ser Pro Lys Lys Gln
                85                  90                  95

Val Ile Val Tyr Leu Tyr Gly Gly Gly Trp Arg Glu Gly Asn Ala Asn
            100                 105                 110

Leu Tyr Arg Phe Val Gly Arg Arg Phe Ala Lys Glu Lys Phe His Thr
        115                 120                 125

Ile Leu Leu Gly Tyr Arg Leu Ser Lys Lys Tyr Lys Tyr Pro Ala Gln
    130                 135                 140

Ile Glu Asp Val Phe Ala Gly Phe Asn Lys Ala Leu Glu Val Leu Lys
145                 150                 155                 160

Asn Lys Asn Ile Asp Tyr Ser Asp Ile Ile Val Ile Gly Ser Ser Ala
                165                 170                 175

Gly Ala His Leu Gly Ala Leu Leu Val Tyr His Lys Glu Met His Lys
            180                 185                 190

Lys Tyr Asn Ile Asp Ser Asn Ile Phe Lys Gly Tyr Val Ser Leu Gly
        195                 200                 205

Gly Pro Thr Asp Leu Asn Val Cys Thr Asn Asp Ile Ile Thr Pro Met
    210                 215                 220

Leu Asp Gln Leu Phe Glu Glu Gly Tyr Asn Arg Glu Asp Ala Asn Pro
225                 230                 235                 240

Tyr Asn His Ile Asp Gly Ser Glu Lys Thr Lys Val Leu Cys Val His
                245                 250                 255

Ser Glu Leu Asp Pro Ile Cys Asp Ala Asp Asn Ser Ile Asn Phe Ser
            260                 265                 270

Asn Lys Val Asn Ser Phe His Asp Gly Leu Ala Glu Cys Ile Ile Phe
        275                 280                 285

Asp Asn Lys Asn Ile Tyr His Asn Asn Leu Val Asn Gly Ile Phe Phe
    290                 295                 300

Glu Ala Met Asp Ser Glu His Ile Leu Asp Lys Val Phe Lys Trp Ile
```

305 310 315 320

Glu Lys Leu Asn

<210> 15
<211> 930
<212> DNA
<213> Brachyspira hyodysenteriae

<400> 15
atggaaatgc ttaatagagt tttaatatca gatgatggtc atagaatgta tacatatatt
60

tttaagcctg attccaaacc aaaagcaata gttcagatag ttcatggact tggagagcat
120

gccggaagat ataaagagat tgctgaaaaa ctcaataaag aaggattttt agtttgtgcc
180

gatgatcata gaggattcgg aagaagcact gtaagtaaag atcaaatagg gcatatgggc
240

gataaaaacg gacatgaact tattatagaa gatatgaggc atttaatggt aaatactaaa
300

gctgattatc ctaatcttcc ttattttatg atggggcata gtatgggttc ttttcttact
360

agaggttttt taattaaata tcataaagat ttaaatggcg ctataataat gggtactaga
420

ggaaaaccta agggaataga aaatctagga aaaactatag ctaatattca gaagtcatta
480

ttcgggggaa gaaaaagagc ttatttactt gataaattat cagtaggcgg atacggtaaa
540

aaattctttc ctaaagataa ttcagatttg gcatggctta cttcggataa agaagaaatt
600

aaaaaagccc aggaagacga atactttgca aataaacctg caagtataga aacatatata
660

caattatttg aacttattga taaaatttca gataaggata attactcttc tatggataaa
720

aatttcccta tacttttaat atcaggagcg aaagaccctg taggagatat gggaaagggc
780

gttaaatggg ttcatgaaat gtatgaatca ttaggattca aagatgttac tataagtctt
840

tatgaaaatg gaagacatga aatacttaac gatgttcaaa gagatgatgt agctaaagag
900

ataattgctt ggcttaatgc tcatatagca
930

<210> 16
<211> 310
<212> PRT
<213> Brachyspira hyodysenteriae

<400> 16

```
Met Glu Met Leu Asn Arg Val Leu Ile Ser Asp Asp Gly His Arg Met
1               5                   10                  15

Tyr Thr Tyr Ile Phe Lys Pro Asp Ser Lys Pro Lys Ala Ile Val Gln
            20                  25                  30

Ile Val His Gly Leu Gly Glu His Ala Gly Arg Tyr Lys Glu Ile Ala
        35                  40                  45

Glu Lys Leu Asn Lys Glu Gly Phe Leu Val Cys Ala Asp Asp His Arg
    50                  55                  60

Gly Phe Gly Arg Ser Thr Val Ser Lys Asp Gln Ile Gly His Met Gly
65                  70                  75                  80

Asp Lys Asn Gly His Glu Leu Ile Ile Glu Asp Met Arg His Leu Met
                85                  90                  95

Val Asn Thr Lys Ala Asp Tyr Pro Asn Leu Pro Tyr Phe Met Met Gly
            100                 105                 110

His Ser Met Gly Ser Phe Leu Thr Arg Gly Phe Leu Ile Lys Tyr His
        115                 120                 125

Lys Asp Leu Asn Gly Ala Ile Ile Met Gly Thr Arg Gly Lys Pro Lys
    130                 135                 140

Gly Ile Glu Asn Leu Gly Lys Thr Ile Ala Asn Ile Gln Lys Ser Leu
145                 150                 155                 160

Phe Gly Gly Arg Lys Arg Ala Tyr Leu Leu Asp Lys Leu Ser Val Gly
                165                 170                 175

Gly Tyr Gly Lys Lys Phe Phe Pro Lys Asp Asn Ser Asp Leu Ala Trp
            180                 185                 190
```

Leu Thr Ser Asp Lys Glu Glu Ile Lys Lys Ala Gln Glu Asp Glu Tyr
        195                 200                 205

Phe Ala Asn Lys Pro Ala Ser Ile Glu Thr Tyr Ile Gln Leu Phe Glu
    210                 215                 220

Leu Ile Asp Lys Ile Ser Asp Lys Asp Asn Tyr Ser Ser Met Asp Lys
225                 230                 235                 240

Asn Phe Pro Ile Leu Leu Ile Ser Gly Ala Lys Asp Pro Val Gly Asp
                245                 250                 255

Met Gly Lys Gly Val Lys Trp Val His Glu Met Tyr Glu Ser Leu Gly
            260                 265                 270

Phe Lys Asp Val Thr Ile Ser Leu Tyr Glu Asn Gly Arg His Glu Ile
        275                 280                 285

Leu Asn Asp Val Gln Arg Asp Asp Val Ala Lys Glu Ile Ile Ala Trp
    290                 295                 300

Leu Asn Ala His Ile Ala
305                 310

<210> 17
<211> 258
<212> DNA
<213> Brachyspira hyodysenteriae

<400> 17
atgaaaatag tttttaatga aagagcttgg caggaatata tagagtgggt atcagaagat
60

aaaaaaatag taaaaaaaat taacgacttg attaaagata taataagaaa tccttgtgat
120

ggaataggaa aagcagaaaa attaaaatat gataaaaaag atctttactc tagaagaata
180

aataaagaac atagattagt atatcatata gaaaataatc aattaataat aacatcttgt
240

aaataccatt atgataag
258

<210> 18
<211> 86
<212> PRT
<213> Brachyspira hyodysenteriae

<400> 18

Met Lys Ile Val Phe Asn Glu Arg Ala Trp Gln Glu Tyr Ile Glu Trp
1 5 10 15

Val Ser Glu Asp Lys Lys Ile Val Lys Lys Ile Asn Asp Leu Ile Lys
20 25 30

Asp Ile Ile Arg Asn Pro Cys Asp Gly Ile Gly Lys Ala Glu Lys Leu
35 40 45

Lys Tyr Asp Lys Lys Asp Leu Tyr Ser Arg Arg Ile Asn Lys Glu His
50 55 60

Arg Leu Val Tyr His Ile Glu Asn Asn Gln Leu Ile Ile Thr Ser Cys
65 70 75 80

Lys Tyr His Tyr Asp Lys
85

<210> 19
<211> 1815
<212> DNA
<213> Brachyspira hyodysenteriae

<400> 19
atgaaaaata tttttaaata tgcttccatt ataggatgca cttttgcctc gcttaatttt 60

gcggctgatt atatagatta taaagtaaaa aacggcgata ctttatttgg aatagctttc 120

gctcatgata tgagtgcaaa tgaattttta aaagttaata atataaaaga tcctgataaa 180

tataatctta gagtaggaga aactttaaaa gtaaaagata aaggttatac tcttgtatat 240

gactctgata ataaagtttt cggcttgaaa ggagaagaag gaaactcata caaagattat 300

aaagtaaaaa acggggatac tttatttggt atagcatttg cccatggaat gactgctaat 360

gaatttctag ctataaataa tattaaagat gccaataaat ataatcttag agtaggacaa 420

actcttaaag tagcaaataa tcaaaaagaa aataatgctt cttcaaataa tataaataat 480

agtgataata cagaaaatta tgatacttat aaagtgcaaa gcggtgatac tttatacgga 540

atagctttct ctcatggtat gacagcaagc gaatttttaa agattaataa tatagatgat 600

cctgataaat ataaactata tgtaggtaaa actatgtatg ttaaatcatc taaaaaagaa 660

aataatttaa acacaaataa tgaaaaagat acaggaaaag aaatagaata ctatactgta 720

aaaagcggcg ataccttata cggaatagct tttcaaaatg atattagcgt aaatgatttt 780

ctaagaatta acaatataga tgatcccttta aaatacaaat taagaacagg cgaaaaatta 840

aaaatatatg caagagaaaa tgcctcaaat acacaaagca aaactataaa aacatataga 900

gtaaaaaacg gagatactct tggagagata gcattaagaa attctatgtc tttgaaagat 960

cttcttcaat taaataatct aaaaaataat tatgtgctta aagtaggaga tactttaaaa 1020

atatatgata atattaatat aacaagttct tcaacatcaa caacatacag aactttggaa 1080

aattataaag taaaaagcgg tgatacttta agcggaatag ctctagcaag aggaatggat 1140

ctagtagaat tatactccat aaataatata aatgacaaat atattttgaa agttggagat 1200

aatcttaaag tatatgctaa ccctaaaaaa acaactactt tagtaatatc aaattataaa 1260

gttcaaagcg gagatagttt atactcaata gcaaaaaaac ataaaatgga tttaagagat 1320

ttaatgcagc ttaataatat aaaaaatgct aatgaatata aattatatgt cggcgccaat 1380

ctaaaagtaa aaacagcaaa aatggtgcct tattctttta atgatgattc tatattacct 1440

gacagctctt ttatatggcc ttataaagga ataataattt caggatatgg agtagcttct 1500

gataaacttg caaacagagg tgtgaatata ttaggagatg taggagacaa agttgtagct 1560

tctgatgacg gaatcgtaga atatgctgat aatataagag gattcggtac tgttataata 1620

cttaaacata aaaacggata taatacttct tatgctcatc tttctaagat aaatgttaaa 1680

cttggagata tagtaaagaa aggagattat ataggagaca ttggcgatac tggtatgata 1740

gatagaagcg aactatattt taagatttct tatcagggaa gatcaataga tcctgttaaa
1800

cttcttccta aaagt
1815

<210> 20
<211> 605
<212> PRT
<213> Brachyspira hyodysenteriae

<400> 20

Met Lys Asn Ile Phe Lys Tyr Ala Ser Ile Ile Gly Cys Thr Phe Ala
1 5 10 15

Ser Leu Asn Phe Ala Ala Asp Tyr Ile Asp Tyr Lys Val Lys Asn Gly
20 25 30

Asp Thr Leu Phe Gly Ile Ala Phe Ala His Asp Met Ser Ala Asn Glu
35 40 45

Phe Leu Lys Val Asn Asn Ile Lys Asp Pro Asp Lys Tyr Asn Leu Arg
50 55 60

Val Gly Glu Thr Leu Lys Val Lys Asp Lys Gly Tyr Thr Leu Val Tyr
65 70 75 80

Asp Ser Asp Asn Lys Val Phe Gly Leu Lys Gly Glu Glu Gly Asn Ser
85 90 95

Tyr Lys Asp Tyr Lys Val Lys Asn Gly Asp Thr Leu Phe Gly Ile Ala
100 105 110

Phe Ala His Gly Met Thr Ala Asn Glu Phe Leu Ala Ile Asn Asn Ile
115 120 125

Lys Asp Ala Asn Lys Tyr Asn Leu Arg Val Gly Gln Thr Leu Lys Val
130 135 140

Ala Asn Asn Gln Lys Glu Asn Asn Ala Ser Ser Asn Asn Ile Asn Asn
145 150 155 160

Ser Asp Asn Thr Glu Asn Tyr Asp Thr Tyr Lys Val Gln Ser Gly Asp
165 170 175

Thr Leu Tyr Gly Ile Ala Phe Ser His Gly Met Thr Ala Ser Glu Phe
180 185 190

Leu Lys Ile Asn Asn Ile Asp Asp Pro Asp Lys Tyr Lys Leu Tyr Val
195 200 205

Gly Lys Thr Met Tyr Val Lys Ser Ser Lys Lys Glu Asn Asn Leu Asn
210 215 220

Thr Asn Asn Glu Lys Asp Thr Gly Lys Glu Ile Glu Tyr Tyr Thr Val
225 230 235 240

Lys Ser Gly Asp Thr Leu Tyr Gly Ile Ala Phe Gln Asn Asp Ile Ser
245 250 255

Val Asn Asp Phe Leu Arg Ile Asn Asn Ile Asp Asp Pro Leu Lys Tyr
260 265 270

Lys Leu Arg Thr Gly Glu Lys Leu Lys Ile Tyr Ala Arg Glu Asn Ala
275 280 285

Ser Asn Thr Gln Ser Lys Thr Ile Lys Thr Tyr Arg Val Lys Asn Gly
290 295 300

Asp Thr Leu Gly Glu Ile Ala Leu Arg Asn Ser Met Ser Leu Lys Asp
305 310 315 320

Leu Leu Gln Leu Asn Asn Leu Lys Asn Asn Tyr Val Leu Lys Val Gly
325 330 335

Asp Thr Leu Lys Ile Tyr Asp Asn Ile Asn Ile Thr Ser Ser Ser Thr
340 345 350

Ser Thr Thr Tyr Arg Thr Leu Glu Asn Tyr Lys Val Lys Ser Gly Asp
355 360 365

Thr Leu Ser Gly Ile Ala Leu Ala Arg Gly Met Asp Leu Val Glu Leu
370 375 380

Tyr Ser Ile Asn Asn Ile Asn Asp Lys Tyr Ile Leu Lys Val Gly Asp
385 390 395 400

Asn Leu Lys Val Tyr Ala Asn Pro Lys Lys Thr Thr Thr Leu Val Ile
405 410 415

Ser Asn Tyr Lys Val Gln Ser Gly Asp Ser Leu Tyr Ser Ile Ala Lys
420 425 430

Lys His Lys Met Asp Leu Arg Asp Leu Met Gln Leu Asn Asn Ile Lys
435 440 445

Asn Ala Asn Glu Tyr Lys Leu Tyr Val Gly Ala Asn Leu Lys Val Lys
450 455 460

Thr Ala Lys Met Val Pro Tyr Ser Phe Asn Asp Asp Ser Ile Leu Pro
465 470 475 480

Asp Ser Ser Phe Ile Trp Pro Tyr Lys Gly Ile Ile Ile Ser Gly Tyr
485 490 495

Gly Val Ala Ser Asp Lys Leu Ala Asn Arg Gly Val Asn Ile Leu Gly
500 505 510

Asp Val Gly Asp Lys Val Val Ala Ser Asp Asp Gly Ile Val Glu Tyr
515 520 525

Ala Asp Asn Ile Arg Gly Phe Gly Thr Val Ile Ile Leu Lys His Lys
530 535 540

Asn Gly Tyr Asn Thr Ser Tyr Ala His Leu Ser Lys Ile Asn Val Lys
545 550 555 560

Leu Gly Asp Ile Val Lys Lys Gly Asp Tyr Ile Gly Asp Ile Gly Asp
565 570 575

Thr Gly Met Ile Asp Arg Ser Glu Leu Tyr Phe Lys Ile Ser Tyr Gln
580 585 590

Gly Arg Ser Ile Asp Pro Val Lys Leu Leu Pro Lys Ser
595 600 605

<210> 21
<211> 264
<212> DNA
<213> Brachyspira hyodysenteriae

<400> 21
atgaataaaa tttttatga taaagcttgg gaagattatc tttattttca aaagaatgac
60

aaaaaaatat tacagaaaat taatgatttt ataaaagata tagaaagaaa tggcttatta
120

attggtatag gaaaaccaga gagattaaaa ggtgagttaa acggattgta ttcaaggcta
180

ataaatcaag aacatagatt agtatattat attgaagata ataatttatt tatagttgga
240

tgtaaaacac attataaaaa taat
264

<210> 22
<211> 88
<212> PRT
<213> Brachyspira hyodysenteriae

<400> 22

Met Asn Lys Ile Phe Tyr Asp Lys Ala Trp Glu Asp Tyr Leu Tyr Phe
1 5 10 15

Gln Lys Asn Asp Lys Lys Ile Leu Gln Lys Ile Asn Asp Phe Ile Lys
20 25 30

Asp Ile Glu Arg Asn Gly Leu Leu Ile Gly Ile Gly Lys Pro Glu Arg
35 40 45

Leu Lys Gly Glu Leu Asn Gly Leu Tyr Ser Arg Leu Ile Asn Gln Glu
50 55 60

His Arg Leu Val Tyr Tyr Ile Glu Asp Asn Asn Leu Phe Ile Val Gly
65 70 75 80

Cys Lys Thr His Tyr Lys Asn Asn
85

<210> 23
<211> 711
<212> DNA
<213> Brachyspira hyodysenteriae

<400> 23
atgatagaat caatacaaag aatacatgcc agaataggcg agattcagga tacttttaat
60

aaattaggtt ttgctcctat taatactcag attcctacta aaccttttgc tgaacattta
120

aatgaagcta tggcagaaaa caaagtcaat aatattgatg gttctatagt taatgataca
180

aataaaaagt tagataatgg aaaagtaatt aatggagata cttcttctga tgctttcaaa
240

ggaaatatat catttggtgt ttatgatagt aatacaaata attttgctaa agctataaat
300

gcttataaaa aagcttcagt agaaagtttc cctactaaat atgatgatat aattaaagag
360

gcagcagaga aatattcttt gcctgaaaat ttaataaaag cggttataaa gcaggaatca
420

aactatgtgc ctaatgctgt aagtcataaa ggtgctgttg gtttgatgca gataatgccg
480

caaacaggtg ttggcttagg tattactgat acagaaatgc ttaaagatcc atacactaat
540

ataatggctg gaagcagata tttatcacag atgttaaaca gatatgatgg aagacttgat
600

ttatctttat ctgcttataa tgccggacct gctttggtag acagattaca gagaatccct
660

aatatagagg aaactcaaaa ctatgttaaa aacattatag gatatataaa g
711

<210> 24
<211> 237
<212> PRT
<213> Brachyspira hyodysenteriae

<400> 24

Met Ile Glu Ser Ile Gln Arg Ile His Ala Arg Ile Gly Glu Ile Gln
1 5 10 15

Asp Thr Phe Asn Lys Leu Gly Phe Ala Pro Ile Asn Thr Gln Ile Pro
20 25 30

Thr Lys Pro Phe Ala Glu His Leu Asn Glu Ala Met Ala Glu Asn Lys
35 40 45

Val Asn Asn Ile Asp Gly Ser Ile Val Asn Asp Thr Asn Lys Lys Leu
50 55 60

Asp Asn Gly Lys Val Ile Asn Gly Asp Thr Ser Ser Asp Ala Phe Lys
65 70 75 80

Gly Asn Ile Ser Phe Gly Val Tyr Asp Ser Asn Thr Asn Asn Phe Ala
85 90 95

Lys Ala Ile Asn Ala Tyr Lys Lys Ala Ser Val Glu Ser Phe Pro Thr
100 105 110

Lys Tyr Asp Asp Ile Ile Lys Glu Ala Ala Glu Lys Tyr Ser Leu Pro
115 120 125

Glu Asn Leu Ile Lys Ala Val Ile Lys Gln Glu Ser Asn Tyr Val Pro
130 135 140

Asn Ala Val Ser His Lys Gly Ala Val Gly Leu Met Gln Ile Met Pro
145 150 155 160

Gln Thr Gly Val Gly Leu Gly Ile Thr Asp Thr Glu Met Leu Lys Asp
165 170 175

Pro Tyr Thr Asn Ile Met Ala Gly Ser Arg Tyr Leu Ser Gln Met Leu
180 185 190

Asn Arg Tyr Asp Gly Arg Leu Asp Leu Ser Leu Ser Ala Tyr Asn Ala
195 200 205

Gly Pro Ala Leu Val Asp Arg Leu Gln Arg Ile Pro Asn Ile Glu Glu
210 215 220

Thr Gln Asn Tyr Val Lys Asn Ile Ile Gly Tyr Ile Lys
225 230 235

<210> 25
<211> 564
<212> DNA
<213> Brachyspira hyodysenteriae

<400> 25
atgcatattt ctggtgattc tcctttagat aggaaagttg gagatgctag ttattatttt 60

gctactgttc aacattgctg gggtgggct acttggaaaa gagcttggaa atattttgat 120

gtaactatgg aaagtataaa ttttgaagat gtaaaaaaaa caattagaaa aagatacaaa 180

gattttaata taaaagatta ctggcaaaga tggtttccta gaataaaaaa agagcattct 240

tctgtatggg attatcaatg gacttactgt attatctcaa aaaatggaat atgcattaat 300

ccaagtataa atttaacttc taatatagga tttggagaag attctaccca cactacaaat 360

gaaaatgatg aaaatataaa tagtaaaaca taccctatgg atactgaaaa tattattcat
420

cctaaagaaa taaaatgtga tgaaagagct gattttgaaa tagctattaa aagatttaat
480

ataaaacctt tctctttaac atctaatata accagagaag ttaaaagaat tataaaacaa
540

ataaaaaact tatttatcaa aaaa
564

<210> 26
<211> 188
<212> PRT
<213> Brachyspira hyodysenteriae

<400> 26

Met His Ile Ser Gly Asp Ser Pro Leu Asp Arg Lys Val Gly Asp Ala
1 5 10 15

Ser Tyr Tyr Phe Ala Thr Val Gln His Cys Trp Gly Trp Ala Thr Trp
20 25 30

Lys Arg Ala Trp Lys Tyr Phe Asp Val Thr Met Glu Ser Ile Asn Phe
35 40 45

Glu Asp Val Lys Lys Thr Ile Arg Lys Arg Tyr Lys Asp Phe Asn Ile
50 55 60

Lys Asp Tyr Trp Gln Arg Trp Phe Pro Arg Ile Lys Lys Glu His Ser
65 70 75 80

Ser Val Trp Asp Tyr Gln Trp Thr Tyr Cys Ile Ile Ser Lys Asn Gly
85 90 95

Ile Cys Ile Asn Pro Ser Ile Asn Leu Thr Ser Asn Ile Gly Phe Gly
100 105 110

Glu Asp Ser Thr His Thr Thr Asn Glu Asn Asp Glu Asn Ile Asn Ser
115 120 125

Lys Thr Tyr Pro Met Asp Thr Glu Asn Ile Ile His Pro Lys Glu Ile
130 135 140

Lys Cys Asp Glu Arg Ala Asp Phe Glu Ile Ala Ile Lys Arg Phe Asn
145 150 155 160

Ile Lys Pro Phe Ser Leu Thr Ser Asn Ile Thr Arg Glu Val Lys Arg
165 170 175

Ile Ile Lys Gln Ile Lys Asn Leu Phe Ile Lys Lys
180 185

<210> 27
<211> 396
<212> DNA
<213> Brachyspira hyodysenteriae

<400> 27
atgtttaata ctcctatatt attaattatt tttaaaagaa aatatactgc attaaaagtt
60

ttagatacaa taagaaatgt aaaacccaaa aaattatata tagcagctga tggctggaga
120

aatgaagaag aaaaaacaaa atgtattgat acaagagaag ctgtattaga agctgtagat
180

tgggaatgcg aagtaaaaac tttatttcaa gataaaaatt taggatgctg ttatggtcct
240

gtaaatgctg taaattggtt atttgaaaat gaagaacaag gaataatact tgaagatgat
300

gttatagctg aaacttcttt ttttattatt gcgagaaatt acttaactat tataaagata
360

atgaaaaaat tatgcatatt tctggtgatt ctcctt
396

<210> 28
<211> 132
<212> PRT
<213> Brachyspira hyodysenteriae

<400> 28

Met Phe Asn Thr Pro Ile Leu Leu Ile Ile Phe Lys Arg Lys Tyr Thr
1 5 10 15

Ala Leu Lys Val Leu Asp Thr Ile Arg Asn Val Lys Pro Lys Lys Leu
20 25 30

Tyr Ile Ala Ala Asp Gly Trp Arg Asn Glu Glu Glu Lys Thr Lys Cys
35 40 45

Ile Asp Thr Arg Glu Ala Val Leu Glu Ala Val Asp Trp Glu Cys Glu
50 55 60

Val Lys Thr Leu Phe Gln Asp Lys Asn Leu Gly Cys Cys Tyr Gly Pro
65 70 75 80

Val Asn Ala Val Asn Trp Leu Phe Glu Asn Glu Glu Gln Gly Ile Ile
85 90 95

Leu Glu Asp Asp Val Ile Ala Glu Thr Ser Phe Phe Ile Ile Ala Arg
100 105 110

Asn Tyr Leu Thr Ile Ile Lys Ile Met Lys Lys Leu Cys Ile Phe Leu
115 120 125

Val Ile Leu Leu
130

<210> 29
<211> 1296
<212> DNA
<213> Brachyspira hyodysenteriae

<400> 29
atggatataa ttataataat agtgttaata cttttaaatg gtatttttgc catgtcggaa
60

attgcagtaa tttctgctag aaaatcttct cttatgaaag acagcaaaga aggaaataaa
120

ggtgcaaaga ctgcattagc tttagcagat aatcctgaca aatttttatc tacaatacaa
180

ataggtataa ctttaatagg tatattaaca ggtatttact caggcgatac agttgccaaa
240

gaagtttcaa atttacttgt aaaaataaat gtaccattaa attatgcttc tttaatagct
300

caggtattgg tggtagcatt ggtaacatat cttacattga tattcggaga gcttgtgcct
360

aaaagaatag gaatggtaat gcctgaaaga atagcaaaag tggttgcagc tcctatgaca
420

atacttgcaa agataggtgc tccttttgtg tggatattat caaatagcgc attgcttgtt
480

tcaagagttt tgggtataaa agatgataaa agtcctgtta ctgaagagga aataaaatct
540

atgatagaag agggcagaca agggggagaa gttaaggaga tagaacagaa tattatagag
600

agggctttct ttttgggaga tagaaaaata gaatctataa tgacacatag aaatgatatg
660

gtatttttag atataaatat gagtaatgat gagataaaaa agatagtatc aaaacattct
720

ttttctgctt atcctgttgt tgataaaaat ttggataata ttgtaggagt tgtaagagta
780

actgatatat tcgataaatt aaatacttca aaggctaaaa tagaaaagtt tgtgaagaaa
840

gctaattact ttcataacaa tatggaagtt tatttggttc ttgaagagat gaaaaagaat
900

aatactaaaa taggtcttgt atcagatgag tttgggaata tagacggaat gattactcaa
960

agcgatatat tcgaggcttt agtgggttct gtaacagaag gaaaagacag taaggatatt
1020

agaaagagaa agagcggaag ttggtttgta gatggtcaat gtcctatgta tgatttctta
1080

gagtattttg aaatagaaga tgaaaatgct tctaataatt ataatactat aagcggtttg
1140

attttagaat tattacagca tgtacctagt gaaggagaat ctttagaatg gaagaattta
1200

aatttagaag ttgttgatat ggacggtgct agaatagata aggttatagt aaataaaata
1260

gaaaaaactg atgaaaagaa tgattcagac actgaa
1296

<210> 30
<211> 432
<212> PRT
<213> Brachyspira hyodysenteriae

<400> 30

Met Asp Ile Ile Ile Ile Ile Val Leu Ile Leu Leu Asn Gly Ile Phe
1 5 10 15

Ala Met Ser Glu Ile Ala Val Ile Ser Ala Arg Lys Ser Ser Leu Met
20 25 30

Lys Asp Ser Lys Glu Gly Asn Lys Gly Ala Lys Thr Ala Leu Ala Leu
35 40 45

Ala Asp Asn Pro Asp Lys Phe Leu Ser Thr Ile Gln Ile Gly Ile Thr
50 55 60

```
Leu Ile Gly Ile Leu Thr Gly Ile Tyr Ser Gly Asp Thr Val Ala Lys
65                  70                  75                  80

Glu Val Ser Asn Leu Leu Val Lys Ile Asn Val Pro Leu Asn Tyr Ala
                85                  90                  95

Ser Leu Ile Ala Gln Val Leu Val Val Ala Leu Val Thr Tyr Leu Thr
            100                 105                 110

Leu Ile Phe Gly Glu Leu Val Pro Lys Arg Ile Gly Met Val Met Pro
        115                 120                 125

Glu Arg Ile Ala Lys Val Val Ala Ala Pro Met Thr Ile Leu Ala Lys
    130                 135                 140

Ile Gly Ala Pro Phe Val Trp Ile Leu Ser Asn Ser Ala Leu Leu Val
145                 150                 155                 160

Ser Arg Val Leu Gly Ile Lys Asp Asp Lys Ser Pro Val Thr Glu Glu
                165                 170                 175

Glu Ile Lys Ser Met Ile Glu Glu Gly Arg Gln Gly Gly Glu Val Lys
            180                 185                 190

Glu Ile Glu Gln Asn Ile Ile Glu Arg Ala Phe Phe Leu Gly Asp Arg
        195                 200                 205

Lys Ile Glu Ser Ile Met Thr His Arg Asn Asp Met Val Phe Leu Asp
    210                 215                 220

Ile Asn Met Ser Asn Asp Glu Ile Lys Lys Ile Val Ser Lys His Ser
225                 230                 235                 240

Phe Ser Ala Tyr Pro Val Val Asp Lys Asn Leu Asp Asn Ile Val Gly
                245                 250                 255

Val Val Arg Val Thr Asp Ile Phe Asp Lys Leu Asn Thr Ser Lys Ala
            260                 265                 270

Lys Ile Glu Lys Phe Val Lys Lys Ala Asn Tyr Phe His Asn Asn Met
        275                 280                 285

Glu Val Tyr Leu Val Leu Glu Glu Met Lys Lys Asn Asn Thr Lys Ile
    290                 295                 300
```

```
Gly Leu Val Ser Asp Glu Phe Gly Asn Ile Asp Gly Met Ile Thr Gln
305                 310                 315                 320

Ser Asp Ile Phe Glu Ala Leu Val Gly Ser Val Thr Glu Gly Lys Asp
                325                 330                 335

Ser Lys Asp Ile Arg Lys Arg Lys Ser Gly Ser Trp Phe Val Asp Gly
            340                 345                 350

Gln Cys Pro Met Tyr Asp Phe Leu Glu Tyr Phe Glu Ile Glu Asp Glu
        355                 360                 365

Asn Ala Ser Asn Asn Tyr Asn Thr Ile Ser Gly Leu Ile Leu Glu Leu
    370                 375                 380

Leu Gln His Val Pro Ser Glu Gly Glu Ser Leu Glu Trp Lys Asn Leu
385                 390                 395                 400

Asn Leu Glu Val Val Asp Met Asp Gly Ala Arg Ile Asp Lys Val Ile
                405                 410                 415

Val Asn Lys Ile Glu Lys Thr Asp Glu Lys Asn Asp Ser Asp Thr Glu
            420                 425                 430
```

<210> 31
<211> 1386
<212> DNA
<213> Brachyspira hyodysenteriae

<400> 31

```
atggaaatac tactagttta tttatttgaa atatttataa ttattattct tattatgctc
60

tctgctttat tttctggaag tgagactgca tatacatcta ttgatgatgt tactttaatg
120

cgtttggtca gagagaaaaa aatcaaagaa gaagataaaa aatattggga aaagtcaagt
180

tctatgatac ctaccctact tgttggtaat aacatagtta atatatcagc aagttccatt
240

ataactgtat ttgctgtaag gcttgctgac attctgccgc atgtatcaac aaatgtgatg
300

gttacaatat caactgccac aataacaata cttattatta tattcggaga aatacttcct
360
```

aaagtcttaa tgagagttaa tgctgaaaaa atgatgcctt atcttctata ttttatgaag
420

ttttgtcatt ttatattcaa gcctataact ttcttaatgg ataaagtaac tacttttata
480

atgaattatt ttgtacctaa aagattaaga gatgctgaaa aaagaagtgc tttatcaagt
540

atggaagata tcactactat aatacatttg gggcataaag aaggaataat aaaagaatat
600

acacatgaaa tgcttacagg agtaatagat tttagaaata aaactgtaga agagataatg
660

acgccccgtg ttgatatggt atgtattgaa gctgaaacag atgtaaatga aataataaaa
720

cttactgtag aatcaggact ttcaagattt cctgtttatg aggaaacagt agatcatata
780

ataggtatat tccatacaag agctttattt aaagaatatg ttaaaggcgg cggaaagatg
840

aacaaagtaa aaaagaaagc aatagattat ataatgcttc cctactttgt acctgaaact
900

aaaactataa gcagcttatt tagtgatatg caaaagaaaa aacttcagat ggtaattact
960

attgatgaat acggcggaac tgccggactt gttactatgg aagatataat agaagagata
1020

atgggtgata tagaagatga aagtgataaa aaagaagctg atgtaataag atttaaggga
1080

aaaagaatta taataaatgg aaatgcttct atagaagatg tcaacaaaac tttaaaatta
1140

gaattagagc atgaagaata tcaaactata gcaggatatg ttattgatat gcttgatcat
1200

atacctgaaa caaatgagag attcatatta aaaggatata gggtaagaat aatgaaagtt
1260

gaagacagaa gaatagttga aatggaattt actcctataa aatttgcaag aacaaatgaa
1320

agtgataata ttgatataca agagacatct gattcagaaa aaaatgattt agaaatttta
1380

aatgaa
1386

<210> 32
<211> 462
<212> PRT
<213> Brachyspira hyodysenteriae

<400> 32

Met Glu Ile Leu Leu Val Tyr Leu Phe Glu Ile Phe Ile Ile Ile Ile
1               5                   10                  15

Leu Ile Met Leu Ser Ala Leu Phe Ser Gly Ser Glu Thr Ala Tyr Thr
            20                  25                  30

Ser Ile Asp Asp Val Thr Leu Met Arg Leu Val Arg Glu Lys Lys Ile
        35                  40                  45

Lys Glu Glu Asp Lys Lys Tyr Trp Glu Lys Ser Ser Ser Met Ile Pro
    50                  55                  60

Thr Leu Leu Val Gly Asn Asn Ile Val Asn Ile Ser Ala Ser Ser Ile
65                  70                  75                  80

Ile Thr Val Phe Ala Val Arg Leu Ala Asp Ile Leu Pro His Val Ser
                85                  90                  95

Thr Asn Val Met Val Thr Ile Ser Thr Ala Thr Ile Thr Ile Leu Ile
            100                 105                 110

Ile Ile Phe Gly Glu Ile Leu Pro Lys Val Leu Met Arg Val Asn Ala
        115                 120                 125

Glu Lys Met Met Pro Tyr Leu Leu Tyr Phe Met Lys Phe Cys His Phe
    130                 135                 140

Ile Phe Lys Pro Ile Thr Phe Leu Met Asp Lys Val Thr Thr Phe Ile
145                 150                 155                 160

Met Asn Tyr Phe Val Pro Lys Arg Leu Arg Asp Ala Glu Lys Arg Ser
                165                 170                 175

Ala Leu Ser Ser Met Glu Asp Ile Thr Thr Ile Ile His Leu Gly His
            180                 185                 190

Lys Glu Gly Ile Ile Lys Glu Tyr Thr His Glu Met Leu Thr Gly Val
        195                 200                 205

Ile Asp Phe Arg Asn Lys Thr Val Glu Glu Ile Met Thr Pro Arg Val
    210                 215                 220

```
Asp Met Val Cys Ile Glu Ala Glu Thr Asp Val Asn Glu Ile Ile Lys
225                 230                 235                 240

Leu Thr Val Glu Ser Gly Leu Ser Arg Phe Pro Val Tyr Glu Glu Thr
                245                 250                 255

Val Asp His Ile Ile Gly Ile Phe His Thr Arg Ala Leu Phe Lys Glu
            260                 265                 270

Tyr Val Lys Gly Gly Gly Lys Met Asn Lys Val Lys Lys Lys Ala Ile
        275                 280                 285

Asp Tyr Ile Met Leu Pro Tyr Phe Val Pro Glu Thr Lys Thr Ile Ser
    290                 295                 300

Ser Leu Phe Ser Asp Met Gln Lys Lys Lys Leu Gln Met Val Ile Thr
305                 310                 315                 320

Ile Asp Glu Tyr Gly Gly Thr Ala Gly Leu Val Thr Met Glu Asp Ile
                325                 330                 335

Ile Glu Glu Ile Met Gly Asp Ile Glu Asp Glu Ser Asp Lys Lys Glu
            340                 345                 350

Ala Asp Val Ile Arg Phe Lys Gly Lys Arg Ile Ile Ile Asn Gly Asn
        355                 360                 365

Ala Ser Ile Glu Asp Val Asn Lys Thr Leu Lys Leu Glu Leu Glu His
    370                 375                 380

Glu Glu Tyr Gln Thr Ile Ala Gly Tyr Val Ile Asp Met Leu Asp His
385                 390                 395                 400

Ile Pro Glu Thr Asn Glu Arg Phe Ile Leu Lys Gly Tyr Arg Val Arg
                405                 410                 415

Ile Met Lys Val Glu Asp Arg Arg Ile Val Glu Met Glu Phe Thr Pro
            420                 425                 430

Ile Lys Phe Ala Arg Thr Asn Glu Ser Asp Asn Ile Asp Ile Gln Glu
        435                 440                 445

Thr Ser Asp Ser Glu Lys Asn Asp Leu Glu Ile Leu Asn Glu
    450                 455                 460
```

<210> 33
<211> 19
<212> DNA
<213> Artificial

<220>
<223> Primer

<400> 33
catatttctg gtgattctc
19

<210> 34
<211> 27
<212> DNA
<213> Artificial

<220>
<223> Primer

<400> 34
tttttgata aataagtttt ttatttg
27

<210> 35
<211> 28
<212> DNA
<213> Artificial

<220>
<223> Primer

<400> 35
tttaatactc ctatattatt aattattt
28

<210> 36
<211> 18
<212> DNA
<213> Artificial

<220>
<223> Primer

<400> 36
aaggagaatc accagaaa
18

<210> 37
<211> 20
<212> DNA
<213> Artificial

<220>

<223> Primer

<400> 37
aaatatgctt ccattatagg
20

<210> 38
<211> 21
<212> DNA
<213> Artificial

<220>
<223> Primer

<400> 38
acttttagga agaagtttaa c
21

<210> 39
<211> 28
<212> DNA
<213> Artificial

<220>
<223> Primer

<400> 39
gatataatta taataatagt gttaatac
28

<210> 40
<211> 19
<212> DNA
<213> Artificial

<220>
<223> Primer

<400> 40
ttcagtgtct gaatcattc
19

<210> 41
<211> 20
<212> DNA
<213> Artificial

<220>
<223> Primer

<400> 41
tatgatgggg catagtatgg
20

<210> 42
<211> 21
<212> DNA
<213> Artificial

<220>
<223> Primer

<400> 42
tatctcctac agggtctttc g
21

<210> 43
<211> 24
<212> DNA
<213> Artificial

<220>
<223> Primer

<400> 43
atgataaagc ttgggaagat tatc
24

<210> 44
<211> 21
<212> DNA
<213> Artificial

<220>
<223> Primer

<400> 44
ataatgtgtt ttacatccaa c
21

<210> 45
<211> 25
<212> DNA
<213> Artificial

<220>
<223> Primer

<400> 45
ttatgctctc tgctttattt tctgg
25

<210> 46
<211> 25
<212> DNA
<213> Artificial

<220>
<223> Primer

<400> 46
tcttttatta ttccttcttt atgcc
25

<210> 47
<211> 20
<212> DNA
<213> Artificial

<220>
<223> Primer

<400> 47
ctatggcaga aaacaaagtc
20

<210> 48
<211> 22
<212> DNA
<213> Artificial

<220>
<223> Primer

<400> 48
gcagataaag ataaatcaag tc
22

<210> 49
<211> 14
<212> DNA
<213> Artificial

<220>
<223> Primer

<400> 49
aaagagcttg gcag
14

<210> 50
<211> 26
<212> DNA
<213> Artificial

<220>
<223> Primer

<400> 50
cttatcataa tggtatttac aagatg
26

<210> 51

<211> 21
<212> DNA
<213> Artificial

<220>
<223> Primer

<400> 51
tgctattgct gatgctacta c
21

<210> 52
<211> 23
<212> DNA
<213> Artificial

<220>
<223> Primer

<400> 52
attatcttga tgaggatgct ttc
23

<210> 53
<211> 25
<212> DNA
<213> Artificial

<220>
<223> Primer

<400> 53
tagataaaga tgatgatgaa aaaag
25

<210> 54
<211> 21
<212> DNA
<213> Artificial

<220>
<223> Primer

<400> 54
aaataaacct aaataagcac c
21

<210> 55
<211> 21
<212> DNA
<213> Artificial

<220>
<223> Primer

<400> 55
tgggtttaga ataggtgcag g
21

<210> 56
<211> 22
<212> DNA
<213> Artificial

<220>
<223> Primer

<400> 56
attttaatgt ttgtcttttt gc
22

<210> 57
<211> 20
<212> DNA
<213> Artificial

<220>
<223> Primer

<400> 57
tagaaaagat gcctatgctg
20

<210> 58
<211> 22
<212> DNA
<213> Artificial

<220>
<223> Primer

<400> 58
ttacaagaga aaaataaacc tg
22

<210> 59
<211> 22
<212> DNA
<213> Artificial

<220>
<223> Primer

<400> 59
tggggaatca tacaaataga ag
22

<210> 60
<211> 20

<212> DNA
<213> Artificial

<220>
<223> Primer

<400> 60
ggctcatcaa aggaagaacc
20

<210> 61
<211> 23
<212> DNA
<213> Artificial

<220>
<223> Primer

<400> 61
ataataaaaa agaagaaaaa gag
23

<210> 62
<211> 25
<212> DNA
<213> Artificial

<220>
<223> Primer

<400> 62
gtctgtatcc taataaaata gtatg
25

<210> 63
<211> 22
<212> DNA
<213> Artificial

<220>
<223> Primer

<400> 63
tcttgggctt acatttactt tg
22

<210> 64
<211> 21
<212> DNA
<213> Artificial

<220>
<223> Primer

<400> 64

tgatttctga cttctttttt g
21

<210> 65
<211> 29
<212> DNA
<213> Artificial

<220>
<223> Primer

<400> 65
tatgcatatg tctggtgatt ctcctttag
29

<210> 66
<211> 25
<212> DNA
<213> Artificial

<220>
<223> Primer

<400> 66
tgcggatcct tttttgataa ataag
25

<210> 67
<211> 25
<212> DNA
<213> Artificial

<220>
<223> Primer

<400> 67
ggaagataac atatgtttaa tactc
25

<210> 68
<211> 32
<212> DNA
<213> Artificial

<220>
<223> Primer

<400> 68
tcctaggatc ctatctaaag gagaatcacc ag
32

<210> 69
<211> 35
<212> DNA

<213> Artificial

<220>
<223> Primer

<400> 69
aaatatgcat atgttatagg atgcactttt gcctc
35

<210> 70
<211> 34
<212> DNA
<213> Artificial

<220>
<223> Primer

<400> 70
cttttaggat ccagtttaac aggatctatt gatc
34

<210> 71
<211> 38
<212> DNA
<213> Artificial

<220>
<223> Primer

<400> 71
aatccatatg gatataatta taataatagt gttaatac
38

<210> 72
<211> 41
<212> DNA
<213> Artificial

<220>
<223> Primer

<400> 72
atctggatcc tattcagtgt ctgaatcatt cttttcatca g
41

<210> 73
<211> 37
<212> DNA
<213> Artificial

<220>
<223> Primer

<400> 73

cttcatatgg aaatgcttaa tagagtttta atatcag
37

<210> 74
<211> 38
<212> DNA
<213> Artificial

<220>
<223> Primer

<400> 74
aataggatcc tatatgagca ttaagccaag caattatc
38

<210> 75
<211> 31
<212> DNA
<213> Artificial

<220>
<223> Primer

<400> 75
atttcatatg ataaagcttg ggaagattat c
31

<210> 76
<211> 31
<212> DNA
<213> Artificial

<220>
<223> Primer

<400> 76
aattggatcc ataatgtgtt ttacatccaa c
31

<210> 77
<211> 35
<212> DNA
<213> Artificial

<220>
<223> Primer

<400> 77
attacatatg gaaatactac tagtttattt atttg
35

<210> 78
<211> 33
<212> DNA

<213> Artificial

<220>
<223> Primer

<400> 78
cattggatcc tgaatcagat gtctcttgta tat
33

<210> 79
<211> 35
<212> DNA
<213> Artificial

<220>
<223> Primer

<400> 79
gatacatatg atacaaagaa tacatgccag aatag
35

<210> 80
<211> 29
<212> DNA
<213> Artificial

<220>
<223> Primer

<400> 80
atgtggatcc catagttttg agtttcctc
29

<210> 81
<211> 24
<212> DNA
<213> Artificial

<220>
<223> Primer

<400> 81
gtttcatatg aaagagcttg gcag
24

<210> 82
<211> 36
<212> DNA
<213> Artificial

<220>
<223> Primer

<400> 82

atatggatcc cttatcataa tggtatttac aagatg
36

<210> 83
<211> 28
<212> DNA
<213> Artificial

<220>
<223> Primer

<400> 83
ttaacatatg gttctctttg cggaagag
28

<210> 84
<211> 34
<212> DNA
<213> Artificial

<220>
<223> Primer

<400> 84
ttaggatccc atcaatacct aatttttcat taac
34

<210> 85
<211> 41
<212> DNA
<213> Artificial

<220>
<223> Primer

<400> 85
ttaacatatg tcagagaata gttttaaaaa tagaataaaa g
41

<210> 86
<211> 34
<212> DNA
<213> Artificial

<220>
<223> Primer

<400> 86
ttaaggatcc gaagcaaatg aaagttcttt aatc
34

<210> 87
<211> 36
<212> DNA

<213> Artificial

<220>
<223> Primer

<400> 87
ttttcatatg gcacaaacta atgaatcagc tatatt
36

<210> 88
<211> 37
<212> DNA
<213> Artificial

<220>
<223> Primer

<400> 88
ttaaggatcc gttaagcagc accagagaat tataatc
37

<210> 89
<211> 37
<212> DNA
<213> Artificial

<220>
<223> Primer

<400> 89
cttacatatg ggaacaagag ttgttttaga aaagatg
37

<210> 90
<211> 34
<212> DNA
<213> Artificial

<220>
<223> Primer

<400> 90
actgggatcc gacttgctta tatccttagt accg
34

<210> 91
<211> 32
<212> DNA
<213> Artificial

<220>
<223> Primer

<400> 91

taatcatatg atgtacagtc aggttatgat ag
32

<210> 92
<211> 30
<212> DNA
<213> Artificial

<220>
<223> Primer

<400> 92
actgggatcc tgtaccgaca ttctcttcac
30

<210> 93
<211> 36
<212> DNA
<213> Artificial

<220>
<223> Primer

<400> 93
aaagcatatg gaagaaaaaa aatccaaatt ggaaag
36

<210> 94
<211> 36
<212> DNA
<213> Artificial

<220>
<223> Primer

<400> 94
atacggatcc aatatatgtt cactgtccat agcctc
36

<210> 95
<211> 36
<212> DNA
<213> Artificial

<220>
<223> Primer

<400> 95
ctttcatatg cttgggctta catttacttt ggttgg
36

<210> 96
<211> 37
<212> DNA

```
<213>  Artificial

<220>
<223>  Primer

<400>  96
ataaggatcc cataagtcat tcttgctcgt tttatag
37
```